# EUROPEAN PATENT SPECIFICATION

(11) **EP 3 288 558 B1**
(45) Date of publication and mention of the grant of the patent: **11.05.2022**
(21) Application number: 16723280.0
(22) Date of filing: 29.04.2016
(51) Int. Cl.: A61K 31/4439, A61K 31/454, A61K 31/496, A61K 31/497, A61K 31/4985, A61K 31/505, A61K 31/506, A61K 31/519, A61K 31/5377, A61K 31/55, C07D 401/12, A61P 35/02, A61P 35/00

(54) **COMBINATIONS OF INHIBITORS OF IRAK4 WITH INHIBITORS OF BTK**
KOMBINATIONEN VON IRAK4 INHIBITOREN UND BTK INHIBITOREN
COMBINAISONS D'INHIBITEURS D'IRAK4 ET INHIBITEURS DE BTK

(30) Priority: 30.04.2015 EP 15166016; 25.01.2016 EP 16152499
(43) Date of publication of application: 07.03.2018
(73) Proprietor: Bayer Pharma Aktiengesellschaft, 13353 Berlin (DE)
(72) Inventor: BOTHE, Ulrich, 13187 Berlin (DE); WENGNER, Antje Margret, 13189 Berlin (DE); SIEBENEICHER, Holger, 10557 Berlin (DE); SCHMIDT, Nicole, San Francisco, California 94105 (US); NUBBEMEYER, Reinhard, 13509 Berlin (DE); BÖMER, Ulf, 16548 Glienicke (DE); GÜNTHER, Judith, 13187 Berlin (DE); STEUBER, Holger, 10115 Berlin (DE); LANGE, Martin, 10405 Berlin (DE); STEGMANN, Christian, 10437 Berlin (DE); SUTTER, Andreas, 13086 Berlin (DE); NEUHAUS, Roland, 12157 Berlin (DE)
(74) Representative: BIP Patents
(86) International application number: PCT/EP2016/059576
(87) International publication number: WO 2016/174183

(56) References cited:
- WO-A1-2015/091426
- WO-A1-2016/083433
- US-A1- 2014 249 142
- Yang Guang ET AL: "Regular Article A mutation in MYD88 (L265P) supports the survival of lymphoplasmacytic cells by activation of Bruton tyrosine kinase in Waldenström macroglobulinemia", Blood, 8 July 2013 (2013-07-08), XP055281747, DOI: 10.1182/blood-2012-12- Retrieved from the Internet: URL:http://www.bloodjournal.org/content/bl oodjournal/122/7/1222.full.pdf?sso-checked =true [retrieved on 2016-06-20]
- ERIC G. VAJDA ET AL: "Abstract 785: IRAK4 inhibitors display synergistic activity when combined with BTK or PI3K inhibitors in B cell lymphomas", CANCER RESEARCH, vol. 75, no. 15 Supplement, 18 April 2015 (2015-04-18), - 22 April 2015 (2015-04-22), pages 785-785, XP055281607, US ISSN: 0008-5472, DOI: 10.1158/1538-7445.AM2015-785

## Description

The present invention relates to combinations of at least two components, component A and component B:
- component A is an IRAK4-inhibiting compound as defined in the claims, or a salt, a solvate or a solvate of a salt thereof;
- component B is a BTK-inhibiting compound as defined in the claims; and, optionally,
- one or more components C which are pharmaceutical products as defined in the claims for use in the treatment and/or prophylaxis of pathological conditions equally defined in the claims. A further aspect of the present invention relates to combinations of at least two components, component A and component B:
   - component A is an IRAK4-inhibiting compound as defined in the claims, or a salt, a solvate or a solvate of a salt thereof;
   - component B is a BTK-inhibiting compound selected from the following list:
      ∘ ibrutinib, or a pharmaceutically acceptable salt thereof;
      ∘ 4-tert-butyl-N-[2-methyl-3-(4-methyl-6-{[4-(morpholin-4-ylcarbonyl)phenyl]amino}-5-oxo-4,5-dihydropyrazin-2-yl)phenyl]benzamide (CGI-1746, CAS 910232-84-7);
      ∘ N-{3-[(5-fluoro-2-{[4-(2-methoxyethoxy)phenyl]amino}pyrimidin-4-yl)amino]phenyl}acrylamide (AVL-292, CAS 1202757-89-8);
      ∘ 6-cyclopropyl-8-fluoro-2-[2-(hydroxymethyl)-3-(1-methyl-5-{[5-(4-methylpiperazin-1-yl)pyridin-2-yl]amino}-6-oxo-1,6-dihydropyridin-3-yl)phenyl]isoquinolin-1(2H)-one (RN486, CAS 1242156-23-5)
for use in the treatment and/or prophylaxis of pathological conditions equally defined in the claims.

The present document further relates to a kit comprising combinations of:
- one or more components A consisting of an IRAK4-inhibiting compound as defined in the claims, or a salt, a solvate or a solvate of a salt thereof;
- a component B which is a BTK-inhibiting compound, or a pharmaceutically acceptable salt thereof as defined in the claims.

Component A can be administered by an oral, intravenous, topical, intraperitoneal, nasal, parenteral, pulmonary, sublingual, lingual, buccal, rectal, dermal, transdermal or conjunctival route, via the ear or as an implant or stent, or as a depot.

Component B can be administered by an oral, intravenous, topical, intraperitoneal, nasal, parenteral, pulmonary, sublingual, lingual, buccal, rectal, dermal, transdermal or conjunctival route, via the ear or as an implant or stent, or as a depot.

### BACKGROUND OF THE INVENTION

### COMPONENT A: IRAK4 INHIBITORS:

Human IRAK4 (interleukin-1 receptor-associated kinase 4) plays a key role in the activation of the immune system. Therefore, this kinase is an important target molecule for the development of inflammation-inhibiting substances. IRAK4 is expressed by a multitude of cells and mediates the signal transduction of Toll-like receptors (TLR), except for TLR3, and receptors of the interleukin (IL)-1β family consisting of the IL-1R (receptor), IL-18R, IL-33R and IL-36R (Janeway and Medzhitov, Annu. Rev. Immunol., 2002; Dinarello, Annu. Rev. Immunol., 2009; Flannery and Bowie, Biochemical Pharmacology, 2010).

Neither IRAK4 knockout mice nor human cells from patients lacking IRAK4 react to stimulation by TLRs (except for TLR3) and the IL-1β family (Suzuki, Suzuki, et al., Nature, 2002; Davidson, Currie, et al., The Journal of Immunology, 2006; Ku, von Bernuth, et al., JEM, 2007; Kim, Staschke, et al., JEM, 2007).

The binding of the TLR ligands or the ligands of the IL-1β family to the respective receptor leads to recruitment and binding of MyD88 [Myeloid differentiation primary response gene (88)] to the receptor. As a result, MyD88 interacts with IRAK4, resulting in the formation of an active complex which interacts with and activates the kinases IRAK1 or IRAK2 (Kollewe, Mackensen, et al., Journal of Biological Chemistry, 2004; Precious et al., J. Biol. Chem., 2009). As a result of this, the NF (nuclear factor)-kB signalling pathway and the MAPK (mitogen-activated protein kinase) signal pathway is activated (Wang, Deng, et al., Nature, 2001). The activation both of the NF-kB signal pathway and of the MAPK signal pathway leads to processes associated with different immune processes. For example, there is increased expression of various inflammatory signal molecules and enzymes such as cytokines, chemokines and COX-2 (cyclooxygenase-2), for example, and increased mRNA stability of inflammation-associated genes, for example COX-2, IL-6, IL-8 (Holtmann, Enninga, et al., Journal of Biological Chemistry, 2001; Datta, Novotny, et al., The Journal of Immunology, 2004). Furthermore, these processes may be associated with the proliferation and differentiation of particular cell types, for example monocytes, macrophages, dendritic cells, T cells and B cells (Wan, Chi, et al., Nat Immunol, 2006; McGettrick and J. O'Neill, British Journal of Haematology, 2007).

This also applies to some oncological disorders. Particular lymphomas, for example ABC-DLBCL (activated B-cell-like diffuse large-cell B-cell lymphoma), mantle cell lymphoma and Waldenström's disease, and also chronic lymphatic leukaemia, melanoma and liver cell carcinoma, are characterized by mutations in MyD88 or changes in MyD88 activity which can be treated by an IRAK4 inhibitor (Ngo, Young, et al., Nature, 2011; Puente, Pinyol, et al., Nature, 2011; Srivastava, Geng, et al., Cancer Research, 2012; Treon, Xu, et al., New England Journal of Medicine, 2012; Choi, Kim, et al., Human Pathology, 2013; Liang, Chen, et al., Clinical Cancer Research, 2013). In addition, MyD88 plays an important role in ras-dependent tumours, and so IRAK4 inhibitors are also suitable for treatment thereof (Kfoury, A., K. L. Corf, et al., Journal of the National Cancer Institute, 2013).

Diffuse large-cell B-cell lymphoma (DLBCL) is an aggressive tumour of B lymphocytes and the most common non-Hodgkin's lymphoma in adults (Morton LM, et al., Blood 2006). In morphological terms, DLBCL subdivides into centroblastic, immunoblastic and anaplastic lymphomas, dividing on the basis of gene expression into activated B-cell-like lymphoma (ABC-DLBCL) or germinal centre B-cell-like lymphoma (GCB-DLBCL) and genetic lymphoma after PRDM1 mutations and BCL2, BCL6, MYC translocations. The standard treatment for DLBCL is R-CHOP, a combination of the chemotherapeutic drugs cyclophosphamide, doxorubicin, vincristine and prednisone (CHOP) and rituximab, a chimeric monoclonal CD20 receptor antibody (Roschewski M et al., Nature Reviews Clinical Oncology, 2014). About one third of sufferers do not respond to the standard treatment or suffer a relapse, which makes it clear that there is a need to develop new therapeutic drugs (Friedberg, J. W. Hematology Am. Soc. Hematol. Educ. Program 2011). The ABC-DLBCL subtype represents about 30% of all DLBCLs and means the worst prognosis for patients (Siegel, R., et al., CA Cancer J. Clin. 2013). It has been shown that the NF-κB signalling pathway, which is important for the survival of DLBCL cells, is regulated both by the activation of the B-cell receptor (BCR) and of the Toll-like receptor (TLR) (Rawlings, D. J., et al. Nat. Rev. Immunol. 2012). In ABC-DLBCL, the NF-κB signalling pathway is often constitutively activated by mutations in these two signal pathways (Compagno, M. et al. Nature 2009). Activating mutations in MYD88, an adapter protein of the TLR signalling pathway, were found in almost 30% of all ABC-DLBCLs. These mutations lead to activation of IRAK4 and subsequent stimulation of the NF-κB signalling pathway, interleukin-6/interleukin-10 secretion and the activation of the JAK-STAT signalling pathway. An essential role has been shown for IRAK4 in the regulation of cell viability (Ngo, VN et al. Nature, 2011). The aberrant activation of the BCR and MYD88 signalling pathways indicates that blockage of the two signalling pathways could be therapeutically effective. Ibrutinib (PCI-32765) is an irreversible inhibitor of Bruton tyrosine kinase (BTK), one component of the BCR signalling pathway (Winer ES, et al., Expert Opin. Investig. Drugs, 2012). In a phase 2 study of ibrutinib in relapsed DLBCL patients, 40% of the patients responded to treatment with ibrutinib, which suggests that further signalling pathways are relevant in DLBCL (Wilson WH et al. ASH Annu Meet Abstr 2012, 120(21):686.). It has been shown that combinations of ibrutinib and various inhibitors of the PI3K signalling pathway and combinations of ibrutinib and inhibitors of the BCL-2 family have an additive or synergistic effect on cell viability in ABC-DLBCL (Mathews Griner LA et al., Proc Natl Acad Sci USA. 2014).

The prior art discloses a multitude of IRAK4 inhibitors (see, for example, Annual Reports in Medicinal Chemistry (2014), 49, 117 - 133).

US8293923 and US20130274241 disclose IRAK4 inhibitors having a 3-substituted indazole structure. There is no description of 2-substituted indazoles.

WO2013106254 and WO2011153588 disclose 2,3-disubstituted indazole derivatives.

WO2007091107 describes 2-substituted indazole derivatives for the treatment of Duchenne muscular dystrophy. The compounds disclosed do not have 6-hydroxyalkyl substitution.

WO2015091426 describes indazoles such as Example 64 substituted at position 2 with a carboxamide side-chain.

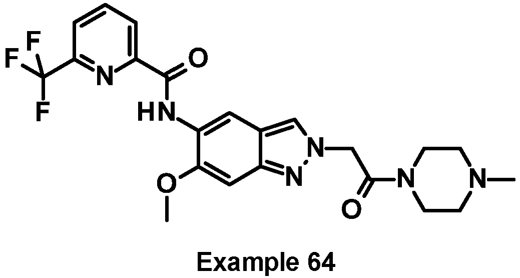

WO2015104662 describes 2-substituted indazoles of the following general formula: 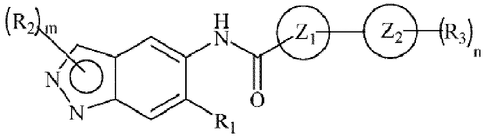 in which R² is an alkyl- or cycloalkyl group. Explicity reported are 2-substituted indazoles with a methyl, 2-methoxyethyl and cyclopentyl group at the 2-position (Examples 1, 4 and 76). In addition, Example 117 represents an indazole derivative with a hydroxyethyl-subsituent at the 1-position. However, no indazole derivatives displaying a 3-Hydroxy-3-methylbutyl-substituent at the 1-position or 2-position are described.

Indazoles displaying a hydroxy-substituted alkyl group in the 2-position are generically covered by the general formula, but are not exemplified, in WO2015104662.

Indazoles diplaying an alkyl group in position 2, which are substituted at the 2-alkyl group with a methylsulfonyl group, are not covered by the general formula and the definitions of the substituent R² in WO2015104662.

WO2015104662 describes indazoles wherein in position 6, examples of substituents for R¹ decribed are cyclopropyl, cyclohexyl, cyano, 3-fluorphenyl and saturated heterocyclic substituents. Indazoles with a hydroxy-substituted alkyl group in position 6 are not explicity described in WO2015104662.

WO2015193846 discloses 2-substituted indazoles of the following general formula: 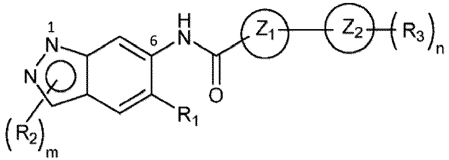 in which Z¹ and Z² are both an optionally substituted cycloalkyl-, aryl- or heteroaryl group. R² can have the meaning of hydrogen, halogen, an amino group, an optionally substituted alkyl-, cycloalkyl-, aryl-, heterocyclo-, arylalkyl- or heterocycloalkyl group. Indazole derivatives are explicity described in which R² means methyl and Z¹ and/or Z² mean heteroaryl groups; the -NH(C=O)Z¹-Z²-(R³)ₙ substituent is bound to the 6-position of the indazole scaffold. Indazole derivates displaying a -NH(C=O)Z¹-Z²-(R³)ₙ substituent bound to the 5-position are not described.

WO 2016/083433 A1 (Bayer Pharma AG) relates to substituted indazoles which include the component A compounds 1 to 21 as defined in the claims, as componds *perse.,* to methods for the production thereof, to the use thereof alone or in combinations to treat and/or prevent diseases, and to use thereof to produce drugs for treating and/or preventing diseases, in particular for treating and/or preventing endometriosis and endometriosis-associated pain and other symptoms associated with endometriosis such as dysmenorrhea, dyspareunia, dysuria, and dyschezia, lymphomas, rheumatoid arthritis, spondyloarthritides (in particular psoriatic spondyloarthritis and Bekhterev's disease), lupus erythematosus, multiple sclerosis, macular degeneration, COPD, gout, fatty liver diseases, insulin resistance, tumor diseases, and psoriasis. However, WO 2016/083433 A1 does not specifically describe or claim the specific combinations as described and claimed in the present document.

Yang Guang et al., in an article entitled: "Regular Article A mutation in MYD88 (L265P) supports the survival of lymphoplasmacytic cells by activation of Bruton tyrosine kinase in Waldenström macroglobulinemia", Blood, 8 July 2013 (2013-07-08), DOI: 10.1182/blood-2021-12-475111 and

Eric G. Vajda et al., in "Abstract 785: IRAK4 inhibitors display synergistic activity when combined with BTK or PI3K inhibitors in B cell lymphomas", Cancer Research, vol. 75, no. 15 Supplement, 18 April 2015 (2015-04-18), - 22 April 2015 (2015-04-22), pages 785-785, US, ISSN: 0008-5472, DOI: 10.1158/1538-7445.AM2015-785, disclose a synergy between inhibitors of BTK and inhibitors of IRAK when inhibiting NF-kB, Waldenström macroglobulinemia and B cell lymphoma. However, no chemical structure of the IRAK inhibitor(s) is disclosed in these two documents.

### COMPONENT B: BTK inhibitors:

Bruton tyrosine kinase (BTK) is an enzyme in mammals which catalyses the phosphorylation of particular proteins. It is one of the tyrosine kinases of the Tec family which is expressed particularly in B cells. BTK assumes important functions in the mediation of the B cell receptor signal within the cell. A mutation in the human BTK gene is the cause of what is called Bruton's syndrome (XLA).

Chronic lymphatic leukaemia (CLL) is incurable to date except by an allogeneic stem cell transplant, and patients having particular risk factors (particularly 17p deletion) barely benefit from CD20 antibodies either. The signalling pathway of the B cell receptor which is essential to B cell lymphoma has now been researched in detail and has led to new therapeutic routes. Bruton's tyrosine kinase (BTK) is a central component in this signalling pathway, and the approval of the BTK inhibitor ibrutinib (Imbruvica^{®}) in October 2014 marks a distinct advance in provision for patients having CLL, and likewise having mantle cell lymphoma.

Component B is a BTK inhibitor selected from the following list:
▪ ibrutinib, or a pharmaceutically acceptable salt thereof;
▪ 4-tert-butyl-N-[2-methyl-3-(4-methyl-6-{[4-(morpholin-4-ylcarbonyl)phenyl]amino}-5-oxo-4,5-dihydropyrazin-2-yl)phenyl]benzamide (CGI-1746, CAS 910232-84-7);
▪ N-{3-[(5-fluoro-2-{[4-(2-methoxyethoxy)phenyl]amino}pyrimidin-4-yl)amino]phenyl}acrylamide (AVL-292, CAS 1202757-89-8);
▪ 6-cyclopropyl-8-fluoro-2-[2-(hydroxymethyl)-3-(1-methyl-5-{[5-(4-methylpiperazin-1-yl)pyridin-2-yl]amino}-6-oxo-1,6-dihydropyridin-3-yl)phenyl]isoquinolin-1(2H)-one (RN486, CAS 1242156-23-5).

### IBRUTINIB :

Ibrutinib (USAN ("United States Adopted Name")), also known as PCI-32765, is 1-{(3R)-3-[4-amino-3-(4-phenoxyphenyl)-1H-pyrazolo[3,4-d]pyrimidin-1-yl]piperidin-1-yl}prop-2-en-1-one
(CAS Registry Number 936563-96-1) of the formula (II) : 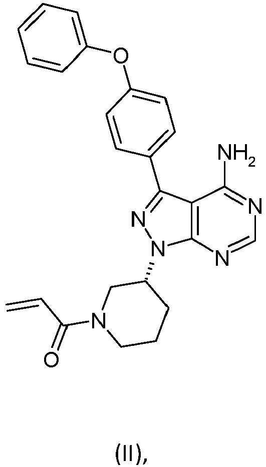 (IBRUTINIB, PCI-32765),
and is referred to hereinafter as "ibrutinib".

Ibrutinib (formerly PCI-32765, from Pharmacyclics and Janssen Pharmaceutica) is a medicament from the group of the tyrosine kinase inhibitors which are used under the *Imbruvica* trade name for treatment of mantle cell lymphoma.

Ibrutinib is a tyrosine kinase inhibitor to be taken orally, which inhibits Bruton tyrosine kinase (*BTK*)*.* The latter plays a central role in intracellular signal transmission in B lymphocytes. The envisaged clinical field of use of ibrutinib is therefore malignant B-cell disorders, in the narrower sense B-cell non-Hodgkin's lymphomas, but also autoimmune disorders in which B cells play a role, such as rheumatoid arthritis.

Ibrutinib showed efficacy in the case of intensively pretreated patients having treatment-resistant chronic lymphatic leukaemia (CLL) or having mantle cell lymphoma (Ibrutinib: Kinase-Inhibitor gegen B-Zell-Malignome aktiv. Deutsches Ärzteblatt, 20 June 2013, retrieved on 23 July 2013). Ibrutinib was approved on 13 November 2013 by the FDA for the treatment of mantle cell lymphoma. The trade name in the United States is Imbruvica. Ibrutinib was approved in February 2014 by the FDA for the treatment of CLL.

In July 2014, the Committee for Medicinal Products for Human Use of the European Medicines Agency (EMA) recommended ibrutinib for approval for the indication of chronic lymphatic leukaemia (CLL). Ibrutinib additionally received a recommendation for approval for the indication of mantle cell lymphoma, and Zydelig for the indication of follicular lymphoma (FL).

See also the following references relating to ibrutinib:
Ibrutinib: Kinase-Inhibitor gegen B-Zell-Malignome aktiv. Deutsches Ärzteblatt, 20 June 2013, retrieved on 23 July 2013.
Ibrutinib Receives Two Oncology Breakthrough Therapy Designations from U.S. Food and Drug Administration. prnewswire.com, 12 February 2013, retrieved on 29 July 2013 (English).
Ibrutinib is specified as a compound per se as compound 14 in European Patent EP 2,201,840 B1 and in US Patent US 7,514,444 B2.
However, the prior art does not contain any combinations as described in the present invention, containing an IRAK4-inhibiting compound of the formula (I) as defined herein, or a salt, a solvate or a solvate of a salt thereof, and ibrutinib, or a pharmaceutically acceptable salt thereof.

### CGI-1746:

The substance CGI-1746 (CAS Registry Number 910232-84-7) has been described in J. A. Di Paolo et al, Nature Chemical Biology, 2011, 7, 1, 41 - 50, DOI:10.1038/nchembio.481 to be a specific BTK-inhibitor (for the preparation see also the supplementary information). 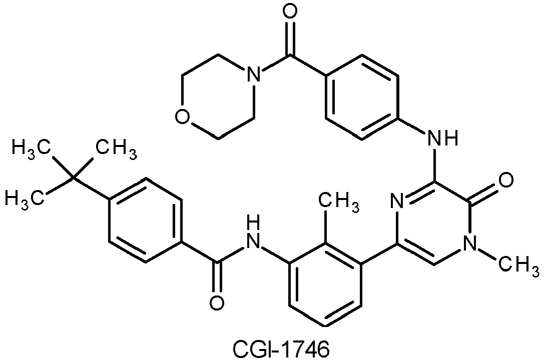 However, the prior art does not contain any combinations as described in the present invention, containing an IRAK4-inhibiting compound of the formula (I) as defined herein, or a salt, a solvate or a solvate of a salt thereof, and CGI-1746, or a pharmaceutically acceptable salt thereof.

### AVL-292:

The substance AVL-292 (CAS Registry Number 1202757-89-8) has been described as an inhibitor of BTK in WO2009158571. In addition, the preparation of AVL-292 has been described.

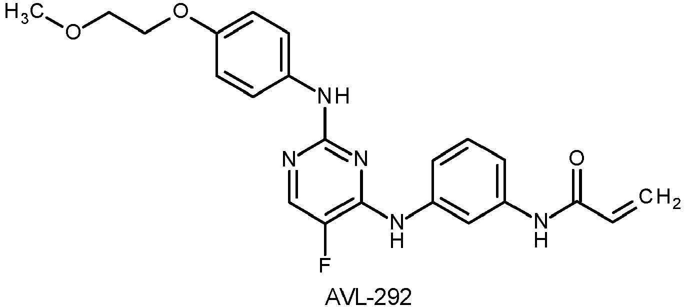

However, the prior art does not contain any combinations as described in the present invention, containing an IRAK4-inhibiting compound of the formula (I) as defined herein, or a salt, a solvate or a solvate of a salt thereof, and AVL-292, or a pharmaceutically acceptable salt thereof.

### RN486:

The BTK-inhibitor RN486 (CAS Registry Number 1242156-23-6, in the present text the term "RN-486" is also used) has been described in L. Yan et al, J. Med. Chem., 2015, 58, 512-516 to be suitable for the treatment of rheumatoid arthritis.

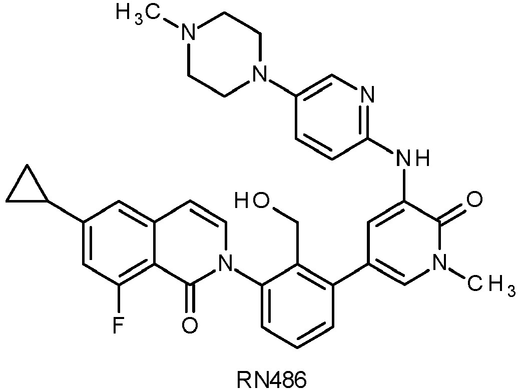

However, the prior art does not contain any combinations as described in the present invention, containing an IRAK4-inhibiting compound of the formula (I) as defined herein, or a salt, a solvate or a solvate of a salt thereof, and RN486, or a pharmaceutically acceptable salt thereof.

### SUMMARY OF THE INVENTION

It has been found that, surprisingly, a synergistic anti-proliferative effect occurs in tumour cell lines when an IRAK4 inhibitor of the formula (I) as defined herein is used in combination with the BTK inhibitor ibrutinib.

A first aspect of the present invention relates to combinations of at least two components, component A and component B:
- a component A, which is an IRAK4-inhibiting compound, which is:
   1) N-[6-(2-hydroxypropan-2-yl)-2-(2-methoxyethyl)-2H-indazol-5-yl]-6-(trifluoromethyl)pyridine-2-carboxamide;
   2) N-[6-(hydroxymethyl)-2-(2-methoxyethyl)-2H-indazol-5-yl]-6-(trifluoromethyl)pyridine-2-carboxamide;
   3) N-[6-(2-hydroxypropan-2-yl)-2-(3-methoxypropyl)-2H-indazol-5-yl]-6-(trifluoromethyl)pyridine-2-carboxamide;
   4) N-[6-(hydroxymethyl)-2-(3-methoxypropyl)-2H-indazol-5-yl]-6-(trifluoromethyl)pyridine-2-carboxamide;
   5) N-[2-(2-hydroxyethyl)-6-(2-hydroxypropan-2-yl)-2H-indazol-5-yl]-6-(trifluoromethyl)pyridine-2-carboxamide;
   6) N-[6-(2-hydroxypropan-2-yl)-2-(3-hydroxypropyl)-2H-indazol-5-yl]-6-(trifluoromethyl)pyridine-2-carboxamide;
   7) N-[2-(2-hydroxyethyl)-6-(hydroxymethyl)-2H-indazol-5-yl]-6-(trifluoromethyl)pyridine-2-carboxamide;
   8) N-[6-(2-hydroxypropan-2-yl)-2-(oxetan-3-ylmethyl)-2H-indazol-5-yl]-6-(trifluoromethyl)pyridine-2-carboxamide;
   9) N-[6-(hydroxymethyl)-2-(oxetan-3-ylmethyl)-2H-indazol-5-yl]-6-(trifluoromethyl)pyridine-2-carboxamide;
   10) N-{6-(2-hydroxypropan-2-yl)-2-[3-(methylsulphonyl)propyl]-2H-indazol-5-yl}-6-(trifluoromethyl)pyridine-2-carboxamide;
   11) N-[2-(3-hydroxy-3-methylbutyl)-6-(2-hydroxypropan-2-yl)-2H-indazol-5-yl]-6-(trifluoromethyl)pyridine-2-carboxamide;
   12) N-{6-(2-hydroxypropan-2-yl)-2-[2-(methylsulphonyl)ethyl]-2H-indazol-5-yl}-6-(trifluoromethyl)pyridine-2-carboxamide;
   13) 6-(difluoromethyl)-N-[2-(3-hydroxy-3-methylbutyl)-6-(2-hydroxypropan-2-yl)-2H-indazol-5-yl]pyridine-2-carboxamide;
   14) 6-(difluoromethyl)-N-{6-(2-hydroxypropan-2-yl)-2-[2-(methylsulphonyl)ethyl]-2H-indazol-5-yl}pyridine-2-carboxamide;
   15) 6-(difluoromethyl)-N-[6-(2-hydroxypropan-2-yl)-2-(3-hydroxypropyl)-2H-indazol-5-yl]pyridine-2-carboxamide;
   16) N-[6-(2-hydroxypropan-2-yl)-2-(4,4,4-trifluorobutyl)-2H-indazol-5-yl]-6-(trifluoromethyl)pyridine-2-carboxamide;
   17) N-{6-(2-hydroxypropan-2-yl)-2-[3-(trifluoromethoxy)propyl]-2H-indazol-5-yl}-6-(trifluoromethyl)pyridine-2-carboxamide;
   18) N-{6-(2-hydroxypropan-2-yl)-2-[3-(2,2,2-trifluoroethoxy)propyl]-2H-indazol-5-yl}-6-(trifluoromethyl)pyridine-2-carboxamide;
   19) 5-fluoro-N-[2-(3-hydroxy-3-methylbutyl)-6-(2-hydroxypropan-2-yl)-2H-indazol-5-yl]-6-methylpyridine-2-carboxamide;
   20) N-[2-(3-hydroxy-3-methylbutyl)-6-(2-hydroxypropan-2-yl)-2H-indazol-5-yl]-6-methylpyridine-2-carboxamide ; or
   21) 6-(2-hydroxypropan-2-yl)-N-[6-(2-hydroxypropan-2-yl)-2-(4,4,4-trifluorobutyl)-2H-indazol-5-yl]pyridine-2-carboxamide;
   or a salt, a solvate, or a solvate of said salt, thereof;
and
- a component B, which is a BTK-inhibiting compound selected from the following list:
   ∘ ibrutinib, or a pharmaceutically acceptable salt thereof;
   ∘ 4-tert-butyl-N-[2-methyl-3-(4-methyl-6-{[4-(morpholin-4-ylcarbonyl)phenyl]amino}-5-oxo-4,5-dihydropyrazin-2-yl)phenyl]benzamide (CGI-1746, CAS 910232-84-7) ;
   ∘ N-{3-[(5-fluoro-2-{[4-(2-methoxyethoxy)phenyl]amino}pyrimidin-4-yl)amino]phenyl}acrylamide (AVL-292, CAS 1202757-89-8); or
   ∘ 6-cyclopropyl-8-fluoro-2-[2-(hydroxymethyl)-3-(1-methyl-5-{[5-(4-methylpiperazin-1-yl)pyridin-2-yl]amino}-6-oxo-1,6-dihydropyridin-3-yl)phenyl]isoquinolin-1(2H)-one (RN486, CAS 1242156-23-5)];
for use in the treatment and/or prophylaxis of non-Hodgkin's lymphoma (abbreviated to "NHL"), especially primary therapy or secondary therapy of recurrent or refractory, indolent or aggressive non-Hodgkin's lymphoma (NHL), especially of follicular lymphoma (abbreviated to "FL"), of chronic lymphatic leukaemia (abbreviated to "CLL"), of marginal-zone lymphoma (abbreviated to "MZL"), of diffuse large-cell B-cell lymphoma (abbreviated to "DLBCL"), especially of activated B-cell-like diffuse large-cell B-cell lymphoma (abbreviated to "ABC-DLBCL"), of mantle cell lymphoma (abbreviated to "MCL"), of transformed lymphoma (abbreviated to "TL"), of peripheral T-cell lymphoma (abbreviated to "PTCL") or of lymphoplasmacytic lymphoma (Waldenström's macroglobulinaemia (abbreviated to "WM")).

A second aspect of the present invention relates to combinations of at least two components A and B:
- a component A, which is an IRAK4-inhibiting compound, which is:
   1) N-[6-(2-hydroxypropan-2-yl)-2-(2-methoxyethyl)-2H-indazol-5-yl]-6-(trifluoromethyl)pyridine-2-carboxamide;
   2) N-[6-(hydroxymethyl)-2-(2-methoxyethyl)-2H-indazol-5-yl]-6-(trifluoromethyl)pyridine-2-carboxamide;
   3) N-[6-(2-hydroxypropan-2-yl)-2-(3-methoxypropyl)-2H-indazol-5-yl]-6-(trifluoromethyl)pyridine-2-carboxamide;
   4) N-[6-(hydroxymethyl)-2-(3-methoxypropyl)-2H-indazol-5-yl]-6-(trifluoromethyl)pyridine-2-carboxamide;
   5) N-[2-(2-hydroxyethyl)-6-(2-hydroxypropan-2-yl)-2H-indazol-5-yl]-6-(trifluoromethyl)pyridine-2-carboxamide;
   6) N-[6-(2-hydroxypropan-2-yl)-2-(3-hydroxypropyl)-2H-indazol-5-yl]-6-(trifluoromethyl)pyridine-2-carboxamide;
   7) N-[2-(2-hydroxyethyl)-6-(hydroxymethyl)-2H-indazol-5-yl]-6-(trifluoromethyl)pyridine-2-carboxamide;
   8) N-[6-(2-hydroxypropan-2-yl)-2-(oxetan-3-ylmethyl)-2H-indazol-5-yl]-6-(trifluoromethyl)pyridine-2-carboxamide;
   9) N-[6-(hydroxymethyl)-2-(oxetan-3-ylmethyl)-2H-indazol-5-yl]-6-(trifluoromethyl)pyridine-2-carboxamide;
   10) N-{6-(2-hydroxypropan-2-yl)-2-[3-(methylsulphonyl)propyl]-2H-indazol-5-yl}-6-(trifluoromethyl)pyridine-2-carboxamide;
   11) N-[2-(3-hydroxy-3-methyl butyl)-6-(2-hydroxypropan-2-yl)-2H-indazol-5-yl]-6-(trifluoromethyl)pyridine-2-carboxamide;
   12) N-{6-(2-hydroxypropan-2-yl)-2-[2-(methylsulphonyl)ethyl]-2H-indazol-5-yl}-6-(trifluoromethyl)pyridine-2-carboxamide;
   13) 6-(difluoromethyl)-N-[2-(3-hydroxy-3-methyl butyl)-6-(2-hydroxypropan-2-yl)-2H-indazol-5-yl]pyridine-2-carboxamide;
   14) 6-(difluoromethyl)-N-{6-(2-hydroxypropan-2-yl)-2-[2-(methylsulphonyl)ethyl]-2H-indazol-5-yl}pyridine-2-carboxamide;
   15) 6-(difluoromethyl)-N-[6-(2-hydroxypropan-2-yl)-2-(3-hydroxypropyl)-2H-indazol-5-yl]pyridine-2-carboxamide;
   16) N-[6-(2-hydroxypropan-2-yl)-2-(4,4,4-trifluorobutyl)-2H-indazol-5-yl]-6-(trifluoromethyl)pyridine-2-carboxamide;
   17) N-{6-(2-hydroxypropan-2-yl)-2-[3-(trifluoromethoxy)propyl]-2H-indazol-5-yl}-6-(trifluoromethyl)pyridine-2-carboxamide;
   18) N-{6-(2-hydroxypropan-2-yl)-2-[3-(2,2,2-trifluoroethoxy)propyl]-2H-indazol-5-yl}-6-(trifluoromethyl)pyridine-2-carboxamide;
   19) 5-fluoro-N-[2-(3-hydroxy-3-methyl butyl)-6-(2-hydroxypropan-2-yl)-2H-indazol-5-yl]-6-methylpyridine-2-carboxamide;
   20) N-[2-(3-hydroxy-3-methylbutyl)-6-(2-hydroxypropan-2-yl)-2H-indazol-5-yl]-6-methylpyridine-2-carboxamide ; or
   21) 6-(2-hydroxypropan-2-yl)-N-[6-(2-hydroxypropan-2-yl)-2-(4,4,4-trifluorobutyl)-2H-indazol-5-yl]pyridine-2-carboxamide;
   or a salt, a solvate, or a solvate of said salt, thereof;
and
- a component B, which is a BTK-inhibiting compound, which is:
   ∘ ibrutinib, or a pharmaceutically acceptable salt thereof;
for use in the treatment and/or prophylaxis of non-Hodgkin's lymphoma (abbreviated to "NHL"), especially primary therapy or secondary therapy of recurrent or refractory, indolent or aggressive non-Hodgkin's lymphoma (NHL), especially of follicular lymphoma (abbreviated to "FL"), of chronic lymphatic leukaemia (abbreviated to "CLL"), of marginal-zone lymphoma (abbreviated to "MZL"), of diffuse large-cell B-cell lymphoma (abbreviated to "DLBCL"), especially of activated B-cell-like diffuse large-cell B-cell lymphoma (abbreviated to "ABC-DLBCL"), of mantle cell lymphoma (abbreviated to "MCL"), of transformed lymphoma (abbreviated to "TL"), of peripheral T-cell lymphoma (abbreviated to "PTCL") or of lymphoplasmacytic lymphoma (Waldenström's macroglobulinaemia (abbreviated to "WM")).

The combinations of at least two components A and B as described and defined herein are also referred to as "combinations of the present invention".

The present invention further relates to a kit comprising a combination of:
- component A, which consists of an IRAK4-inhibiting compound as defined in the claims, or a salt, a solvate or a solvate of a salt thereof;
- component B, which is a BTK-inhibiting compound as defined in the claims, for example ibrutinib, or a pharmaceutically acceptable salt thereof;
and optionally
- component C, which consists of one or more pharmaceutical products as defined in the claims. One or two of the above-defined compounds A and B in any of the above-described combinations can be present in a pharmaceutical formulation/composition ready for simultaneous, separate or sequential administration. The components may each independently be administered via an oral, intravenous, topical, intraperitoneal or nasal route, or as a depot.

The combinations are intended to be especially suitable for treatment and for prevention of proliferative and inflammatory disorders characterized by an overreacting immune system. Particular mention should be made here of inflammatory skin disorders, cardiovascular disorders, lung disorders, eye disorders, autoimmune disorders, gynaecological disorders, especially endometriosis, and cancer.

The combinations are intended to be particularly suitable for the treatment of cancer. The combinations are intended to be very particularly suitable for the treatment of the following types of cancer: non-Hodgkin's lymphoma (abbreviated to "NHL"), especially primary therapy or secondary therapy of recurrent or refractory, indolent or aggressive non-Hodgkin's lymphoma (NHL), especially of follicular lymphoma (abbreviated to "FL"), of chronic lymphatic leukaemia (abbreviated to "CLL"), of marginal-zone lymphoma (abbreviated to "MZL"), of diffuse large-cell B-cell lymphoma (abbreviated to "DLBCL"), especially of activated B-cell-like diffuse large-cell B-cell lymphoma (abbreviated to "ABC-DLBCL"), of mantle cell lymphoma (abbreviated to "MCL"), of transformed lymphoma (abbreviated to "TL"), of peripheral T-cell lymphoma (abbreviated to "PTCL") or of lymphoplasmacytic lymphoma (Waldenström's macroglobulinaemia (abbreviated to "WM")).

### COMPONENT A OF THE COMBINATION

Component A compounds are as defined in the claims.

In the case of the synthesis intermediates and working examples of the component A compounds of the combinations of the invention described hereinafter, any compound specified in the form of a salt of the corresponding base or acid is generally a salt of unknown exact stoichiometric composition, as obtained by the respective preparation and/or purification process. Unless specified in more detail, additions to names and structural formulae, such as "hydrochloride", "trifluoroacetate", "sodium salt" or "x HCl", "x CF₃COOH", "x Na⁺" should not be understood in a stoichiometric sense in the case of such salts, but have merely descriptive character with regard to the salt-forming components present therein.

This applies correspondingly if synthesis intermediates or working examples of the component A compounds of the inventive combinations, or salts thereof, were obtained in the form of solvates, for example hydrates, of unknown stoichiometric composition (if they are of a defined type) by the preparation and/or purification processes described.

Preferred salts in the context of the present invention are physiologically acceptable salts of the compounds. Salts which are not themselves suitable for pharmaceutical applications can be used, for example, for the isolation or purification of the compounds.

Physiologically acceptable salts of the compounds include acid addition salts of mineral acids, carboxylic acids and sulphonic acids, for example salts of hydrochloric acid, hydrobromic acid, sulphuric acid, phosphoric acid, methanesulphonic acid, ethanesulphonic acid, toluenesulphonic acid, benzenesulphonic acid, naphthalenedisulphonic acid, acetic acid, trifluoroacetic acid, propionic acid, lactic acid, tartaric acid, malic acid, citric acid, fumaric acid, maleic acid and benzoic acid.

Physiologically acceptable salts of the compounds also include salts of conventional bases, by way of example and with preference alkali metal salts (e.g. sodium and potassium salts), alkaline earth metal salts (e.g. calcium and magnesium salts) and ammonium salts derived from ammonia or organic amines having 1 to 16 carbon atoms, by way of example and with preference ethylamine, diethylamine, triethylamine, ethyldiisopropylamine, monoethanolamine, diethanolamine, triethanolamine, dicyclohexylamine, dimethylaminoethanol, procaine, dibenzylamine, N-methylmorpholine, arginine, lysine, ethylenediamine and N-methylpiperidine.

Solvates in the context of the invention are described as those forms of the compounds which form a complex in the solid or liquid state by coordination with solvent molecules. Hydrates are a specific form of the solvates in which the coordination is with water.

If the component A compounds as defined in the claims as constituents of the inventive combinations can occur in tautomeric forms, the present invention encompasses all the tautomeric forms.

The present invention provides the following component A compounds as defined in the claims as constituents of the inventive combinations:
1) N-[6-(2-hydroxypropan-2-yl)-2-(2-methoxyethyl)-2H-indazol-5-yl]-6-(trifluoromethyl)pyridine-2-carboxamide
2) N-[6-(hydroxymethyl)-2-(2-methoxyethyl)-2H-indazol-5-yl]-6-(trifluoromethyl)pyridine-2-carboxamide
3) N-[6-(2-hydroxypropan-2-yl)-2-(3-methoxypropyl)-2H-indazol-5-yl]-6-(trifluoromethyl)pyridine-2-carboxamide
4) N-[6-(hydroxymethyl)-2-(3-methoxypropyl)-2H-indazol-5-yl]-6-(trifluoromethyl)pyridine-2-carboxamide
5) N-[2-(2-hydroxyethyl)-6-(2-hydroxypropan-2-yl)-2H-indazol-5-yl]-6-(trifluoromethyl)pyridine-2-carboxamide
6) N-[6-(2-hydroxypropan-2-yl)-2-(3-hydroxypropyl)-2H-indazol-5-yl]-6-(trifluoromethyl)pyridine-2-carboxamide
7) N-[2-(2-hydroxyethyl)-6-(hydroxymethyl)-2H-indazol-5-yl]-6-(trifluoromethyl)pyridine-2-carboxamide
8) N-[6-(2-hydroxypropan-2-yl)-2-(oxetan-3-ylmethyl)-2H-indazol-5-yl]-6-(trifluoromethyl)pyridine-2-carboxamide
9) N-[6-(hydroxymethyl)-2-(oxetan-3-ylmethyl)-2H-indazol-5-yl]-6-(trifluoromethyl)pyridine-2-carboxamide
10) N-{6-(2-hydroxypropan-2-yl)-2-[3-(methylsulphonyl)propyl]-2H-indazol-5-yl}-6-(trifluoromethyl)pyridine-2-carboxamide
11) N-[2-(3-hydroxy-3-methylbutyl)-6-(2-hydroxypropan-2-yl)-2H-indazol-5-yl]-6-(trifluoromethyl)pyridine-2-carboxamide
12) N-{6-(2-hydroxypropan-2-yl)-2-[2-(methylsulphonyl)ethyl]-2H-indazol-5-yl}-6-(trifluoromethyl)pyridine-2-carboxamide
13) 6-(difluoromethyl)-N-[2-(3-hydroxy-3-methylbutyl)-6-(2-hydroxypropan-2-yl)-2H-indazol-5-yl]pyridine-2-carrboxamide
14) 6-(difluoromethyl)-N-{6-(2-hydroxypropan-2-yl)-2-[2-(methylsulphonyl)ethyl]-2H-indazol-5-yl}pyridine-2-carboxamide
15) 6-(difluoromethyl)-N-[6-(2-hydroxypropan-2-yl)-2-(3-hydroxypropyl)-2H-indazol-5-yl]pyridine-2-carboxamide
16) N-[6-(2-hydroxypropan-2-yl)-2-(4,4,4-trifluorobutyl)-2H-indazol-5-yl]-6-(trifluoromethyl)pyridine-2-carboxamide
17) N-{6-(2-hydroxypropan-2-yl)-2-[3-(trifluoromethoxy)propyl]-2H-indazol-5-yl}-6-(trifluoromethyl)pyridine-2-carboxamide
18) N-{6-(2-hydroxypropan-2-yl)-2-[3-(2,2,2-trifluoroethoxy)propyl]-2H-indazol-5-yl}-6-(trifluoromethyl)pyridine-2-carboxamide
19) 5-fluoro-N-[2-(3-hydroxy-3-methylbutyl)-6-(2-hydroxypropan-2-yl)-2H-indazol-5-yl]-6-methylpyridine-2-carboxamide
20) N-[2-(3-hydroxy-3-methylbutyl)-6-(2-hydroxypropan-2-yl)-2H-indazol-5-yl]-6-methylpyridine-2-carboxamide
21) 6-(2-hydroxypropan-2-yl)-N-[6-(2-hydroxypropan-2-yl)-2-(4,4,4-trifluorobutyl)-2H-indazol-5-yl]pyridine-2-carboxamide.

The component A compounds of the inventive combinations act as inhibitors of IRAK4 kinase and have a valuable spectrum of pharmacological activity.

The present invention provides the inventive combinations for use in treatment and/or prophylaxis of diseases in man and animals.

Very particular preference is given to the treatment and/or prophylaxis of diseases which are caused by uncontrolled cell growth, cell proliferation and/or cell survival, a disproportionate cellular immune response or a disproportionate cellular inflammatory reaction, for example haematological tumours, a solid tumour and/or metastases thereof, for example leukaemias and myelodysplastic syndrome, malignant lymphoma, head and neck tumours including brain tumours and metastases, tumours of the thorax including non-small-cell and small-cell lung tumours, gastrointestinal tumours, endocrine tumours, breast tumours and other gynaecological tumours, urological tumours including kidney, bladder and prostate tumours, skin tumours and sarcoma and/or metastases thereof.

In the context of the present invention, the term "treatment" or "treating" includes inhibition, retardation, checking, alleviating, attenuating, restricting, reducing, suppressing, repelling or healing of a disease, a condition, a disorder, an injury or a health problem, or the development, the course or the progression of such states and/or the symptoms of such states. The term "therapy" is understood here to be synonymous with the term "treatment".

The terms "prevention", "prophylaxis" and "preclusion" are used synonymously in the context of the present invention and refer to the avoidance or reduction of the risk of contracting, experiencing, suffering from or having a disease, a condition, a disorder, an injury or a health problem, or a development or advancement of such states and/or the symptoms of such states.

The treatment or prevention of a disease, a condition, a disorder, an injury or a health problem may be partial or complete.

The inventive combinations can be used alone or, if required, in combination with one or more other pharmaceutical products (referred to herein as "component C") as defined in the claims, provided that this combination does not lead to undesirable and unacceptable side effects. The present invention therefore further provides medicaments comprising an inventive combination and one or more further active ingredients, especially for prophylaxis and therapy of the disorders mentioned above.

For example, the inventive combinations can be combined with known antihyperproliferative, cytostatic or cytotoxic substances for treatment of cancer. The combination of the inventive combinations with other substances commonly used for cancer treatment, or else with radiotherapy, is particularly appropriate.

Components "C" are pharmaceutical products according to the following list: 131I-chTNT, abarelix, abiraterone, aclarubicin, ado-trastuzumab emtansine, afatinib, aflibercept, aldesleukin, alemtuzumab, alendronic acid, alitretinoin, altretamine, amifostine, aminoglutethimide, hexyl-5-aminolevulinate, amrubicin, amsacrine, anastrozole, ancestim, anethole dithiolethione, angiotensin II, antithrombin III, aprepitant, arcitumomab, arglabin, arsenic trioxide, asparaginase, axitinib, azacitidine, belotecan, bendamustine, belinostat, bevacizumab, bexarotene, bicalutamide, bisantrene, bleomycin, bortezomib, buserelin, bosutinib, brentuximab vedotin, busulfan, cabazitaxel, cabozantinib, calcium folinate, calcium levofolinate, capecitabine, capromab, carboplatin, carfilzomib, carmofur, carmustine, catumaxomab, celecoxib, celmoleukin, ceritinib, cetuximab, chlorambucil, chlormadinone, chlormethine, cidofovir, cinacalcet, cisplatin, cladribine, clodronic acid, clofarabine, copanlisib, crisantaspase, crizotinib, cyclophosphamide, cyproterone, cytarabine, dacarbazine, dactinomycin, dabrafenib, dasatinib, daunorubicin, decitabine, degarelix, denileukin-diftitox, denosumab, depreotide, deslorelin, dexrazoxane, dibrospidium chloride, dianhydrogalactitol, diclofenac, docetaxel, dolasetron, doxifluridine, doxorubicin, doxorubicin + estrone, dronabinol, edrecolomab, elliptinium acetate, endostatin, enocitabine, enzalutamide, epirubicin, epitiostanol, epoetin-alfa, epoetin-beta, epoetin-zeta, eptaplatin, eribulin, erlotinib, esomeprazole, estramustine, etoposide, everolimus, exemestane, fadrozole, fentanyl, fluoxymesterone, floxuridine, fludarabine, fluorouracil, flutamide, folic acid, formestane, fosaprepitant, fotemustine, fulvestrant, gadobutrol, gadoteridol, gadoteric acid meglumine salt, gadoversetamide, gadoxetic acid disodium salt (gd-EOB-DTPA disodium salt), gallium nitrate, ganirelix, gefitinib, gemcitabine, gemtuzumab, glucarpidase, glutoxim, goserelin, granisetron, granulocyte colony stimulating factor (G-CSF), granulocyte macrophage colony stimulating factor (GM-CSF), histamine dihydrochloride, histrelin, hydroxycarbamide, I-125 seeds, ibandronic acid, ibritumomab-tiuxetan, idarubicin, ifosfamide, imatinib, imiquimod, improsulfan, indisetron, incadronic acid, ingenol mebutate, interferon-alfa, interferon-beta, interferon-gamma, iobitridol, iobenguane I-123, iomeprol, ipilimumab, irinotecan, itraconazole, ixabepilone, lanreotide, lansoprazole, lapatinib, lasocholine, lenalidomide, lentinan, letrozole, leuprorelin, levamisole, levonorgestrel, levothyroxin-sodium, lipegfilgrastim, lisuride, lobaplatin, lomustine, lonidamine, masoprocol, medroxyprogesteron, megestrol, melarsoprol, melphalan, mepitiostan, mercaptopurine, mesna, methadone, methotrexate, methoxsalen, methylaminolevulinate, methylprednisolone, methyltestosterone, metirosine, mifamurtide, miltefosine, miriplatin, mitobronitol, mitoguazone, mitolactol, mitomycin, mitotan, mitoxantrone, mogamulizumab, molgramostim, mopidamol, morphine hydrochloride, morphine sulfate, nabilone, nabiximols, nafarelin, naloxone + pentazocine, naltrexone, nartograstim, nedaplatin, nelarabine, neridronic acid, nivolumab pentetreotide, nilotinib, nilutamide, nimorazole, nimotuzumab, nimustine, nitracrine, nivolumab, obinutuzumab, octreotide, ofatumumab, omacetaxin mepesuccinate, omeprazole, ondansetron, orgotein, orilotimod, oxaliplatin, oxycodone, oxymetholone, ozogamicin, p53 gene therapy, paclitaxel, palladium-103 seed, palonosetron, pamidronic acid, panitumumab, pantoprazole, pazopanib, pegaspargase, pembrolizumab, Peg-interferon alfa-2b, pemetrexed, pentostatin, peplomycin, perflubutane, perfosfamide, pertuzumab, picibanil, pilocarpine, pirarubicin, pixantron, plerixafor, plicamycin, poliglusam, polyoestradiol phosphate, polyvinylpyrrolidone + sodium hyaluronate, polysaccharide-K, pomalidomide, ponatinib, porfimer-sodium, pralatrexate, prednimustine, prednisone, procarbazine, procodazole, propranolol, quinagolide, rabeprazole, racotumomab, radium-223 chloride, radotinib, raloxifene, raltitrexed, ramosetron, ramucirumab, ranimustine, rasburicase, razoxan, refametinib, regorafenib, risedronic acid, rhenium-186 etidronate, rituximab, romidepsin, romurtid, roniciclib, samarium-153 lexidronam, satumomab, secretin, sipuleucel-T, sizofiran, sobuzoxane, sodium glycididazole, sorafenib, stanozolol, streptozocin, sunitinib, talaporfin, tamibarotene, tamoxifen, tapentadol, tasonermin, teceleukin, technetium-99m nofetumomab merpentan, 99mTc-HYNIC-[Tyr3]-octreotide, tegafur, tegafur + gimeracil + oteracil, temoporfin, temozolomide, temsirolimus, teniposide, testosterone, tetrofosmin, thalidomide, thiotepa, thymalfasin, thyrotropin alfa, tioguanine, tocilizumab, topotecan, toremifene, tositumomab, trabectedin, tramadol, trastuzumab, treosulfan, tretinoin, trifluridine + tipiracil, trametinib, trilostane, triptorelin, trofosfamide, thrombopoietin, ubenimex, valrubicin, vandetanib, vapreotide, vatalanib, vemurafenib, vinblastine, vincristine, vindesine, vinflunine, vinorelbine, vismodegib, vorinostat, yttrium-90 glass microbeads, zinostatin, zinostatin stimalamer, zoledronic acid, zorubicin.

The inventive combinations can also achieve positive effects in combination with other therapies directed against angiogenesis, for example with bevacizumab, axitinib, regorafenib, cediranib, sorafenib, sunitinib or thalidomide. Combinations with antihormones and steroidal metabolic enzyme inhibitors are particularly suitable because of their favourable profile of side effects.

Generally, the following aims can be pursued with the combination of the inventive combinations with other cytostatically or cytotoxically active agents:
- improved efficacy in slowing the growth of a tumour, in reducing its size or even in completely eliminating it, compared with treatment with an individual active ingredient;
- the possibility of using the chemotherapeutics used in a lower dosage than in the case of monotherapy;
- the possibility of a more tolerable therapy with fewer side effects compared with individual administration;
- the possibility of treatment of a broader spectrum of neoplastic disorders;
- the achievement of a higher rate of response to the therapy;
- a longer survival time of the patient compared with present-day standard therapy.

In addition, the inventive combinations can also be used in conjunction with radiotherapy and/or surgical intervention.

The inventive combinations may act systemically and/or locally. For this purpose, they can be administered in a suitable manner, for example by the oral, parenteral, pulmonal, nasal, sublingual, lingual, buccal, rectal, dermal, transdermal or conjunctival route, via the ear or as an implant or stent.

The inventive combinations can be administered in suitable administration forms for these administration routes.

Suitable administration forms for oral administration are those which work according to the prior art and release the inventive combinations rapidly and/or in a modified manner and which contain the inventive combinations in crystalline and/or amorphized and/or dissolved form, for example tablets (uncoated or coated tablets, for example with gastric juice-resistant or retarded-dissolution or insoluble coatings which control the release of the inventive combinations), tablets or films/oblates which disintegrate rapidly in the oral cavity, films/lyophilizates, capsules (for example hard or soft gelatin capsules), sugar-coated tablets, granules, pellets, powders, emulsions, suspensions, aerosols or solutions.

Parenteral administration can be effected with bypassing of an absorption step (e.g. intravenously, intraarterially, intracardially, intraspinally or intralumbally) or with inclusion of an absorption (e.g. intramuscularly, subcutaneously, intracutaneously, percutaneously or intraperitoneally). Administration forms suitable for parenteral administration include preparations for injection and infusion in the form of solutions, suspensions, emulsions, lyophilizates or sterile powders.

For the other administration routes, suitable examples are inhalable medicament forms (including powder inhalers, nebulizers), nasal drops, solutions or sprays, tablets, films/oblates or capsules for lingual, sublingual or buccal administration, suppositories, ear or eye preparations, vaginal capsules, aqueous suspensions (lotions, shaking mixtures), lipophilic suspensions, ointments, creams, transdermal therapeutic systems (e.g. patches), milk, pastes, foams, sprinkling powders, implants or stents.

Preference is given to oral or parenteral administration, especially oral administration.

The inventive combinations can be converted to the administration forms mentioned. This can be accomplished in a manner known per se by mixing with inert, nontoxic, pharmaceutically suitable excipients. These excipients include carriers (for example microcrystalline cellulose, lactose, mannitol), solvents (e.g. liquid polyethylene glycols), emulsifiers and dispersing or wetting agents (for example sodium dodecylsulphate, polyoxysorbitan oleate), binders (for example polyvinylpyrrolidone), synthetic and natural polymers (for example albumin), stabilizers (e.g. antioxidants, for example ascorbic acid), colorants (e.g. inorganic pigments, for example iron oxides) and flavour and/or odour correctants.

In general, it has been found to be advantageous in the case of parenteral administration to administer amounts of about 0.001 to 1 mg/kg, preferably about 0.01 to 0.5 mg/kg, of body weight to achieve effective results. In the case of oral administration the dosage is about 0.01 to 100 mg/kg, preferably about 0.01 to 20 mg/kg and most preferably 0.1 to 10 mg/kg of body weight.

It may nevertheless be necessary in some cases to deviate from the stated amounts, specifically as a function of the body weight, route of administration, individual response to the active ingredient, nature of the preparation and time or interval over which administration takes place. Thus, in some cases less than the abovementioned minimum amount may be sufficient, while in other cases the upper limit mentioned must be exceeded. In the case of administration of greater amounts, it may be advisable to divide them into several individual doses over the day.

The working examples which follow illustrate the invention. The invention is not restricted to the examples.

Unless stated otherwise, the percentages in the tests and examples which follow are percentages by weight; parts are parts by weight. Solvent ratios, dilution ratios and concentration data for the liquid/liquid solutions are based in each case on volume.

### Preparation of the component A compounds as constituents of the inventive combinations

### Synthesis of the component A compounds as constituents of the inventive combinations

### Abbreviations and elucidations

| | |
|---|---|
| DMF | *N*,*N*-dimethylformamide |
| DMSO | dimethyl sulphoxide |
| THF | tetrahydrofuran |
| RT | room temperature |
| HPLC | high-performance liquid chromatography |
| H | hour(s) |
| min | minute(s) |
| UPLC | ultrahigh-performance liquid chromatography |
| DAD | diode array detector |
| ELSD | evaporating light scattering detector |
| ESI | electrospray ionization |
| SQD | single quadrupole detector |
| CPG | core-pulled precision glass |

The term sodium chloride solution always means a saturated aqueous sodium chloride solution.

The chemical names of the intermediates and examples were generated using the ACD / LABS (Batch Version 12.01.) software.

### Methods

In some cases, the compounds and precursors and/or intermediates thereof were analysed by LC-MS.

### Method A1: UPLC (MeCN-HCOOH):

Instrument: Waters Acquity UPLC-MS SQD 3001; column: Acquity UPLC BEH C18 1.7 50 × 2.1 mm; eluent A: water + 0.1% by vol. of formic acid (99%), eluent B: acetonitrile; gradient: 0-1.6 min 1-99% B, 1.6-2.0 min 99% B; flow rate 0.8 ml/min; temperature: 60°C; injection: 2 µl; DAD scan: 210-400 nm; MS ESI+, ESI-, scan range 160-1000 m/z; ELSD.

### Method A2: UPLC (MeCN-NH₃):

Instrument: Waters Acquity UPLC-MS SQD 3001; column: Acquity UPLC BEH C18 1.7 50 × 2.1 mm; eluent A: water + 0.2% by vol. of ammonia (32%), eluent B: acetonitrile; gradient: 0-1.6 min 1-99% B, 1.6-2.0 min 99% B; flow rate 0.8 ml/min; temperature: 60°C; injection: 2 µl; DAD scan: 210-400 nm; MS ESI+, ESI-, scan range 160-1000 m/z; ELSD.

### Method A3: (LC-MS)

Instrument: Agilent 1290 Infinity LC; column: Acquity UPLC BEH C18 1.7 50 × 2.1 mm; eluent A: water + 0.05% by vol. of formic acid, eluent B: acetonitrile + 0.05% by vol. of formic acid; gradient: 0-1.7 min 2-90% B, 1.7-2.0 min 90% B; flow rate 1.2 ml/min; temperature: 60°C; injection: 2 µl ; DAD scan: 190-390 nm; MS: Agilent TOF 6230.

### Method A4: (LC-MS)

Instrument: Waters Acquity; column: Kinetex (Phenomenex), 50 × 2 mm; eluent A: water + 0.05% by vol. of formic acid, eluent B: acetonitrile + 0.05% by vol. of formic acid; gradient: 0-1.9 min 1-99% B, 1.9-2.1 min 99% B; flow rate 1.5 ml/min; temperature: 60°C; injection: 0.5 µl; DAD scan: 200-400 nm.

In some cases, the compounds of the formula (I) as constituents of the inventive combinations and the precursors and/or intermediates thereof were purified by the following preparative HPLC methods:
Method P1: system: Waters Autopurification system: Pump 2545, Sample Manager 2767, CFO, DAD 2996, ELSD 2424, SQD; column: XBridge C18 5 µm 100 × 30 mm; eluent A: water + 0.1% by vol. of formic acid, eluent B: acetonitrile; gradient: 0-8 min 10-100% B, 8-10 min 100% B; flow: 50 ml/min; temperature: room temperature; solution: max. 250 mg / max. 2.5 ml DMSO or DMF; injection: 1 × 2.5 ml; detection: DAD scan range 210-400 nm; MS ESI+, ESI-, scan range 160-1000 m/z.
Method P2: system: Waters Autopurification system: Pump 254, Sample Manager 2767, CFO, DAD 2996, ELSD 2424, SQD 3100; column: XBridge C18 5 µm 10 × 30 mm; eluent A: water + 0.2% by vol. of ammonia (32%), eluent B: methanol; gradient: 0-8 min 30-70% B; flow: 50 ml/min; temperature: room temperature; detection: DAD scan range 210-400 nm; MS ESI+, ESI-, scan range 160-1000 m/z; ELSD.
Method P3: system: Labomatic, pump: HD-5000, fraction collector: LABOCOL Vario-4000, UV detector: Knauer UVD 2.1S; column: XBridge C18 5 µm 100×30 mm; eluent A: water + 0.2% by vol. of ammonia (25%), eluent B: acetonitrile; gradient: 0-1 min 15% B, 1-6.3 min 15-55% B, 6.3-6.4 min 55-100% B, 6.4-7.4 min 100% B; flow: 60 ml/min; temperature: room temperature; solution: max. 250 mg / 2 ml DMSO; injection: 2 × 2 ml; detection: UV 218 nm; Software: SCPA PrepCon5.
Method P4: system: Labomatic, pump: HD-5000, fraction collector: LABOCOL Vario-4000, UV detector: Knauer UVD 2.1S; column: Chromatorex RP C18 10 µm 125 × 30 mm; eluent A: water + 0.1% by vol. of formic acid, eluent B: acetonitrile; gradient: 0-15 min 65 - 100% B; flow: 60 ml/min; temperature: room temperature; solution: max. 250 mg / 2 ml DMSO; injection: 2 × 2 ml; detection: UV 254 nm; Software: SCPA PrepCon5.
Method P5: system: Sepiatec: Prep SFC100, column: Chiralpak IA 5 µm 250x20 mm; eluent A: carbon dioxide, eluent B: ethanol; gradient: isocratic 20% B; flow: 80 ml/min; temperature: 40°C; solution: max. 250 mg / 2 ml DMSO; injection: 5 × 0.4 mL; detection: UV 254 nm.
Method P6: system: Agilent: Prep 1200, 2 × prep pump, DLA, MWD, Gilson: Liquid Handler 215; column: Chiralcel OJ-H 5 µm 250 × 20 mm; eluent A: hexane, eluent B: ethanol; gradient: isocratic 30% B; flow: 25 ml/min; temperature: 25°C; solution: 187 mg / 8 ml ethanol/methanol; injection: 8 × 1.0 ml; detection: UV 280 nm.
Method P7: system: Labomatic, pump: HD-5000, fraction collector: LABOCOL Vario-4000, UV detector: Knauer UVD 2.1S; column: XBridge C18 5 µm 100 × 30 mm; eluent A: water + 0.1% by vol. of formic acid, eluent B: acetonitrile; gradient: 0-3 min: 65% B isocratic, 3-13 min: 65-100% B; flow: 60 ml/min; temperature: room temperature; solution: max. 250 mg / 2 ml DMSO; injection: 2 × 2 ml; detection: UV 254 nm.
Method P8: system: Agilent: Prep 1200, 2 × prep pump, DLA, MWD, Gilson: Liquid Handler 215; column: Chiralpak IF 5 µm 250 × 20 mm; eluent A: ethanol, eluent B: methanol; gradient: isocratic 50% B; flow: 25 ml/min; temperature: 25°C; solution: 600 mg / 7 ml N,N-dimethylformamide; injection: 10 × 0.7 ml; detection: UV 254 nm.

In some cases, substance mixtures were purified by column chromatography on silica gel.

For preparation of the compounds of the formula (I) as constituents of the inventive combinations and the precursors and/or intermediates thereof, a column chromatography purification ("flash chromatography") was conducted on silica gel using Isolera^{®} devices from Biotage. This involved using cartridges from Biotage, for example the "SNAP Cartridge, KP_SIL" cartridge of different size and "Interchim Puriflash Silica HP 15UM flash column" cartridges from Interchim of different size.

### Starting materials

### Intermediate V2-1

Methyl 6-(2-hydroxypropan-2-yl)pyridine-2-carboxylate 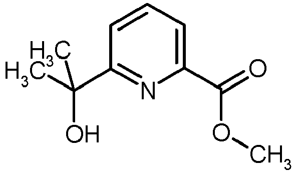 2.00 g (9.26 mmol) of 2-(6-bromopyridin-2-yl)propan-2-ol (CAS 638218-78-7) were dissolved in 20 ml of methanol and 20 ml of DMSO. Subsequently, 250 mg of 1,3-bis(diphenylphosphino)propane, 130 mg of palladium(II) acetate and 3 ml of triethylamine were added. The reaction mixture was purged three times with carbon monoxide at room temperature and stirred under a 13 bar carbon monoxide atmosphere for 30 min. The carbon monoxide atmosphere was removed by applying a vacuum and the mixture was stirred under a 14 bar carbon monoxide atmosphere at 100°C for 24 h. The autoclave was decompressed, water was added to the reaction mixture, and the reaction mixture was extracted three times with ethyl acetate, washed with saturated aqueous sodium hydrogencarbonate solution and sodium chloride solution, filtered through a hydrophobic filter and concentrated. This gave 1.60 g of a crude product.

UPLC-MS (Method A1): Rₜ = 0.76 min (UV detector: TIC), mass found 195.00.

### Intermediate V3-1

Potassium 6-(2-hydroxypropan-2-yl)pyridine-2-carboxylate 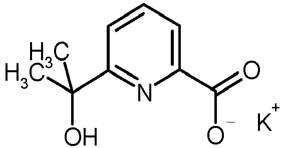 1.60 g of the crude product of Intermediate 0-1 were initially charged in 15 ml of methanol, 0.74 g of potassium hydroxide was added and the mixture was stirred at 50°C for 16.5 h. After concentration, this gave 2.1 g of a solid which was used without further purification. UPLC-MS (Method A1): Rₜ = 0.47 min (UV detector: TIC), mass found 181.00.

### Intermediate 1-1

Methyl 5-nitro-1H-indazole-6-carboxylate 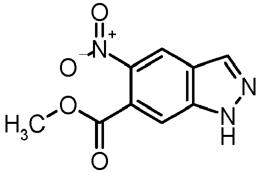 4.60 g (26.1 mmol) of methyl 1H-indazole-6-carboxylate (CAS No: 170487-40-8) were dissolved in 120 ml of sulphuric acid (96%) and cooled to -15°C in a three-neck flask having a CPG stirrer, dropping funnel and internal thermometer. Over a period of 15 min, the nitrating acid (10 ml of 96% sulphuric acid in 5 ml of 65% nitric acid), which had been prepared and cooled beforehand, was added dropwise to this solution. After the dropwise addition had ended, the mixture was stirred for a further 1 h (internal temperature at - 13°C). The reaction mixture was added to ice, and the precipitate formed was filtered off with suction, washed with water and dried in a drying cabinet at 50°C under reduced pressure. 5.49 g of the title compound were obtained.
UPLC-MS (Method A2): Rₜ = 0.75 min
MS (ESIpos): m/z = 222(M+H)⁺
¹H NMR (400 MHz, DMSO-d6): δ [ppm] = 3.87 (s, 3 H), 7.96 (s, 1 H), 8.44 (s, 1 H), 8.70 (s, 1 H), 13.98 (br. s., 1 H).

### Intermediate 2-1

Methyl 5-amino-1H-indazole-6-carboxylate 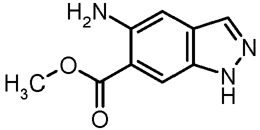 4.40 g (19.8 mmol) of methyl 5-nitro-1H-indazole-6-carboxylate (Intermediate 1-1) were dissolved in 236 ml of methanol and hydrogenated with 1.06 g (0.99 mmol) of palladium on activated carbon under standard hydrogen pressure at 25°C for 3 h. The reaction mixture was filtered through Celite, the filter was washed with methanol, and the filtrate was concentrated. 3.53 g of the title compound were obtained.

¹H NMR (300 MHz, DMSO-d6): δ [ppm] = 3.85 (s, 3 H) 6.01 (s, 2 H) 6.98 (s, 1 H) 7.79 - 7.91 (m, 1 H) 7.99 (s, 1 H) 12.84 (br. s., 1 H).

### Intermediate 3-1

Methyl 5-({[6-(trifluoromethyl)pyridin-2-yl]carbonyl}amino)-1H-indazole-6-carboxylate

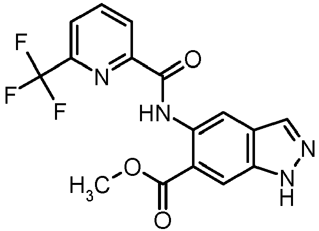

4.95 g (25.9 mmol) of 6-(trifluoromethyl)pyridine-2-carboxylic acid were initially charged in 45 ml of THF. 9.07 g (28.2 mmol) of O-(benzotriazol-1-yl)-N,N,N',N'-tetramethyluronium tetrafluoroborate and 4.92 ml (28.2 mmol) of N-ethyl-N-isopropylpropan-2-amine were added and the mixture was stirred at 25°C for 30 min. Subsequently, 4.50 g (23.5 mmol) of methyl 5-amino-1H-indazole-6-carboxylate (Intermediate 2-1) were added and the mixture was stirred at 25°C for 24 h. The reaction mixture was filtered with suction through a membrane filter and washed with THF and with water, and dried in a drying cabinet overnight. 7.60 g of the title compound were obtained.

UPLC-MS (Method A2): Rₜ = 1.16 min

MS (ESIpos): m/z = 365 (M+H)^{+ 1}H NMR (400 MHz, DMSO-d6): δ [ppm] = 3.97 (s, 3 H), 8.13 - 8.27 (m, 2 H), 8.30 (s, 1 H), 8.33 - 8.45 (m, 1 H), 8.45 - 8.51 (m, 1 H), 9.15 (s, 1 H), 12.57 (s, 1 H), 13.44 (s, 1 H).

### Intermediate 3-2

Methyl 5-({[6-(difluoromethyl)pyridin-2-yl]carbonyl}amino)-1H-indazole-6-carboxylate 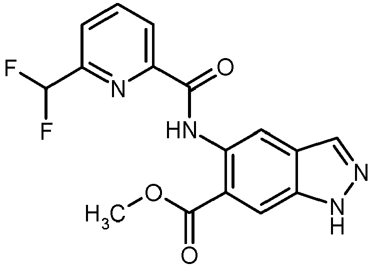 2.85 g (23.5 mmol) of 6-(difluoromethyl)pyridine-2-carboxylic acid were initially charged in 30 ml of THF. 6.05 g (18.8 mmol) of O-(benzotriazol-1-yl)-N,N,N,N-tetramethyluronium tetrafluoroborate and 3.3 ml of N-ethyl-N-isopropylpropan-2-amine were added and the mixture was stirred at room temperature for 10 minutes. Subsequently, 3.00 g (15.7 mmol) of methyl 5-amino-1H-indazole-6-carboxylate were added and the mixture was stirred at room temperature overnight. The reaction mixture was admixed with water, and the precipitate was filtered off with suction and washed repeatedly with water and dichloromethane. This gave 1.53 g (27% of theory) of the title compound. The phases of the filtrate were separated, the organic phase was concentrated, admixed with a little dichloromethane and suspended in an ultrasound bath, and the precipitate was filtered off with suction. This gave a further 1.03 g of the title compound.

1H-NMR (first product fraction, 300MHz, DMSO-d6): δ [ppm]= 3.99 (s, 3H), 7.09 (t, 1H), 8.00 (d, 1H), 8.21 - 8.40 (m, 4H), 9.14 (s, 1H), 12.53 (s, 1H), 13.44 (s, 1H).

### Intermediate 3-3

Methyl 5-({[6-(2-hydroxypropan-2-yl)pyridin-2-yl]carbonyl}amino)-1H-indazole-6-carboxylate

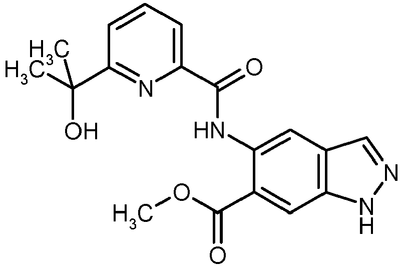

2.10 g of potassium 6-(2-hydroxypropan-2-yl)pyridine-2-carboxylate (Intermediate V3-1) were initially charged in 15 ml of THF. 3.69 g (11.5 mmol) of O-(benzotriazol-1-yl)-N,N,N',N'-tetramethyluronium tetrafluoroborate and 2.00 ml of N-ethyl-N-isopropylpropan-2-amine were added and the mixture was stirred at room temperature for 15 min. Subsequently, 1.83 g (9.58 mmol) of methyl 5-amino-1H-indazole-6-carboxylate (Intermediate 2-1) were added and the mixture was stirred at room temperature for 19 h. The mixture was admixed with water and ethyl acetate, the undissolved solids were filtered off, the phases of the filtrate were separated, and the aqueous phase was extracted twice with ethyl acetate, washed with sodium chloride solution, filtered through a hydrophobic filter, concentrated and purified by column chromatography on silica gel (hexane/ethyl acetate). After the solvents had been removed, 1.56 g of the title compound were obtained as a yellow foam. UPLC-MS (Method A1): Rₜ = 1.00 min (UV detector: TIC Smooth), mass found 354.00.

1H-NMR (500MHz,DMSO-d6): δ [ppm] = 1.63 (s, 6H), 3.97 (s, 3H), 5.37(s ,1H), 7.90 - 7.95 (m, 1H), 8.03-8.07 (m, 2H), 8.23(s, 1H),8.29 (s, 1H), 9.19 (s, 1H), 12.79 (s, 1H), 13.41 (br.s., 1H).

### Intermediate 4-1

Methyl 2-(oxetan-3-ylmethyl)-5-({[6-(trifluoromethyl)pyridin-2-yl]carbonyl}amino)-2H-indazole-6-carboxylate 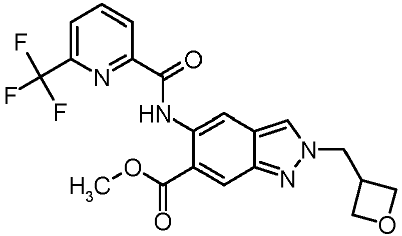 1.00 mg (2.66 mmol) of methyl 5-({[6-(trifluoromethyl)pyridin-2-yl]carbonyl}amino)-1H-indazole-6-carboxylate (Intermediate 3-1) was dissolved in 10 ml of DMF and, after addition of 1.10 mg (7.99 mmol) of potassium carbonate and 221 mg (1.33 mmol) of potassium iodide, the mixture was stirred at 25°C for 30 min. 603 mg (3.99 mmol) of 3-bromomethyloxetane were added, and the mixture was stirred at 25°C for 24 h. The reaction mixture was partitioned between water and ethyl acetate. The mixture was extracted twice with ethyl acetate, and the combined organic phases were filtered through a hydrophobic filter and concentrated. The residue was purified by column chromatography on silica gel (hexane/ethyl acetate). This gave 260 mg of the title compound.

UPLC-MS (Method A2): Rₜ = 1.24 min

MS (ESIpos): m/z = 435(M+H)⁺

¹H NMR (400 MHz, DMSO-d6): δ [ppm] = 3.49 - 3.64 (m, 1 H), 3.95 (s, 3 H), 4.49 (t, 2 H), 4.68 (dd, 2 H), 4.81 (d, 2 H), 8.20 (dd, 1 H), 8.35 - 8.41 (m, 1 H), 8.43 - 8.49 (m, 2 H), 8.55 - 8.58 (m, 1 H), 9.06 (s, 1 H), 12.53 (s, 1 H).

### Intermediate 4-2

Methyl 2-(2-methoxyethyl)-5-({[6-(trifluoromethyl)pyridin-2-yl]carbonyl}amino)-2H-indazole-6-carboxylate

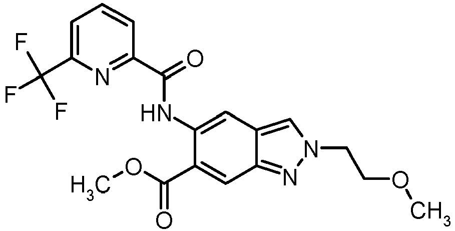

1.00 mg (2.75 mmol) of methyl 5-({[6-(trifluoromethyl)pyridin-2-yl]carbonyl}amino)-1H-indazole-6-carboxylate (Intermediate 3-1) was dissolved in 5 ml of DMF, and 387 µl (4.12 mmol) of 2-bromoethyl methyl ether, 1.14 g (8.23 mmol) of potassium carbonate and 228 mg (1.37 mmol) of potassium iodide were added while stirring. The reaction mixture was stirred at 25°C for 24 h, diluted with water and extracted twice with ethyl acetate. The combined organic phases were filtered through a hydrophobic filter and concentrated. The residue was purified by column chromatography on silica gel (hexane/ethyl acetate). This gave 12 mg of the title compound.

UPLC-MS (Method A1): Rₜ = 1.24 min

MS (ESIpos): m/z = 423 (M+H)⁺

¹H NMR (400 MHz, DMSO-d6): δ [ppm] = 3.24 (s, 3 H), 3.86 (t, 2 H), 3.96 (s, 3 H), 4.65 (t, 2 H), 8.21 (dd, 1 H), 8.35 - 8.42 (m, 1 H), 8.43 - 8.51 (m, 2 H), 8.52 (d, 1 H), 9.06 (s, 1 H), 12.53 (s, 1 H).

### Intermediate 4-3

Methyl 2-(3-methoxypropyl)-5-({[6-(trifluoromethyl)pyridin-2-yl]carbonyl}amino)-2H-indazole-6-carboxylate

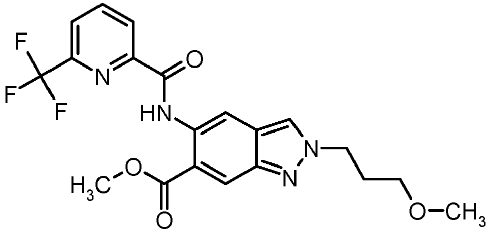

1.00 mg (2.75 mmol) of methyl 5-({[6-(trifluoromethyl)pyridin-2-yl]carbonyl}amino)-1H-indazole-6-carboxylate (Intermediate 3-1) was dissolved in 5 ml of DMF, and 460 µl (4.12 mmol) of 1-bromo-3-methoxypropane, 1.14 g (8.23 mmol) of potassium carbonate and 228 mg (1.37 mmol) of potassium iodide were added while stirring. The reaction mixture was stirred at 25°C for 72 h, diluted with water and extracted twice with ethyl acetate. The combined organic phases were filtered through a hydrophobic filter and concentrated. The residue was purified by column chromatography on silica gel (hexane/ethyl acetate). This gave 28 mg of the title compound.

UPLC-MS (Method A1): Rₜ = 1.29 min

MS (ESIpos): m/z = 437 (M+H)⁺

¹H NMR (400 MHz, DMSO-d6): δ [ppm] = 2.17 (quin, 2 H), 3.24 (s, 3 H), 3.33 - 3.36 (m, 2 H), 3.96 (s, 3 H), 4.53 (t, 2 H), 8.21 (dd, 1 H), 8.35 - 8.42 (m, 1 H), 8.45 - 8.49 (m, 2 H), 8.54 (d, 1 H), 9.06 (s, 1 H), 12.54 (s, 1 H).

### Intermediate 4-4

Methyl 2-(3-hydroxy-3-methylbutyl)-5-({[6-(trifluoromethyl)pyridin-2-yl]carbonyl}amino)-2H-indazole-6-carboxylate

### Preparation Method 1

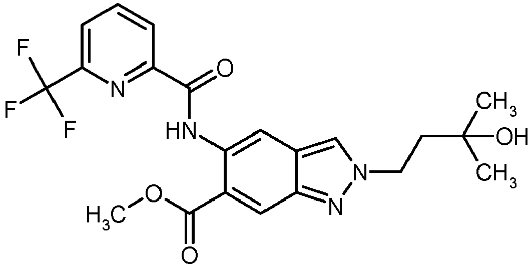

930 mg (2.55 mmol) of methyl 5-({[6-(trifluoromethyl)pyridin-2-yl]carbonyl}amino)-1H-indazole-6-carboxylate (Intermediate 3-1), 1.06 g of potassium carbonate and 212 mg of potassium iodide were initially charged in 9 ml of DMF and the mixture was stirred for 15 min. Then 0.62 ml of 4-bromo-2-methylbutan-2-ol was added and the mixture was stirred at 60°C overnight. The mixture was admixed with water and extracted twice with ethyl acetate, and the extract was washed three times with saturated sodium chloride solution, filtered and concentrated. Column chromatography purification on silica gel (hexane/ethyl acetate) gave 424 g of the title compound.

UPLC-MS (Method A2): Rₜ = 1.21 min (UV detector: TIC), mass found 450.00.

¹H-NMR (400MHz, DMSO-d₆): δ [ppm]= 1.16 (s, 6 H) 2.02 - 2.11 (m, 2 H) 3.96 (s, 3 H) 4.51 - 4.60 (m, 3 H) 8.20 (dd, *J*=7.83, 1.01 Hz, 1 H) 8.39 (s, 1 H) 8.45 (s, 2 H) 8.55 (d, *J*=0.76 Hz, 1 H) 9.05 (s, 1 H) 12.52 (s, 1 H).

### Preparation Method 2

1.95 g (7.03 mmol) of methyl 5-amino-2-(3-hydroxy-3-methylbutyl)-2H-indazole-6-carboxylate (Intermediate 7-1) were initially charged in 30 ml of THF. 1.45 g (7.73 mmol) of 6-(trifluoromethyl)pyridine-2-carboxylic acid, 2.71 g (8.44 mmol) of O-(benzotriazol-1-yl)-N,N,N',N'-tetramethyluronium tetrafluoroborate and 1.47 ml (8.44 mmol) of N-ethyl-N-isopropylpropan-2-amine were added and the mixture was stirred at 25°C for 20.5 h. Water was added, the mixture was extracted three times with ethyl acetate and the extracts were washed with sodium chloride solution, filtered through a hydrophobic filter and concentrated. The residue was separated by column chromatography on silica gel (hexane/ethyl acetate). This gave 2.79 g of the title compound.

UPLC-MS (Method A1): Rₜ = 1.23 min (UV detector: TIC), mass found 450.00.

### Intermediate 4-5

Methyl 2-(2-{[tert-butyl(dimethyl)silyl]oxy}ethyl)-5-({[6-(trifluoromethyl)pyridin-2-yl]carbonyl}amino)-2H-indazole-6-carboxylate

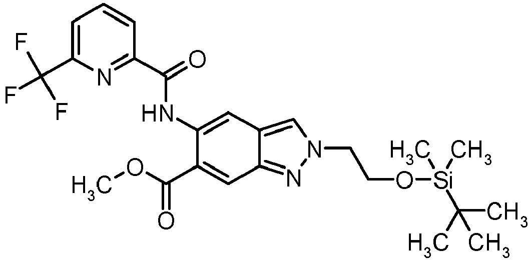

1.00g (2.66 mmol, 97%) of methyl 5-({[6-(trifluoromethyl)pyridin-2-yl]carbonyl}amino)-1H-indazole-6-carboxylate (Intermediate 3-1) was initially charged in 50 ml of DMF, 1.10 g (7.99 mmol) of potassium carbonate and 221 mg (1.33 mmol) of potassium iodide were added while stirring, and the mixture was stirred at 25°C for 30 min. Subsequently, 857 µl (3.99 mmol) of (2-bromoethoxy)(tert-butyl)dimethylsilane were added and the mixture was stirred at 25°C for 24 h. The reaction mixture was diluted with water and extracted with ethyl acetate. The combined organic phases were filtered through a hydrophobic filter and concentrated. The residue was purified by column chromatography on silica gel (hexane/ethyl acetate). This gave 400 mg of the title compound.

UPLC-MS (Method A1): Rₜ = 1.58 min

MS (ESIpos): m/z = 523(M+H)^{+ 1}H NMR (300 MHz, DMSO-d6): δ [ppm] = -0.18 - -0.13 (m, 6 H), 0.74 (s, 9 H), 3.96 (s, 3 H), 4.08 (t, 2 H), 4.57 (t, 2 H), 8.15 - 8.25 (m, 1 H), 8.32 - 8.43 (m, 1 H), 8.43 - 8.52 (m, 3 H), 9.07 (s, 1 H), 12.53 (s, 1 H).

### Intermediate 4-6

Methyl 2-(3-{[tert-butyl(dimethyl)silyl]oxy}propyl)-5-({[6-(trifluoromethyl)pyridin-2-yl]carbonyl}amino)-2H-indazole-6-carboxylate

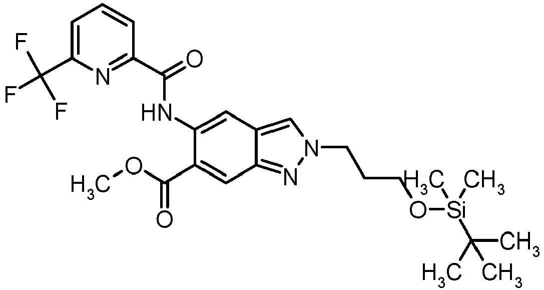

Analogously to Intermediate 4-5, 1.00 g (2.75 mmol) of methyl 5-({[6-(trifluoromethyl)pyridin-2-yl]carbonyl}amino)-1H-indazole-6-carboxyate (Intermediate 7-1) was dissolved in 10 ml of DMF 1.14 g (8.24 mmol) of potassium carbonate and 228 mg (1.37 mmol) of potassium iodide were added while stirring, and the mixture was stirred at 25°C for 30 min. Subsequently, 1.04 g (4.12 mmol) of (3-bromopropoxy)(tert-butyl)dimethylsilane were added and the mixture was stirred at 25°C for 24 h. The reaction mixture was filtered and the filtercake was washed with ethyl acetate. The reaction mixture was partitioned between water and ethyl acetate and the aqueous phase was extracted twice with ethyl acetate. The combined organic phases were filtered through a hydrophobic filter and concentrated. Purification of the residue by preparative HPLC gave 428 mg of the title compound.

UPLC-MS (Method A1): Rₜ = 1.63 min

MS (ESIpos): m/z = 537 (M+H)⁺

¹H NMR (400 MHz, DMSO-d6): δ [ppm] = -0.02 - 0.06 (m, 6 H), 0.87 (s, 9 H), 2.14 (quin, 2 H), 3.62 (t, 2 H), 3.96 (s, 3 H), 4.54 (t, 2 H), 8.20 (d, 1 H), 8.35 - 8.42 (m, 1 H), 8.43 - 8.48 (m, 2 H), 8.49 - 8.53 (m, 1 H), 9.06 (s, 1 H).

### Intermediate 4-7

Methyl 5-({[6-(2-hydroxypropan-2-yl)pyridin-2-yl]carbonyl}amino)-2-(4,4,4-trifluorobutyl)-2H-indazole-6-carboxylate

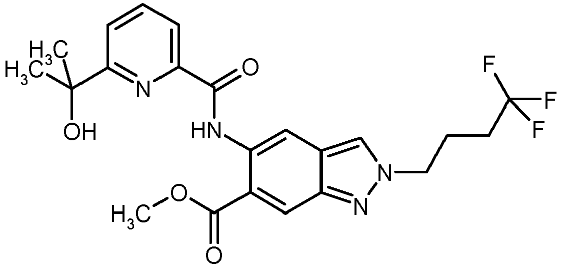

300 mg of methyl 5-({[6-(2-hydroxypropan-2-yl)pyridin-2-yl]carbonyl}amino)-1H-indazole-6-carboxylate (Intermediate 3-3) were initially charged in 4.5 ml of DMF. 287 mg of 1,1,1-trifluoro-4-iodobutane and 333 mg of potassium carbonate were added and the mixture was stirred at 100°C for 23 h. Water was added, and the mixture was extracted three times with ethyl acetate. The mixture was concentrated and the product was purified by preparative HPLC. This gave 72 mg of the title compound.

UPLC-MS (Method A1): Rₜ = 1.26 min (UV detector: TIC), mass found 464.17.

### Intermediate 4-8

Methyl 5-{[(5-fluoro-6-methylpyridin-2-yl)carbonyl]amino}-2-(3-hydroxy-3-methylbutyl)-2H-indazole-6-carboxylate

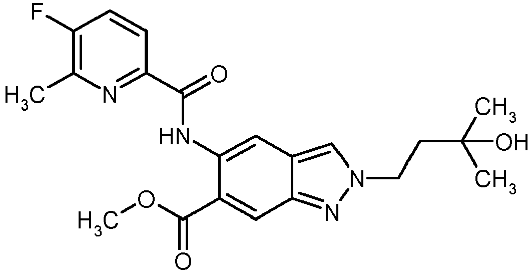

195 mg of methyl 5-amino-2-(3-hydroxy-3-methylbutyl)-2H-indazole-6-carboxylate (Intermediate 7-1) were reacted with 78 mg of 5-fluoro-6-methylpyridine-2-carboxylic acid analogous to Intermediate 4-4 (Preparation Method 2) within 19.5 h. 228 mg of a crude product were obtained after analogous aqueous workup.

UPLC-MS (Method A1): Rₜ = 1.20 min (UV detector: TIC), mass found 414.00.

### Intermediate 4-9

Methyl 2-(3-hydroxy-3-methylbutyl)-5-{[(6-methylpyridin-2-yl)carbonyl]amino}-2H-indazole-6-carboxylate

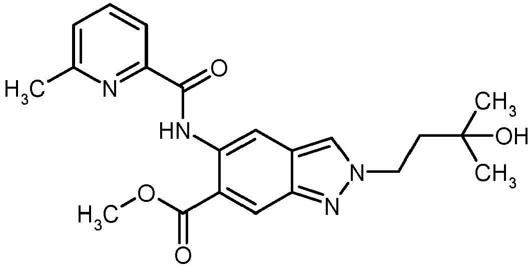

195 mg of methyl 5-amino-2-(3-hydroxy-3-methylbutyl)-2H-indazole-6-carboxylate (Intermediate 7-1) were reacted with 70 mg of 6-methylpyridine-2-carboxylic acid analogous to the preparation of Intermediate 4-4 (Preparation Method 2) within 19.5 h. 278 mg of the title compound as crude product were obtained after analogous aqueous workup.

UPLC-MS (Method A1): Rₜ = 1.14 min (UV detector: TIC), mass found 396.00.

### Intermediate 4-10

Methyl 2-[3-(2,2,2-trifluoroethoxy)propyl]-5-({[6-(trifluoromethyl)pyridin-2-yl]carbonyl}amino)-2H-indazole-6-carboxylate

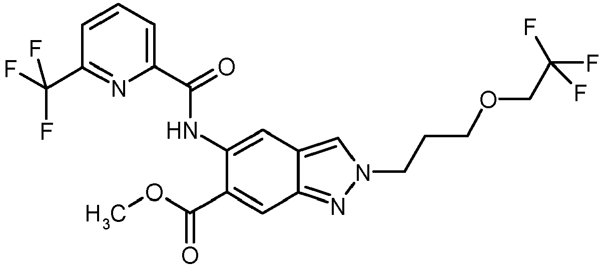

A mixture of 250 mg of methyl 5-({[6-(trifluoromethyl)pyridin-2-yl]carbonyl}amino)-1H-indazole-6-carboxylate (Intermediate 7-1), 193 mg of 3-bromopropyl 2,2,2-trifluoroethyl ether, 242 mg of potassium carbonate and 145 mg of potassium iodide in 3 ml of DMF was stirred at 100°C for 20 h. Water was added, the mixture was extracted with ethyl acetate and the extract was washed with sodium chloride solution and concentrated. Purification by preparative HPLC gave 52 mg of the title compound.

UPLC-MS (Method A1): Rₜ = 1.39 min (UV detector: TIC), mass found 504.12.

### Intermediate 5-1

N-[6-(2-Hydroxypropan-2-yl)-1H-indazol-5-yl]-6-(trifluoromethyl)pyridine-2-carboxamide

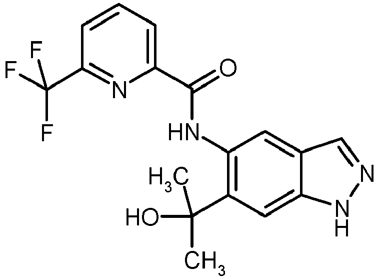

To a solution, cooled in an ice-water cooling bath, of 1.50 g (4.12 mmol) of methyl 5-({[6-(trifluoromethyl)pyridin-2-yl]carbonyl}amino)-1H-indazole-6-carboxylate (Intermediate 3-1) in 20 ml of THF were cautiously added 6.9 ml (5 equivalents) of a 3M methylmagnesium bromide solution in diethyl ether. The mixture was stirred while cooling with an ice bath for 1 h and at room temperature for 19.5 h. Another 2 equivalents of methylmagnesium bromide solution were added and the mixture was stirred at room temperature for a further 24 h. Saturated aqueous ammonium chloride solution was added and the mixture was stirred and extracted three times with ethyl acetate. The combined organic phases were washed with sodium chloride solution, filtered through a hydrophobic filter and concentrated. The residue was purified by column chromatography on silica gel (hexane/ethyl acetate). This gave 763 mg of the title compound.

¹H-NMR (400MHz, DMSO-d₆): δ [ppm]= 1.63 (s, 6H), 5.99 (s, 1H), 7.49 (s, 1H), 8.06 (s, 1H), 8.14 - 8.19 (m, 1H), 8.37 (t, 1H), 8.46 (d, 1H), 8.78 (s, 1H), 12.32 (s, 1H), 12.97 (s, 1H).

### Intermediate 5-2

6-(Difluoromethyl)-N-[6-(2-hydroxypropan-2-yl)-1H-indazol-5-yl]pyridine-2-carboxamide 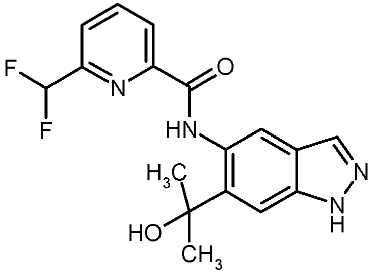 Analogously to the preparation of Intermediate 5-1, 2.40 g (6.93 mmol) of methyl 5-({[6-(difluoromethyl)pyridin-2-yl]carbonyl}amino)-1H-indazole-6-carboxylate (Intermediate 3-2) in 10 ml of THF were reacted with three portions of 3M methylmagnesium bromide solution in diethyl ether (6.9 ml, then stirring at room temperature for 45 min; 11.6 ml, then stirring at room temperature for 2 h; 6.9 ml, then stirring at room temperature for 2 h). After the workup as for Intermediate 5-1, 2.39 g of a crude product were obtained, which were used further without further purification.

### Intermediate 6-1

Methyl 2-(3-hydroxy-3-methylbutyl)-5-nitro-2H-indazole-6-carboxylate 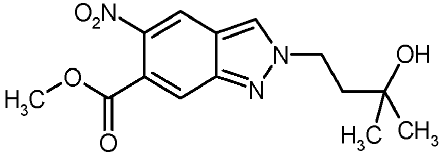 5.00 g (22.6 mmol) of methyl 5-nitro-1H-indazole-6-carboxylate(Intermediate 1-1) were initially charged in 40 ml of DMF. 5.65 g (33.9 mmol) of 4-bromo-2-methylbutan-2-ol, 9.37 g (67.8 mmol) of potassium carbonate and 5.63 g (33.9 mmol) of potassium iodide were added and the mixture was stirred at 100°C for 20 h. Water was added, the mixture was extracted three times with ethyl acetate and the extracts were washed with sodium chloride solution, filtered through a hydrophobic filter and concentrated. The residue was purified by column chromatography on silica gel (hexane/ethyl acetate). The solids obtained were extracted by stirring with diethyl ether, filtered off with suction, washed with diethyl ether and dried. This gave 2.49 g of the title compound.

UPLC-MS (Method A1): Rₜ = 0.93 min (UV detector: TIC), mass found 307.00.

¹H-NMR (400MHz, DMSO-d₆): δ [ppm]= 1.15 (s, 6H), 2.02 - 2.11 (m, 2H), 3.84 (s, 3H), 4.54 (s, 1H), 4.58 - 4.65 (m, 2H), 8.05 (s, 1H), 8.69 (s, 1H), 8.86 (s, 1H).

### Intermediate 7-1

Methyl 5-amino-2-(3-hydroxy-3-methylbutyl)-2H-indazole-6-carboxylate 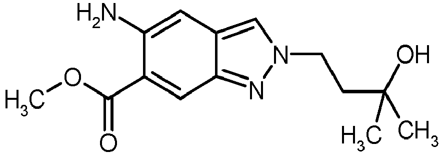 4.53 g of iron and 217 mg of ammonium chloride were added to 2.49 g (8.10 mmol) of methyl 2-(3-hydroxy-3-methylbutyl)-5-nitro-2H-indazole-6-carboxylate (Intermediate 6-1) in 30 ml of ethanol and 10 ml of water, and the mixture was stirred at 90°C for 21.5 h. The mixture was filtered through Celite and washed through with ethanol three times, and the filtrate was concentrated and the residue was admixed with water. Extraction was effected three times with ethyl acetate (to improve the phase separation, sodium chloride solution was added). The combined organic phases were washed with sodium chloride solution, filtered through a hydrophobic filter and concentrated. This gave 1.95 g (85% of theory) of the title compound.

UPLC-MS (Method A1): Rₜ = 0.67 min (UV detector: TIC), mass found 277.00.

¹H-NMR (400MHz, DMSO-d₆): δ [ppm]= 1.14 (s, 6H), 1.96 - 2.08 (m, 2H), 3.85 (s, 3H), 4.39 - 4.51 (m, 3H), 5.81 (s, 2H), 6.80 (s, 1H), 8.05 (s, 1H), 8.18 (s, 1H).

### Working examples

### Example 1

N-[6-(2-Hydroxypropan-2-yl)-2-(2-methoxyethyl)-2H-indazol-5-yl]-6-(trifluoromethyl)pyridine-2-carboxamide

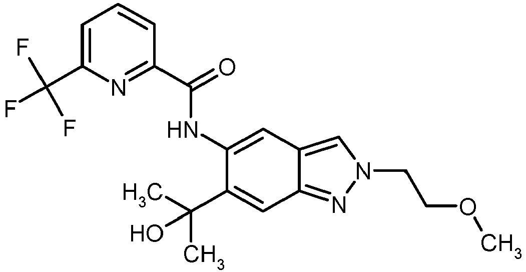

75 mg (0.18 mmol) of methyl 2-(2-methoxyethyl)-5-({[6-(trifluoromethyl)pyridin-2-yl]carbonyl}amino)-2H-indazole-6-carboxylate (Intermediate 4-2) were dissolved in 500 µl of THF and admixed with 887 µl (0.89 mmol) of a 1 M methylmagnesium bromide solution in THF. The reaction mixture was stirred at 25°C for 60 min. Subsequently, 1 ml of a saturated aqueous ammonium chloride solution was added cautiously and the mixture was filtered. The aqueous phase was extracted twice with ethyl acetate, and the organic phases were combined, filtered through a hydrophobic filter and concentrated. The residue was dissolved in 3 ml of DMSO and purified by preparative HPLC. The product-containing fractions were freeze-dried. This gave 20 mg of the title compound.

UPLC-MS (Method A1): Rₜ = 1.08 min

MS (ESIpos): m/z = 423 (M+H)⁺

¹H NMR (300 MHz, DMSO-d6): δ [ppm] = 1.62 (s, 6 H), 3.22 (s, 3 H), 3.82 (t, J=5.2 Hz, 2 H), 4.55 (t, J=5.2 Hz, 2 H), 5.96 (s, 1 H), 7.57 (s, 1 H), 8.16 (d, J=7.2 Hz, 1 H), 8.29 - 8.42 (m, 2 H), 8.42 - 8.50 (m, 1 H), 8.71 (s, 1 H), 12.36 (s, 1 H)

### Example 2

N-[6-(Hydroxymethyl)-2-(2-methoxyethyl)-2H-indazol-5-yl]-6-(trifluoromethyl)pyridine-2-carboxamide

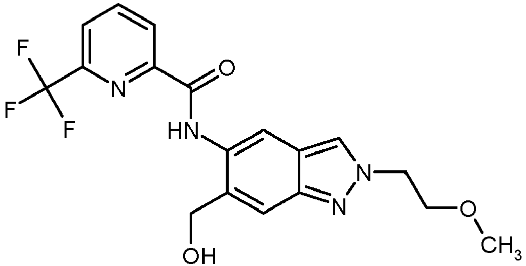

13 mg (0.36 mmol) of lithium aluminium hydride were suspended in 1 ml of THF and the mixture was cooled to 0°C. 75 mg (0.17 mmol) of methyl 2-(2-methoxyethyl)-5-({[6-(trifluoromethyl)pyridin-2-yl]carbonyl}amino)-2H-indazole-6-carboxylate (Intermediate 4-2) dissolved in 500 µl of THF were added dropwise and the mixture was stirred at 25°C for 60 min. The mixture was diluted with water and extracted twice with ethyl acetate, and the combined organic phases were washed with sodium chloride solution, filtered through a hydrophobic filter, concentrated and dried under reduced pressure. This gave 36 mg of the title compound.

UPLC-MS (Method A2): Rₜ = 0.97 min

MS (ESIpos): m/z = 409 (M+H)⁺

¹H NMR (400 MHz, DMSO-d6): δ [ppm] = 1.62 (s, 6 H), 3.86 (q, 2 H), 4.43 (t, 2 H), 4.95 (t, 1 H), 5.94 (s, 1 H), 7.57 (s, 1 H), 8.16 (dd, 1 H), 8.30 (s, 1 H), 8.37 (t, 1 H), 8.45 (d, 1 H), 8.72 (s, 1 H), 12.36 (s, 1 H).

### Example 3

N-[6-(2-Hydroxypropan-2-yl)-2-(3-methoxypropyl)-2H-indazol-5-yl]-6-(trifluoromethyl)pyridine-2-carboxamide

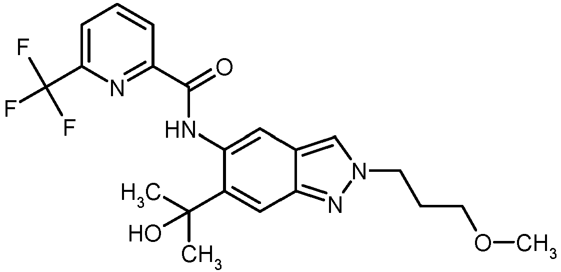

75 mg (0.17 mmol) of methyl 2-(3-methoxypropyl)-5-({[6-(trifluoromethyl)pyridin-2-yl]carbonyl}amino)-2H-indazole-6-carboxylate (Intermediate 4-3) were dissolved in 500 µl of THF and admixed with 859 µl (0.86 mmol) of a 1 M methylmagnesium bromide solution in THF. The reaction mixture was stirred at 25°C for 60 min. Subsequently, 1 ml of a saturated ammonium chloride solution was added cautiously and the mixture was filtered. The aqueous phase was extracted twice with ethyl acetate, and the organic phases were combined, filtered through a hydrophobic filter and concentrated. The residue was dissolved in 3 ml of DMSO and purified by preparative HPLC. The product-containing fractions were freeze-dried. This gave 25 mg of the title compound.

UPLC-MS (Method A1): Rₜ = 1.13 min

MS (ESIpos): m/z = 437 (M+H)⁺

¹H NMR (400 MHz, DMSO-d6): δ [ppm] = 1.62 (s, 6 H), 2.14 (quin, 2 H), 3.23 (s, 3 H), 3.26 - 3.32 (m, 2 H), 4.44 (t, 2 H), 5.95 (s, 1 H), 7.58 (s, 1 H), 8.16 (d, 1 H), 8.31 - 8.40 (m, 2 H), 8.43 - 8.48 (m, 1 H), 8.72 (s, 1 H), 12.36 (s, 1 H).

### Example 4

N-[6-(Hydroxymethyl)-2-(3-methoxypropyl)-2H-indazol-5-yl]-6-(trifluoromethyl)pyridine-2-carboxamide

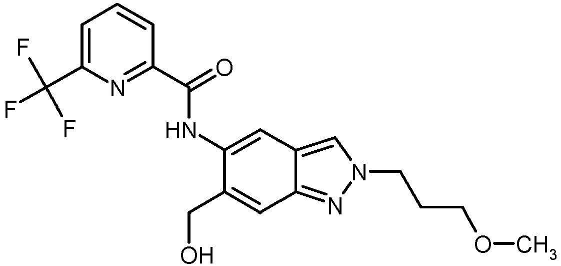

13 mg of lithium aluminium hydride were suspended in THF and the mixture was cooled to 0°C. 75 mg (0.17 mmol) of methyl 2-(3-methoxypropyl)-5-({[6-(trifluoromethyl)pyridin-2-yl]carbonyl}amino)-2H-indazole-6-carboxylate (Intermediate 4-3) in THF were added dropwise and the mixture was allowed to come to room temperature within 30 min. The mixture was diluted with water and filtered, the residue was washed with ethyl acetate and the filtrate was extracted with ethyl acetate. The combined ethyl acetate phases were washed with sodium chloride solution, filtered through a hydrophobic filter and concentrated. The residue was purified by preparative HPLC.

¹H NMR (300 MHz, DMSO-d₆): δ [ppm] = 2.14 (quin, 2 H), 3.23 (s, 3 H), 3.29 (t, 2 H), 4.45 (t, *J*=7.0 Hz, 2 H), 4.68 (d, 2 H), 5.77 (t, 1 H), 7.58 (s, 1 H), 8.18 (d, 1 H), 8.32 - 8.48 (m, 3 H), 8.51 (s, 1H), 11.21 (s, 1H).

### Example 5

N-[2-(2-Hydroxyethyl)-6-(2-hydroxypropan-2-yl)-2H-indazol-5-yl]-6-(trifluoromethyl)pyridine-2-carboxamide

### Stage A:

Preparation of N-[2-(2-{[tert-butyl(dimethyl)silyl]oxy}ethyl)-6-(2-hydroxypropan-2-yl)-2H-indazol-5-yl]-6-(trifluoromethyl)pyridine-2-carboxamide

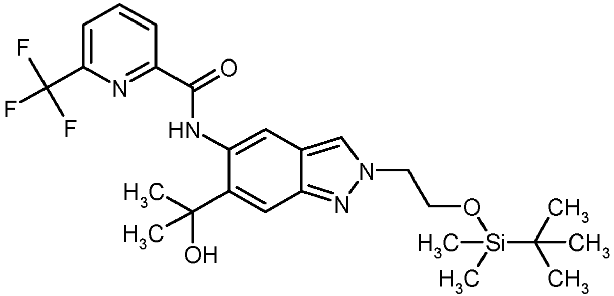

100 mg (0.19 mmol) of methyl 2-(2-{[tert-butyl(dimethyl)silyl]oxy}ethyl)-5-({[6-(trifluoromethyl)pyridin-2-yl]carbonyl}amino)-2H-indazole-6-carboxylate (Intermediate 4-5) were dissolved in 1 ml of THF and admixed with 669 µl (0.67 mmol) of a 1 M methylmagnesium bromide solution in THF. The reaction mixture was stirred at 25°C for 60 min. Another 287 µl (0.29 mmol) of a 1 M methylmagnesium bromide solution in THF were added and the mixture was stirred at 25°C for 3 h. Subsequently, 20 ml of a saturated ammonium chloride solution were added cautiously and the mixture was filtered. The aqueous phase was extracted twice with ethyl acetate, and the organic phases were combined, dried over magnesium sulphate, filtered, concentrated and dried under reduced pressure. This gave 50 mg of N-[2-(2-{[tert-butyl(dimethyl)silyl]oxy}ethyl)-6-(2-hydroxypropan-2-yl)-2H-indazol-5-yl]-6-(trifluoromethyl)pyridine-2-carboxamide.

UPLC-MS (Method A2): Rₜ = 1.51 min

MS (ESIpos): m/z = 523(M+H)⁺

¹H NMR (300 MHz, DMSO-d6): δ [ppm] = -0.17 - -0.09 (m, 6 H), 0.78 (s, 9 H), 1.62 (s, 6 H), 4.04 (t, 2 H), 4.47 (t, 2 H), 5.98 (s, 1 H), 7.57 (s, 1 H), 8.16 (d, 1 H), 8.29 (s, 1 H), 8.37 (t, 1 H), 8.45 (d, 1 H), 8.73 (s, 1 H), 12.38 (s, 1 H).

### Stage B:

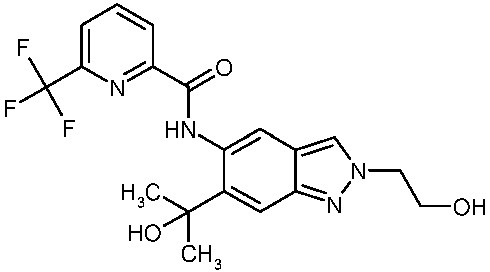

50 mg (96 µmol) of N-[2-(2-{[tert-butyl(dimethyl)silyl]oxy}ethyl)-6-(hydroxymethyl)-2H-indazol-5-yl]-6-(trifluoromethyl)pyridine-2-carboxamide were dissolved in 1.0 ml of THF and admixed with 144 µl (0.14 mmol) of a 1 M solution of tetrabutylammonium fluoride in THF. The reaction mixture was stirred at room temperature for 1 h. The mixture was diluted with water and extracted twice with ethyl acetate, and the combined organic phases were washed with saturated sodium chloride solution, filtered through a hydrophobic filter and concentrated. This gave 36 mg of N-[2-(2-hydroxyethyl)-6-(2-hydroxypropan-2-yl)-2H-indazol-5-yl]-6-(trifluoromethyl)pyridine-2-carboxamide (Example 5).

¹H-NMR (400MHz, DMSO-d₆): d [ppm]= 1.62 (s, 6H), 3.86 (q, 2H), 4.43 (t, 2H), 4.95 (t, 1H), 5.94 (s, 1H), 7.57 (s, 1H), 8.16 (dd, 1H), 8.30 (s, 1H), 8.37 (t, 1H), 8.45 (d, 1H), 8.72 (s, 1H), 12.36 (s, 1H).

UPLC-MS (Method A2): Rₜ = 0.97 min (UV detector: TIC), mass found 408.00.

### Example 6

N-[6-(2-Hydroxypropan-2-yl)-2-(3-hydroxypropyl)-2H-indazol-5-yl]-6-(trifluoromethyl)pyridine-2-carboxamide

### Stage A:

Preparation of N-[2-(3-{[tert-butyl(dimethyl)silyl]oxy}propyl)-6-(2-hydroxypropan-2-yl)-2H-indazol-5-yl]-6-(trifluoromethyl)pyridine-2-carboxamide 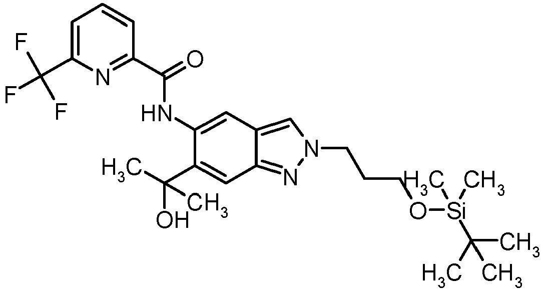 50 mg (0.09 mmol) of methyl 2-(3-{[tert-butyl(dimethyl)silyl]oxy}propyl)-5-({[6-(trifluoromethyl)pyridin-2-yl]carbonyl}amino)-2H-indazole-6-carboxylate (Intermediate 4-6) were dissolved in 500 ml of THF and admixed with 326 µl (0.33 mmol) of a 1 M methylmagnesium bromide solution in THF. The reaction mixture was stirred at 25°C for 60 min. Subsequently, 20 ml of a saturated ammonium chloride solution were added cautiously and the mixture was extracted twice with ethyl acetate. The combined organic phases were filtered through a hydrophobic filter, concentrated and dried under reduced pressure. The residue was purified by preparative HPLC. 40 mg of N-[2-(3-{[tert-butyl(dimethyl)silyl]oxy}propyl)-6-(2-hydroxypropan-2-yl)-2H-indazol-5-yl]-6-(trifluoromethyl)pyridine-2-carboxamide were obtained.

UPLC-MS (Method A1): Rₜ = 1.58 min

MS (ESIpos): m/z = 537(M+H)⁺

¹H NMR (300 MHz, DMSO-d6): δ [ppm] = 0.02 - 0.05 (m, 6 H), 0.84 - 0.91 (m, 9 H), 1.62 (s, 6 H), 2.02 - 2.18 (m, 2 H), 3.55 - 3.62 (m, 2 H), 4.45 (t, 2 H), 5.96 (s, 1 H), 7.57 (s, 1 H), 8.16 (d, 1 H), 8.31 (s, 1 H), 8.33 - 8.42 (m, 1 H), 8.45 (d, 1 H), 8.72 (s, 1 H), 12.37 (s, 1 H).

### Stage B:

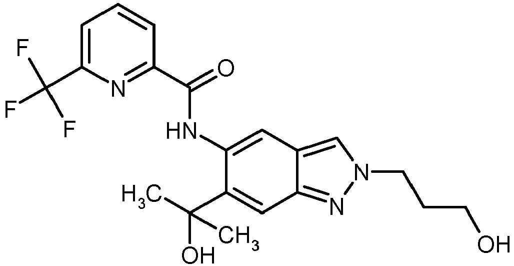

37 mg (0.07 mmol) of N-[2-(3-{[tert-butyl(dimethyl)silyl]oxy}propyl)-6-(2-hydroxypropan-2-yl)-2H-indazol-5-yl]-6-(trifluoromethyl)pyridine-2-carboxamide were dissolved in 500 µl of THF and admixed with 207 µl (0.21 mmol) of a 1 M solution of tetrabutylammonium fluoride in THF. The reaction mixture was stirred at 25°C for 2 h. The mixture was diluted with water and extracted twice with ethyl acetate, and the combined organic phases were washed with saturated sodium chloride solution, filtered and concentrated. After purification by preparative HPLC, 10 mg of N-[6-(2-hydroxypropan-2-yl)-2-(3-hydroxypropyl)-2H-indazol-5-yl]-6-(trifluoromethyl)pyridine-2-carboxamide (Example 6) were obtained.

UPLC-MS (Method A2): Rₜ = 1.00 min

MS (ESIpos): m/z = 423 (M+H)⁺

¹H NMR (400 MHz, DMSO-d6): δ [ppm] = 1.62 (s, 6 H), 2.00 - 2.07 (m, 2 H), 3.07 - 3.22 (m, 1 H), 3.39 (t, 2 H), 4.45 (t, 2 H), 4.63 (br. s., 1 H), 5.94 (br. s., 1 H), 7.56 (s, 1 H), 8.14 (d, 1 H), 8.28 - 8.39 (m, 2 H), 8.41 - 8.47 (m, 1 H), 8.72 (s, 1 H), 12.31 (br. s., 1 H).

### Example 7

N-[2-(2-Hydroxyethyl)-6-(hydroxymethyl)-2H-indazol-5-yl]-6-(trifluoromethyl)pyridine-2-carboxamide

### Stage A:

N-[2-(2-{[tert-Butyl(dimethyl)silyl]oxy}ethyl)-6-(hydroxymethyl)-2H-indazol-5-yl]-6-(trifluoromethyl)pyridine-2-carboxamide

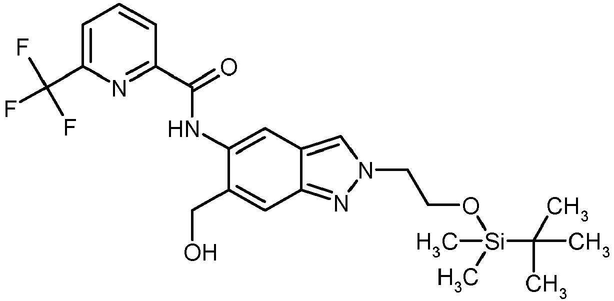

100 mg (0.19 mmol) of methyl 2-(2-{[tert-butyl(dimethyl)silyl]oxy}ethyl)-5-({[6-(trifluoromethyl)pyridin-2-yl]carbonyl}amino)-2H-indazole-6-carboxylate (Intermediate 4-5) were dissolved in 1 ml of THF and admixed with 191 µl (0.38 mmol) of a 2 M lithium borohydride solution. The mixture was left to stir at 25°C for 24 h. 14 mg (0.38 mmol) of sodium borohydride and 500 µl of methanol were added, and the mixture was stirred at 25°C for 4 h. Another 14 mg (0.38 mmol) of sodium borohydride were added, and the mixture was stirred at 25°C for 24 h. Water was added cautiously and the mixture was concentrated. The mixture was then extracted twice with ethyl acetate, and the combined organic phases were washed with saturated sodium chloride solution, filtered through a hydrophobic filter and concentrated. The residue was taken up in 2 ml of DMSO and purified by preparative HPLC. This gave 30 mg of N-[2-(2-{[tert-butyl(dimethyl)silyl]oxy}ethyl)-6-(hydroxymethyl)-2H-indazol-5-yl]-6-(trifluoromethyl)pyridine-2-carboxamide.

UPLC-MS (Method A2): Rₜ = 1.44 min

MS (ESIpos): m/z = 495(M+H)⁺

¹H NMR (300 MHz, DMSO-d6): δ [ppm] = -0.16 - -0.12 (m, 6 H), 0.75 - 0.79 (m, 9 H), 4.05 (t, 2 H), 4.48 (t, 2 H), 4.69 (d, 2 H), 5.75 - 5.77 (m, 1 H), 7.57 (s, 1 H), 8.18 (dd, 1 H), 8.30 - 8.33 (m, 1 H), 8.38 (t, 1 H), 8.45 (d, 1 H), 8.51 (s, 1 H), 11.20 (s, 1 H).

### Stage B:

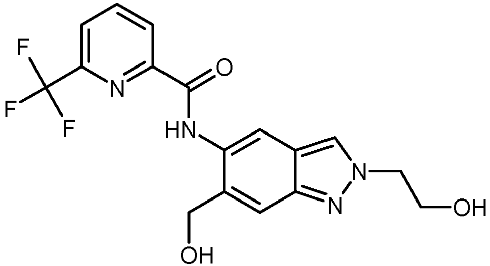

33 mg (0.07 mmol) of N-[2-(2-{[tert-butyl(dimethyl)silyl]oxy}ethyl)-6-(hydroxymethyl)-2H-indazol-5-yl]-6-(trifluoromethyl)pyridine-2-carboxamide were dissolved in 1 ml of THF and admixed with 100 µl (0.10 mmol) of a 1 M solution of tetrabutylammonium fluoride in THF. The reaction mixture was stirred at 25°C for 1 h. The mixture was diluted with water and extracted twice with ethyl acetate, and the combined organic phases were washed with saturated sodium chloride solution, filtered through a hydrophobic filter, concentrated and dried under reduced pressure. 25 mg of N-[2-(2-hydroxyethyl)-6-(hydroxymethyl)-2H-indazol-5-yl]-6-(trifluoromethyl)pyridine-2-carboxamide (Example 7) were obtained.

UPLC-MS (Method A2): Rₜ = 0.87 min

MS (ESIpos): m/z = 381 (M+H)⁺

¹H NMR (300 MHz, DMSO-d6): δ [ppm] = 3.87 (q, 2 H), 4.44 (t, 2 H), 4.69 (d, 2 H), 4.98 (t, 1 H), 5.70 - 5.81 (m, 1 H), 7.57 (s, 1 H), 8.11 - 8.23 (m, 1 H), 8.31 - 8.42 (m, 2 H), 8.43 - 8.49 (m, 1 H), 8.51 (s, 1 H), 11.20 (s, 1 H).

### Example 8

N-[6-(2-Hydroxypropan-2-yl)-2-(oxetan-3-ylmethyl)-2H-indazol-5-yl]-6-(trifluoromethyl)pyridine-2-carboxamide

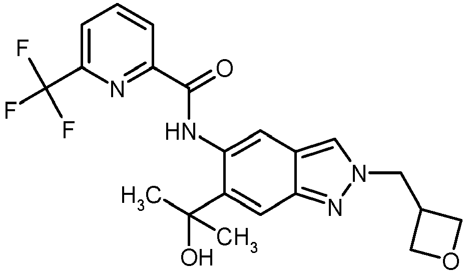

50 mg (0.12 mmol) of methyl 2-(oxetan-3-ylmethyl)-5-({[6-(trifluoromethyl)pyridin-2-yl]carbonyl}amino)-2H-indazole-6-carboxylate (Intermediate 4-1) were dissolved in 500 µl of THF and admixed with 576 µl (0.58 mmol) of a 1 M methylmagnesium bromide solution in THF. The reaction mixture was stirred at 25°C for 60 min. Subsequently, 20 ml of a saturated aqueous ammonium chloride solution were added cautiously and the mixture was concentrated. The aqueous phase was extracted twice with ethyl acetate, and the organic phases were combined, dried over magnesium sulphate, filtered and concentrated. The residue was dissolved in 2.0 ml of DMSO and purified by preparative HPLC. The product-containing fractions were freeze-dried. This gave 30 mg of the title compound.

UPLC-MS (Method A2): Rₜ = 1.03 min

MS (ESIpos): m/z = 435 (M+H)⁺

¹H NMR (400 MHz, DMSO-d6): δ [ppm] = 1.62 (s, 6 H), 3.45 - 3.61 (m, 1 H), 4.48 (t, 2 H), 4.66 (dd, 2 H), 4.72 (d, 2 H), 5.94 (s, 1 H), 7.57 (s, 1 H), 8.16 (d, 1 H), 8.33 - 8.42 (m, 2 H), 8.42 - 8.47 (m, 1 H), 8.72 (s, 1 H), 12.36 (s, 1 H).

### Example 9

N-[6-(Hydroxymethyl)-2-(oxetan-3-ylmethyl)-2H-indazol-5-yl]-6-(trifluoromethyl)pyridine-2-carboxamide

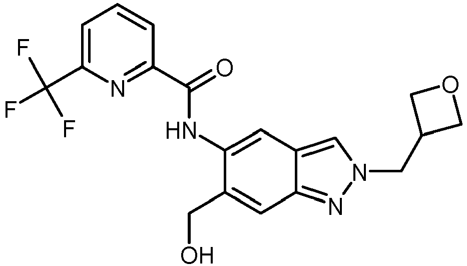

75 mg (0.17 mmol) of methyl 2-(oxetan-3-ylmethyl)-5-({[6-(trifluoromethyl)pyridin-2-yl]carbonyl}amino)-2H-indazole-6-carboxylate (Intermediate 4-1) were dissolved in 1 ml of a mixture of THF/methanol (1:1), and 8 mg (0.21 mmol) of sodium borohydride were added. The mixture was left to stir at 25°C for 60 min. The reaction mixture was concentrated, and the residue was admixed with water. The suspension was stirred vigorously for 15 min, and the solids were filtered off with suction, washed twice with water and twice with diethyl ether, and dried under reduced pressure. This gave 48 mg of the title compound.

UPLC-MS (Method A2): Rₜ = 0.94 min

MS (ESIpos): m/z = 407 (M+H)⁺

¹H NMR (300 MHz, DMSO-d6): δ [ppm] = 3.55 (s, 1 H), 4.48 (t, 2 H), 4.61 - 4.77 (m, 6 H),

7.57 (s, 1 H), 8.18 (dd, 1 H), 8.33 - 8.49 (m, 3 H), 8.51 (s, 1 H), 11.21 (s, 1 H).

### Example 10

N-{6-(2-Hydroxypropan-2-yl)-2-[3-(methylsulphonyl)propyl]-2H-indazol-5-yl}-6-(trifluoromethyl)pyridine-2-carboxamide

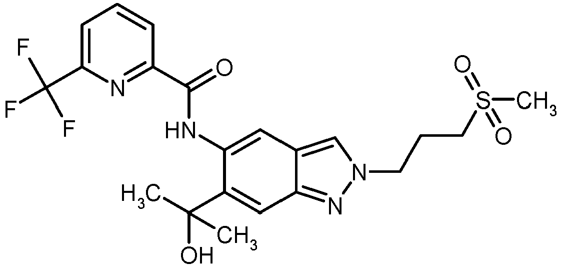

A mixture of 500 mg (1.32 mmol) of N-[6-(2-hydroxypropan-2-yl)-1H-indazol-5-yl]-6-(trifluoromethyl)pyridine-2-carboxamide (Intermediate 5-1), 569 mg of potassium carbonate and 114 mg of potassium iodide in 5.0 ml of DMF was stirred at room temperature for 15 min. 414 mg of 1-bromo-3-(methylsulphonyl)propane were added and the mixture was stirred at room temperature overnight. Water was added, the mixture was twice extracted with ethyl acetate and the extracts were washed with sodium chloride solution and concentrated. The residue was purified by column chromatography (dichloromethane/methanol gradient). Extracting the product fraction by stirring with diethyl ether gave 59 mg of the title compound.

UPLC-MS (Method A2): Rₜ = 1.02 min

MS (ESIpos): m/z = 485 (M+H)⁺

¹H-NMR (300MHz, DMSO-d₆): δ [ppm]= 1.63 (s, 6H), 2.26 - 2.42 (m, 2H), 2.99 (s, 3H), 3.06 - 3.16 (m, 2H), 4.55 (t, 2H), 5.96 (s, 1H), 7.60 (s, 1H), 8.16 (d, 1H), 8.33 - 8.48 (m, 3H), 8.73 (s, 1H), 12.37 (s, 1H).

### Example 11

N-[2-(3-Hydroxy-3-methylbutyl)-6-(2-hydroxypropan-2-yl)-2H-indazol-5-yl]-6-(trifluoromethyl)pyridine-2-carboxamide

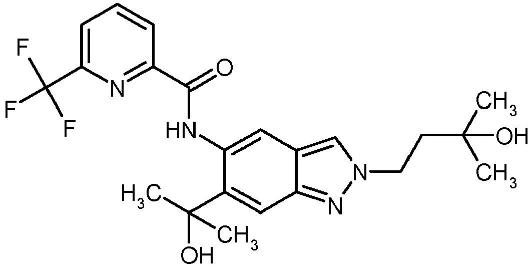

### Preparation Method 1

705 mg (1.57 mmol) of methyl 2-(3-hydroxy-3-methylbutyl)-5-({[6-(trifluoromethyl)pyridin-2-yl]carbonyl}amino)-2H-indazole-6-carboxylate (Intermediate 4-4) were initially charged in 10 ml of THF and cooled in an ice-water cooling bath. 2.6 ml (5.0 equivalents) of 3M methylmagnesium bromide solution (in diethyl ether) were added and the mixture was left to stir while cooling with an ice bath for 1 h and at room temperature for 4.5 h. Another 1 equivalent of the methylmagnesium bromide solution was added and the mixture was left to stir at room temperature for 20.5 h. Another 1 equivalent again of the methylmagnesium bromide solution was added and the mixture was left to stir at room temperature for 22 h. The reaction mixture was admixed with saturated aqueous ammonium chloride solution, stirred and extracted three times with ethyl acetate. The combined organic phases were washed with sodium chloride solution, filtered through a hydrophobic filter and concentrated. This gave 790 mg of a residue which was purified by means of preparative HPLC. This gave 234 mg of the title compound and 164 mg of a product fraction which was extracted by stirring with diethyl ether. After filtration with suction followed by drying, a further 146 mg of the title compound were obtained.

UPLC-MS (Method A1): Rₜ = 1.10 min (UV detector: TIC), mass found 450.00.

¹H-NMR (400MHz, DMSO-d₆): δ [ppm]= 1.14 (s, 6H), 1.61 (s, 6H), 1.99 - 2.08 (m, 2H), 4.42 - 4.55 (m, 3H), 5.93 (s, 1H), 7.56 (s, 1H), 8.15 (dd, 1H), 8.32 - 8.39 (m, 2H), 8.41 - 8.47 (m, 1H), 8.70 (s, 1H), 12.34 (s, 1H).

### Preparation Method 2

A mixture of 500 mg (1.37 mmol) of N-[6-(2-hydroxypropan-2-yl)-1H-indazol-5-yl]-6-(trifluoromethyl)pyridine-2-carboxamide (Intermediate 5-1), 569 mg of potassium carbonate and 114 mg of potassium iodide in 5 ml of DMF was stirred at room temperature for 15 min. 344 mg (1.5 equivalents) of 4-bromo-2-methylbutan-2-ol were added and the mixture was heated to 100°C for 2 h. Another 0.5 equivalent of 4-bromo-2-methylbutan-2-ol was added and the mixture was stirred at room temperature overnight. The mixture was admixed with water and extracted twice with ethyl acetate, and the combined organic phases were washed with saturated sodium chloride solution and filtered through a hydrophobic filter and concentrated. The residue was purified by column chromatography purification on silica gel (hexane/ethyl acetate). This gave 100 mg of a product fraction which was extracted by stirring with diethyl ether. After drying, 60 mg of the title compound were obtained.

¹H-NMR (400MHz, DMSO-d₆): δ [ppm]= 1.14 (s, 6 H), 1.61 (s, 6H), 1.99 - 2.07 (m, 2 H), 4.43 - 4.52 (m, 3 H) 5.94 (s, 1 H) 7.57 (s, 1 H) 8.15 (dd, 1H) 8.33 - 8.40 (m, 2 H), 8.42 - 8.48 (m, 1 H), 8.71 (s, 1 H), 12.35 (s, 1 H).

### Example 12

N-{6-(2-Hydroxypropan-2-yl)-2-[2-(methylsulphonyl)ethyl]-2H-indazol-5-yl}-6-(trifluoromethyl)pyridine-2-carboxamide

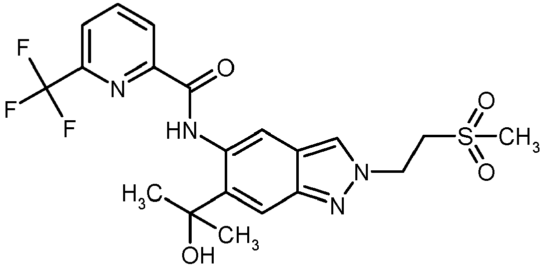

160 mg (0.44 mmol) of N-[6-(2-hydroxypropan-2-yl)-1H-indazol-5-yl]-6-(trifluoromethyl)pyridine-2-carboxamide (Intermediate 5-1) were suspended together with 182 mg of potassium carbonate and 36 mg of potassium iodide in 1.0 ml of DMF, and the mixture was stirred at room temperature for 15 min. Then 123 mg of 2-bromoethyl methyl sulphone were added and the mixture was stirred at room temperature overnight. Water was added, the mixture was extracted twice with ethyl acetate and the extracts were washed with saturated aqueous sodium chloride solution, filtered through a hydrophobic filter and concentrated. Purification of the residue by preparative HPLC gave 20 mg of the title compound.

UPLC (Method A2): Rₜ = 1.01 min;

MS (ESIpos): m/z = 471 (M+H)⁺

¹H NMR (400 MHz, DMSO-d₆): δ [ppm]= 1.63 (s, 6 H), 2.90 (s, 3 H), 3.85 (t, 2 H), 4.86 (t, 2 H), 5.97 (s, 1 H), 7.59 (s, 1 H), 8.13 - 8.19 (m, 1 H), 8.37 (s, 1 H), 8.41 - 8.48 (m, 2 H), 8.74 (s, 1 H), 12.37 (s, 1 H).

### Example 13

6-(Difluoromethyl)-N-[2-(3-hydroxy-3-methylbutyl)-6-(2-hydroxypropan-2-yl)-2H-indazol-5-yl] pyridine-2-carboxamide

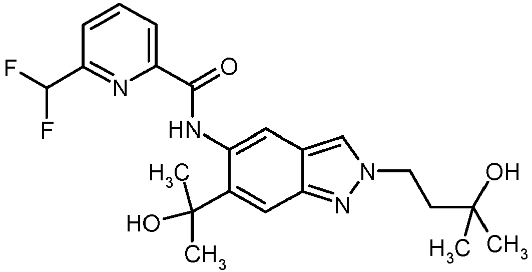

A mixture of 250 mg of 6-(difluoromethyl)-N-[6-(2-hydroxypropan-2-yl)-1H-indazol-5-yl]pyridine-2-carboxamide (crude product of Intermediate 5-2), 144 mg of potassium iodide and 239 mg of potassium carbonate in 2.5 ml of DMF was stirred at room temperature for 15 min. 145 mg (0.87 mmol) of 4-bromo-2-methylbutan-2-ol were added, the mixture was stirred at 110°C for 3 h, another 96 mg of 4-bromo-2-methylbutan-2-ol were added and the mixture was stirred at 110°C for 4 h. Water was added, the mixture was extracted twice with ethyl acetate and the extracts were washed with saturated aqueous sodium chloride solution, filtered through a hydrophobic filter and concentrated. Purification was effected by column chromatography on silica gel (hexane/ethyl acetate). This gave 61 mg of the title compound.

UPLC-MS (Method A1): Rₜ = 1.00 min (UV detector: TIC), mass found 432.00.

¹H-NMR (300MHz, DMSO-d₆): δ [ppm]= 1.14 (s, 6H), 1.63 (s, 6H), 1.97 - 2.08 (m, 2H), 4.41 - 4.55 (m, 3H), 5.99 (s, 1H), 7.03 (t, 1H), 7.56 (s, 1H), 7.94 - 8.00 (m, 1H), 8.24 - 8.38 (m, 3H), 8.71 (s, 1H), 12.49 (s, 1H).

### Example 14

6-(Difluoromethyl)-N-{6-(2-hydroxypropan-2-yl)-2-[2-(methylsulphonyl)ethyl]-2H-indazol-5-yl}pyridine-2-carboxamide

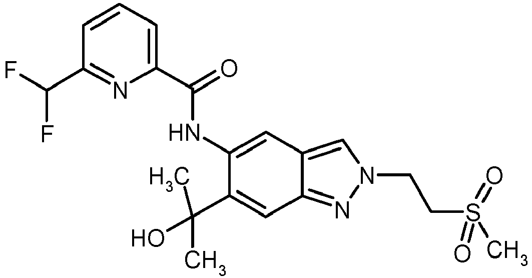

A mixture of 250 mg of 6-(difluoromethyl)-N-[6-(2-hydroxypropan-2-yl)-1H-indazol-5-yl]pyridine-2-carboxamide (crude product of Intermediate 5-2), 144 mg of potassium iodide and 239 mg of potassium carbonate in 2.5 ml of DMF was stirred at room temperature for 15 min. 162 mg of 2-bromoethyl methyl sulphone were added and the mixture was stirred at 110°C for 3 h. Water was added, the mixture was extracted twice with ethyl acetate and the extracts were washed with saturated aqueous sodium chloride solution, filtered through a hydrophobic filter and concentrated. The residue was purified by preparative HPLC and the product fractions were additionally purified by column chromatography purification on silica gel (hexane/ethyl acetate). This gave 40 mg of the title compound.

¹H-NMR (400MHz, DMSO-d₆): δ [ppm]= 1.65 (s, 6H), 2.90 (s, 3H), 3.85 (t, 2H), 4.85 (t, 2H), 6.03 (s, 1H), 7.04 (t, 1H), 7.59 (s, 1H), 7.98 (d, 1H), 8.25 - 8.36 (m, 2H), 8.43 (s, 1H), 8.75 (s, 1H), 12.52 (s, 1H).

### Example 15

6-(Difluoromethyl)-N-[6-(2-hydroxypropan-2-yl)-2-(3-hydroxypropyl)-2H-indazol-5-yl]pyridine-2-carboxamide

### Stage A:

Preparation of N-[2-(3-{[tert-butyl(dimethyl)silyl]oxy}propyl)-6-(2-hydroxypropan-2-yl)-2H-indazol-5-yl]-6-(difluoromethyl)pyridine-2-carboxamide

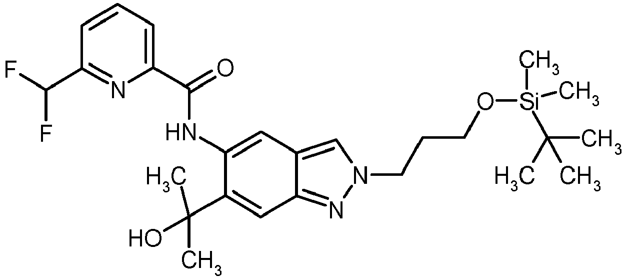

A mixture of 250 mg of 6-(difluoromethyl)-N-[6-(2-hydroxypropan-2-yl)-1H-indazol-5-yl]pyridine-2-carboxamide (Intermediate 5-2), 48 mg of potassium iodide and 239 mg of potassium carbonate in 2.5 ml of DMF was stirred at room temperature for 15 min. 219 mg (0.87 mmol, 1.5 equivalents) of (3-bromopropoxy)(tert-butyl)dimethylsilane were added and the mixture was stirred at 110°C for 3 h. 1 equivalent of (3-bromopropoxy)(tert-butyl)dimethylsilane was added and the mixture was stirred at 100°C for 4 h. Water was added, the mixture was extracted with ethyl acetate and the extract was washed with aqueous sodium chloride solution, filtered through a hydrophobic filter and concentrated. The residue was purified by column chromatography (hexane/ethyl acetate). This gave 92 mg of the title compound.

### Stage B:

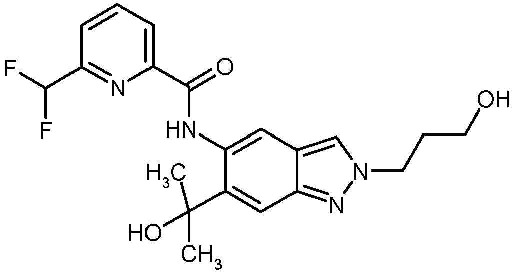

Analogously to the preparation of Example 6, Stage B, 92 mg of N-[2-(3-{[tert-butyl(dimethyl)silyl]oxy}propyl)-6-(2-hydroxypropan-2-yl)-2H-indazol-5-yl]-6-(difluoromethyl)pyridine-2-carboxamide were reacted with 0.53 ml of a 1 M solution of tetrabutylammonium fluoride in THF within 1 h. Aqueous workup as in Example 6 and purification by preparative HPLC gave 46 mg of the title compound. UPLC-MS (Method A1): Rₜ = 0.92 min (UV detector: TIC), mass found 404.00.

¹H-NMR (400MHz, DMSO-d₆): δ [ppm]= 1.64 (s, 6H), 2.05 (quin, 2H), 3.35 - 3.46 (m), 4.45 (t, 2H), 4.64 (t, 1H), 5.99 (s, 1H), 7.04 (t, 1H), 7.57 (s, 1H), 7.95 - 7.99 (m, 1H), 8.25 - 8.36 (m, 3H), 8.73 (s, 1H), 12.50 (s, 1H).

### Example 16

N-[6-(2-Hydroxypropan-2-yl)-2-(4,4,4-trifluorobutyl)-2H-indazol-5-yl]-6-(trifluoromethyl)pyridine-2-carboxamide

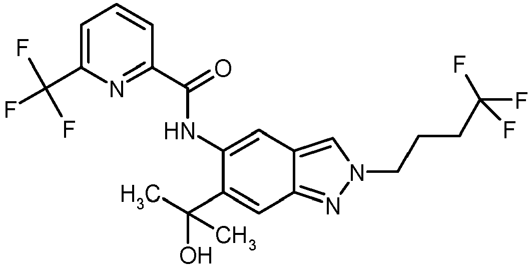

A mixture of 210 mg (0.58 mmol) of N-[6-(2-hydroxypropan-2-yl)-1H-indazol-5-yl]-6-(trifluoromethyl)pyridine-2-carboxamide (Intermediate 5-1) in 3 ml of DMF was admixed with 0.11 ml of 1,1,1-trifluoro-4-iodobutane and 239 mg of potassium carbonate, and the mixture was stirred at 80°C for 6 h. After addition of water, the mixture was extracted three times with ethyl acetate, and the combined organic phases were washed with saturated sodium chloride solution, filtered through a hydrophobic filter and concentrated. The crude product was purified by preparative HPLC. This gave 19 mg of the title compound.

UPLC-MS (Method A1): Rₜ = 1.27 min (UV detector: TIC), mass found 474.15.

¹H-NMR (400MHz, DMSO-d₆): δ [ppm]= 1.62 (s, 6H), 2.10 - 2.33 (m), 4.49 (t, 2H), 5.94 (s, 1H), 7.59 (s, 1H), 8.13 - 8.18 (m, 1H), 8.32 - 8.41 (m, 2H), 8.41 - 8.47 (m, 1H), 8.72 (s, 1H), 12.35 (s, 1H).

### Example 17

N-{6-(2-Hydroxypropan-2-yl)-2-[3-(trifluoromethoxy)propyl]-2H-indazol-5-yl}-6-(trifluoromethyl)pyridine-2-carboxamide

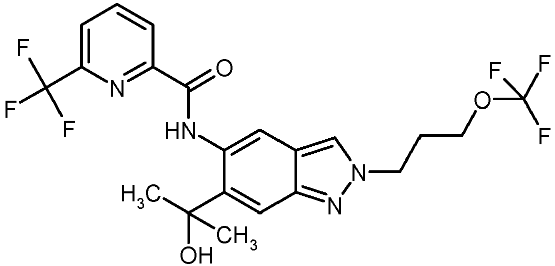

150 mg of N-[6-(2-hydroxypropan-2-yl)-1H-indazol-5-yl]-6-(trifluoromethyl)pyridine-2-carboxamide (Intermediate 5-1) were initially charged in 2 ml of THF. 58 mg of 3-(trifluoromethoxy)propan-1-ol, 131 mg of triphenylphosphine and 71 µl of diisopropyl azodicarboxylate (DIAD, CAS 2446-83-5) were added and the mixture was stirred at room temperature for 19 h. 0.83 ml of sodium hydroxide solution (2M) was added and the mixture was stirred at 40°C for 5 h. The mixture was diluted with water and extracted three times with ethyl acetate, and the combined organic phases were concentrated and purified by preparative HPLC. 16 mg of the title compound were obtained as a crude product.

UPLC-MS (Method A2): Rₜ = 1.26 min (UV detector: TIC), mass found 490.14.

¹H-NMR (400MHz, DMSO-d₆, selected signals): δ [ppm]= 1.61 (s, 6H), 1.84 (d, 1H), 2.32 (quint., 2H), 4.08 (t, 2H), 4.51 (t, 2H), 7.58 (s, 1H), 8.15 (d, 1H), 8.31 - 8.39 (m, 2H), 8.44 (d, 1H), 8.72 (s, 1H), 12.35 (s, 1H).

### Example 18

N-{6-(2-Hydroxypropan-2-yl)-2-[3-(2,2,2-trifluoroethoxy)propyl]-2H-indazol-5-yl}-6-(trifluoromethyl)pyridine-2-carboxamide

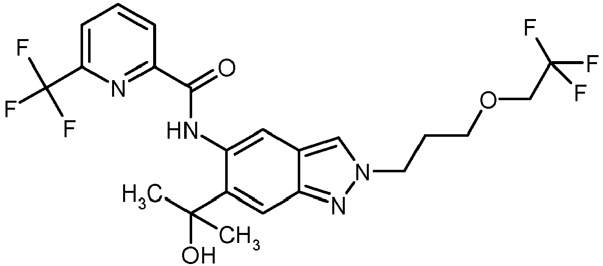

Analogously to the preparation of Example 11 (Preparation Method 1), 52 mg of methyl 2-[3-(2,2,2-trifluoroethoxy)propyl]-5-({[6-(trifluoromethyl)pyridin-2-yl]carbonyl}amino)-2H-indazole-6-carboxylate (Intermediate 4-10) in 3 ml of THF were reacted with 2 × 171 microlitres of 3M magnesium bromide solution in diethyl ether. Purification by preparative HPLC gave 12 mg of the title compound.

UPLC-MS (Method A1): Rₜ = 1.25 min (UV detector: TIC), mass found 504.16.

¹H-NMR (500 MHz, DMSO-d₆): δ [ppm]= 1.63 (s, 6H), 2.20(quin, 2H), 3.58(t, 2H),4.05(q, 2H), 4.47(t, 2H),5.94(s, 1H), 7.58 (s, 1H), 8.15 (dd, 1H), 8.32 (s, 1H), 8.36 (t, 1H), 8.45(d, 1H), 8.73 (s, 1H), 12.36 (s,1H).

### Example 19

5-Fluoro-N-[2-(3-hydroxy-3-methylbutyl)-6-(2-hydroxypropan-2-yl)-2H-indazol-5-yl]-6-methylpyridine-2-carboxamide

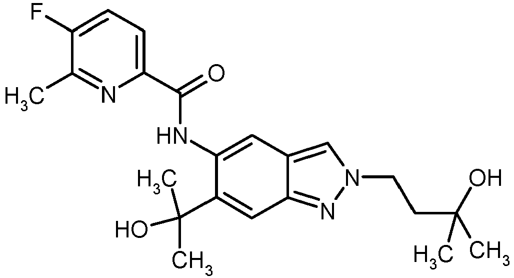

228 mg of methyl 5-{[(5-fluoro-6-methylpyridin-2-yl)carbonyl]amino}-2-(3-hydroxy-3-methylbutyl)-2H-indazole-6-carboxylate (Intermediate 4-8) were initially charged in 4.5 ml of THF and cooled with an ice cooling bath. 0.63 ml of 3M methylmagnesium bromide solution (in diethyl ether) was added and the mixture was left to stir while cooling with an ice bath for 2 h and at room temperature for 21 h. The reaction mixture was admixed with saturated aqueous ammonium chloride solution and extracted three times with ethyl acetate. The combined organic phases were concentrated. The residue was purified by preparative HPLC. This gave 82 mg of the title compound.

UPLC-MS (Method A2): Rₜ = 1.03 min (UV detector: TIC), mass found 414.21.

¹H-NMR (400MHz, DMSO-d₆): δ [ppm]= 1.13 (s, 6H), 1.63 (s, 6H), 1.99 - 2.05 (m, 2H), 2.55 - 2.59 (m, 3H), 4.42 - 4.50 (m, 3H), 5.95 (s, 1H), 7.54 (s, 1H), 7.83 (t, 1H), 8.05 (dd, 1H), 8.31 (s, 1H), 8.68 (s, 1H), 12.33 (s, 1H).

### Example 20

N-[2-(3-Hydroxy-3-methylbutyl)-6-(2-hydroxypropan-2-yl)-2H-indazol-5-yl]-6-methylpyridine-2-carboxamide

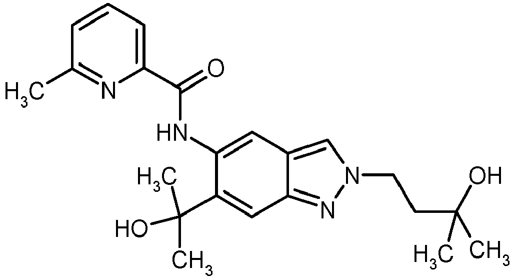

278 mg of methyl 2-(3-hydroxy-3-methylbutyl)-5-{[(6-methylpyridin-2-yl)carbonyl]amino}-2H-indazole-6-carboxylate (Intermediate 4-9) were initially charged in 5.0 ml of THF and cooled with an ice cooling bath. 0.97 ml of 3M methylmagnesium bromide solution (in diethyl ether) was added and the mixture was left to stir while cooling with an ice bath for 2 h and at room temperature for 20.5 h. Another 0.48 ml of 3M methylmagnesium bromide solution was added and the mixture was left to stir at room temperature for 67 h. The mixture was admixed with saturated aqueous ammonium chloride solution and extracted three times with ethyl acetate, and the extracts were washed with sodium chloride solution, filtered through a hydrophobic filter and concentrated. The residue was purified by preparative HPLC. This gave 111 mg of the title compound.

UPLC-MS (Method A2): Rₜ = 0.97 min (UV detector: TIC), mass found 396.22.

¹H-NMR (400MHz, DMSO-d₆): δ [ppm]= 1.15 (s, 6H), 1.64 (s, 6H), 2.00 - 2.08 (m, 2H), 2.61 (s, 3H), 4.41 - 4.59 (m, 3H), 5.92 (s, 1H), 7.50 (dd, 1H), 7.56 (s, 1H), 7.90 - 7.99 (m, 2H), 8.33 (s, 1H), 8.70 (s, 1H), 12.39 (s, 1H).

### Example 21

6-(2-Hydroxypropan-2-yl)-N-[6-(2-hydroxypropan-2-yl)-2-(4,4,4-trifluorobutyl)-2H-indazol-5-yl]pyridine-2-carboxamide

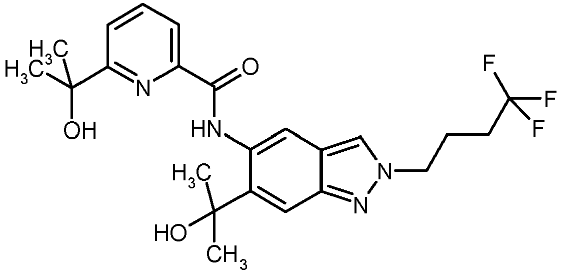

A solution of 72 mg (0.155 mmol) of methyl 5-({[6-(2-hydroxypropan-2-yl)pyridin-2-yl]carbonyl}amino)-2-(4,4,4-trifluorobutyl)-2H-indazole-6-carboxylate (Intermediate 4-7) in 10 ml of THF was cooled in an ice/water cooling bath. 0.26 ml of 3M methylmagnesium bromide solution in diethyl ether was added and the mixture was stirred for 2 h and then at room temperature for 20 h. Another 1 equivalent of the 3M methylmagnesium bromide solution was added and the mixture was stirred at room temperature for 24 h. Saturated aqueous ammonium chloride solution was added, the mixture was three times extracted with ethyl acetate and the extracts were washed with sodium chloride solution and concentrated. Preparative HPLC gave 22 mg (31% of theory) of the title compound. UPLC-MS (Method A2): Rₜ = 1.15 min (UV detector: TIC), mass found 464.20.

¹H-NMR (400MHz, DMSO-d₆): δ [ppm]= 1.56 (s, 6H), 1.64 (s, 6H), 2.07 - 2.34 (m, 4H), 4.49 (t, 2H), 5.32 (s, 1H), 6.05 (s, 1H), 7.60 (s, 1H), 7.87 (dd, 1H), 7.99 - 8.05 (m, 2H), 8.35 (s, 1H), 8.79 (s, 1H), 12.45 (s, 1H).

### Assessment of physiological efficacy

### IRAK4 kinase assay

The IRAK4-inhibitory activity of the component A compounds as constituents of the inventive combinations was measured in the Irak4TR-FRET assay (TR-FRET = Time Resolved Fluorescence Resonance Energy Transfer) described hereinafter.

Recombinant fusion protein from N-terminal GST (glutathione S-transferase) and human Irak4, expressed in baculovirus-infected insect cells (Hi5, BTI-TN-5B1-4, cell line purchased from Invitrogen, catalogue No. B855-02) and purified via affinity chromatography, was used as enzyme. The substrate used for the kinase reaction was the biotinylated peptide biotin-Ahx-KKARFSRFAGSSPSQASFAEPG (C-terminus in amide form) which can be purchased, for example, from Biosyntan GmbH (Berlin-Buch).

For the assay, 11 different concentrations in the range from 20 µM to 0.073 nM were prepared from a 2 mM solution of the test substance in DMSO. 50 nl of the respective solution were pipetted into a black low-volume 384-well microtitre plate (Greiner Bio-One, Frickenhausen, Germany), 2 µl of a solution of Irak4 in assay buffer [50 mM HEPES pH 7.5, 5 mM MgCl2, 1.0 mM dithiothreitol, 30 µM activated sodium orthovanadate, 0.1% (w/v) of bovine gamma-globulin (BGG) 0.04% (v/v) nonidet-P40 (Sigma)] were added and the mixture was incubated for 15 min to allow prebinding of the substances to the enzyme prior to the kinase reaction. The kinase reaction was then started by addition of 3 µl of a solution of adenosine triphosphate (ATP, 1.67 mM = final concentration in 5 µl of assay volume: 1 mM) and peptide substrate (0.83 µM = final concentration in 5 µl assay volume: 0.5 µM) in assay buffer, and the resulting mixture was incubated at 22°C for the reaction time of 45 min. The concentration of the Irak4 was adjusted to the respective activity of the enzyme and set such that the assay was carried out in the linear range. Typical concentrations were in the order of about 0.2 nM. The reaction was stopped by addition of 5 µl of a solution of TR-FRET detection reagents [0.1 µM streptavidin-XL665 (Cisbio Bioassays; France, catalogue No. 610SAXLG)] and 1.5 nM anti-phosphoserine antibody [Merck Millipore, "STK Antibody", catalogue No. 35-002] and 0.6 nM LANCE EU-W1024-labelled anti-mouse-IgG antibody (Perkin-Elmer, product No. AD0077; alternatively, it is possible to use a terbium cryptate-labelled anti-mouse-IgG antibody from Cisbio Bioassays) in aqueous EDTA solution (100 mM EDTA, 0.4 % [w/v] bovine serum albumin [BSA] in 25 mM HEPES pH 7.5).

The resulting mixture was incubated at 22°C for 1 h to allow formation of a complex of the biotinylated phosphorylated substrate and the detection reagents. The amount of the phosphorylated substrate was then evaluated by measuring the resonance energy transfer from europium chelate-labelled anti-mouse-IgG antibody to streptavidin-XL665. To this end, the fluorescence emissions at 620 nm and 665 nm were measured after excitation at 350 nm in a TR-FRET measuring instrument, for example a Rubystar (BMG Labtechnologies, Offenburg, Germany) or a Viewlux (Perkin-Elmer). The ratio of the emissions at 665 nm and 622 nm was taken as a measure of the amount of phosphorylated substrate. The data were normalized (enzyme reaction without test substance = 0% inhibition; all other assay components but no enzyme = 100% inhibition). Typically, the test substances were tested on the same microtitre plates at 11 different concentrations in the range from 20 µM to 0.073 nM (20 µM, 5.7 µM, 1.6 µM, 0.47 µM, 0.13 µM, 38 nM, 11 nM, 3.1 nM, 0.89 nM, 0.25 nM and 0.073 nM). The dilution series were prepared prior to the assay (2 mM to 7.3 nM in 100% DMSO) by serial dilutions. The IC50 values were calculated using a 4-parameter fit.

**Table 1: IC₅₀ values of the component A compounds as constituents of the inventive combinations in the IRAK4 kinase assay**

| Example | IC₅₀ [nM] |
|---|---|
| 1 | 30.6 |
| 2 | 135.6 |
| 3 | 7.2 |
| 4 | 52.7 |
| 5 | 264.5 |
| 6 | 35.7 |
| 7 | 867.3 |
| 8 | 15.0 |
| 9 | 103.8 |
| 10 | 18.5 |
| 11 | 3.4 |
| 12 | 10.7 |
| 13 | 1.3 |
| 14 | 10.8 |
| 15 | 12.3 |
| 16 | 21.5 |
| 17 | 36.0 |
| 18 | 47.5 |
| 19 | 8.9 |
| 20 | 13.3 |
| 21 | 117.2 |

### TNF-α secretion in THP-1 cells

With the aid of this test, it is possible to test substances for their ability to inhibit secretion of TNF-α (tumour necrosis factor alpha) in THP-1 cells (human monocytic acute leukaemia cell line). TNF-α is a cytokine involved in inflammatory processes. In this test, TNF-α secretion is triggered by incubation with bacterial lipopolysaccharide (LPS).

THP-1 cells are kept in continuous suspension cell culture [RPMI 1460 medium with L-Glutamax (Gibco, Cat No. 61870-044) supplemented with foetal calf serum (FCS) 10% (Invitrogen, Cat No. 10082-147), 1% penicillin/streptomycin (Gibco BRL, Cat No. 15140-114)] and should not exceed a cell concentration of 1×10⁶ cells/ml. The assay is carried out in cell culture medium (RPMI 1460 medium with L-Glutamax supplemented with FCS 10%). In each case 2-2.5 µl of the cell suspension (corresponds to 4000 cells) per well were dispensed into a 384-well test plate (Greiner, Cat No. 784076), in each of which 40-50 nl substance had been dissolved in 100% DMSO. Here, in each case 10 different concentrations in the range from 20 µM to 0.073 nM were used for each substance. The cells were incubated at room temperature for 15 min. 2-2.5 µl of 0.1 µg/ml LPS (Sigma, *Escherichia coli* 055:B5, Cat. No. L5418) dissolved in cell culture medium (final concentration 0.05 µg/ml) were then dispensed into each well. As a neutral control, cells were treated with 0.05 µg/ml LPS and 1% DMSO and, as inhibitor control, only once with 1% DMSO.

The plates are centrifuged at 80 g for 30 s and incubated at 37°C, 5% CO₂ and 95% atmospheric humidity for 17 h. The amount of TNF-α was determined using the TNF-alpha HTRF Detection Kit (Cisbio, Cat No. 62TNFPEB/C). To this end, in each case 2 µl of the detection solution consisting of anti-TNF-α-XL665 conjugate and anti-TNF-α-cryptate conjugate, dissolved in accordance with the manufacturer's instructions in the reconstitution buffer, were added for the HTRF (Homogeneous Time-Resolved Fluorescence) test. After the addition, the mixture was incubated either at room temperature for 3 h or at 4°C overnight. The signals were then read at 620/665 nm using an HTRF-enabled measuring instrument such as the BMG PheraStar.

The activity of the substances is expressed as the ratio between neutral and inhibitor control in per cent. The IC₅₀ values were calculated using a 4-parameter fit.

**Table 2: IC₅₀ values of the component A compounds with respect to the secretion of TNF-α in THP-1 cells**

| Example | IC₅₀ [µM] |
|---|---|
| 1 | 1.0 |
| 2 | 15.1 |
| 3 | 0.7 |
| 4 | 5.6 |
| 5 | 5.4 |
| 6 | 0.9 |
| 7 | 16.4 |
| 8 | 1.0 |
| 9 | 6.5 |
| 10 | 1.0 |
| 11 | 0.2 |
| 12 | 0.3 |
| 13 | 0.1 |
| 14 | 0.2 |
| 15 | 0.2 |
| 16 | 0.2 |
| 17 | 0.5 |
| 18 | 0.3 |
| 19 | 0.1 |
| 20 | 0.2 |
| 21 | 1.8 |

### In Vitro LPS (lipopolysaccharide)-induced cytokine production in human PBMCs (peripheral blood mononuclear cells)

The effect of the component A compounds as constituents of the inventive combinations on induced cytokine production in human PBMCs was examined. Here, cytokine production was induced by LPS, a TLR4 ligand, which leads to activation of the IRAK4-mediated signalling pathway.

The human PBMCs were obtained from anti-coagulated human whole blood. To this end, 15 ml of Ficoll-Paque (Biochrom, Cat. No. L6115) were initially charged in Leucosep tubes and 20 ml of human blood were added. After centrifugation of the blood at 800 g for 15 min at room temperature, the plasma including the platelets was removed and discarded. The PBMCs were transferred into centrifugation tubes and made up with PBS (phosphatebuffered saline) (Gibco, Cat. No. 14190). The cell suspension was centrifuged at room temperature at 250 g for 10 min and the supernatant was discarded. The PBMCs were resuspended in complete medium (RPMI 1640, without L-glutamine (PAA, Cat. No. E15-039), 10% FCS; 50 U/ml penicillin, 50 µg/ml streptomycin (PAA, Cat. No. P11-010) and 1% L-glutamine (Sigma, Cat. No. G7513)).

The assay was also carried out in complete medium. The PBMCs were sown in 96-well plates at a cell density of 2.5×10⁵ cells/well. The compounds were subjected to serial dilution in a constant volume of 100% DMSO and employed in the assay at 8 different concentrations in the range from 10 µM to 3 nM such that the final DMSO concentration was 0.4% DMSO. Prior to the actual stimulation, the cells were then pre-incubated therewith for 30 min. To induce cytokine secretion, the cells were stimulated with 0.1 µg/ml LPS (Sigma, *Escherichia coli* 0128:B12, Cat. No. L2887) for 24 hours. Cell viability was determined using the CellTiter-Glo luminescent assay (Promega, Cat. No. G7571 (G755/G756A)) in accordance with the manufacturer's instructions. The amount of secreted TNF- 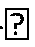 in the cell culture supernatant was determined using the Human ProInflammatory 9-Plex Tissue Culture Kit (MSD, Cat. No. K15007B) in accordance with the manufacturer's instructions. Mentioned by way of example are Example Compound 11 and Example Compound 12 of activity ≤ 1 µM.

### In vivo B-cell lymphoma-associated xenotransplantation model

The anti-tumour activity of the component A compounds as constituents of the inventive combinations was examined in murine xenotransplantation models. For this purpose, female C.B-17 SCID mice were implanted subcutaneously with tumour cell lines of human B-cell lymphoma, e.g. TMD-8. At a mean tumour size of 20-30 mm², monotherapeutic treatment or treatment in combination with the standard ibrutinib treatment commenced, each of which were administered orally. This was preceded by randomization of the animals. The treatment was ended as soon as the untreated control group had tumours of area ≤ 150 mm². The tumour size and the body weight were determined weekly for three weeks. Changes in the body weight were a measure of treatment-related toxicity (> 10% = critical, stoppage in treatment until recovery, > 20% = toxic, termination). The tumour area was detected by means of an electronic caliper gauge [length (mm) x width (mm)]. The antitumour efficacy defined the ratio of the tumour area of treatment versus control [tumour area of the treatment group on day X/tumour area of the control group on day X]. The compound having a T/C greater than 0.5 was defined as active (effective). Statistical analysis was effected using single-factor ANOVA and comparison with the control group by means of pair-by-pair comparative analysis (Dunnett's test).

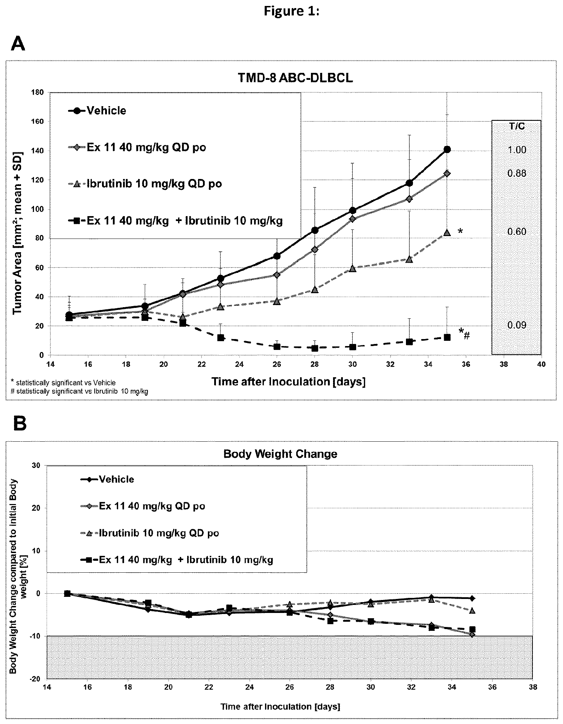

Figure 1 shows the efficacy of Example Compound 11 in monotherapy and in combination with ibrutinib in the treatment of human TMD-8 ABC-DLBCL tumours. TMD-8 tumour cells were implanted subcutaneously on day 0 into female C.B-17 SCID mice. The treatment was started on day 15 at a tumour area of about 26 mm². Example Compound 11 was administered orally at a daily dosage of 40 mg/kg. Ibrutinib was likewise administered orally at a daily dosage of 10 mg/kg. Tumour growth was assessed by determining the tumour area (see Figure 1, upper part A), and the health of the animals by determining the body weight (see Figure 1, lower part B).

Example Compound 11 did not show any inhibitory effect on the tumour growth of TMD-8 when it was administered as monotherapy. Ibrutinib in monotherapy showed a moderate inhibitory effect on the tumour growth of TMD-8, with a T/C value of 0.60, but one which was statistically significant compared to the control group. In the combination treatment comprising Example Compound 11 and ibrutinib, a significant rise in antitumour action was recorded, which is reflected in the T/C value of 0.09 and a statistically significant reduction in tumour area compared to the control and ibrutinib.

The treatments were very well tolerated; no critical weight loss was recorded.

In summary, it was shown in this study that the combination of Example Compound 11 with the BTK inhibitor ibrutinib can achieve a distinct rise in the antitumour effect of the respective monotherapies in the model of an ABC-DLBCL.

### Figure 1:

Antitumour activity of Example Compound 11 in monotherapy and in combination with ibrutinib in TMD-8 C.B-17 SCID mice

| TMD-8: human ABC-DLBCL xenograft model | | | | |
|---|---|---|---|---|
| Study No. | ONC2014.00714 | | | |
| Substance | Dosage | T/C^{a} area | Max. weight loss^{b} (%) | Toxicity |
| Vehicle | 10 ml/kg QD p.o. + 10 ml/kg QD p.o. | 1.00 | - | 0/8 |
| Example 11 | 40 mg/kg QD p.o. | 0.88 | -9 | 0/8 |
| Ibrutinib | 10 mg/kg QD p.o. | 0.60^{#} | -5 | 0/8 |
| Example 11 + Ibrutinib | 40 mg/kg QD p.o. + 10 mg/kg QD p.o. | 0.09^{∗#} | -8 | 0/8 |

| | | | | |
|---|---|---|---|---|
| ^{∗} P < 0.05 (compared to vehicle control) ^{#} P < 0.05 (compared to ibrutinib monotherapy) a) T/C = ratio of the tumour area of treatment versus control [tumour area of the treatment group on day X/tumour area of the control group on day X]. b) Loss of body weight: Changes in body weight compared to the initial body weight at the start of treatment (> 10% = critical, stoppage in treatment until recovery, > 20% = toxic, termination). | | | | |

Figure 1 shows the antitumour activity of Example Compound 11 in monotherapy and in combination with ibrutinib in TMD-8 C.B-17 SCID mice. Legend for Figure 1: The abbreviation Ex means Example, QD means once per day and po means peroral.

### Cell proliferation measurement

The antiproliferative activity of the compounds of the general formula (I) as constituents of the inventive combinations was examined in vitro in human ABC-DLBCL cells. For this purpose 4000 TMD-8 or HBL-1 cells (both from ATCC) or OCI-LY10 at 30 µl/cavity in growth medium (RPMI (Biochrom: FG 1215), 20% FCS (Biochrom: S 0615)) were transferred into a 384-cavity plate (Perkin Elmer, white) and incubated at 37°C overnight. After 24 h, cells on one plate (0 h plate) were treated with 30 µl/cavity of CTG solution (Promega Cell Titer Glo (catalogue # G755B and G756B)) and incubated at room temperature for 10 min, and luminescence was measured by means of a VICTOR V (Perkin Elmer), in order to determine cell viability on commencement of treatment. The cells on the test plate were treated with the compounds of the general formula (I) as constituents of the inventive combinations and incubated at 37°C for 72 h. The compounds were added to the cells by means of an HP D300 digital dispenser in a 7-fold dilution series - either alone or as a combination of two compounds of different concentrations (ratios of substance 1 (example compound of the general formula (I) as constituent of the inventive combinations) and substance 2 (BTK inhibitors ibrutinib or RN486 or AVL-292 or AVL-292 as constituent of the inventive combinations): 1:0; 0.85:0.15; 0.7:0.3; 0.5:0.5; 0.3:0.7; 0.15:0.85; 0:1). As control, the cells were treated with vehicle (DMSO). After 72 h, the cells were treated with 30 µl/cavity of CTG solution (Promega Cell Titer Glo (catalogue # G755B and G756B)) and incubated at room temperature for 10 min, and luminescence was measured by means of a VICTOR V (Perkin Elmer), in order to determine cell viability at the end of treatment. The percentage effect on cell growth and the IC50 derived therefrom were determined for each test substance using the values from the 0 h plate (= maximum inhibition) and the DMSO control (= minimum inhibition). The IC50 values were calculated using a 4-parameter fit. The combinatorial effect to test substances was determined on the basis of the above-described IC50 determination. The combination index (CI) was calculated on the basis of Chou's formula (Chou TC et al., Pharmacological Reviews September 2006). This index allows a quantitative determination of substance interactions. A Cl < 1, =1, and > 1 respectively describe synergistic, additive and antagonistic effects. Visualization is effected by means of isobolograms.

In the combination treatment with Example Compound 3 or 11 or 12 or 13 or 19 and ibrutinib or RN486 or AVL-292 or CGI-1746, a distinct rise in antitumour action was almost always recorded compared to single treatments.

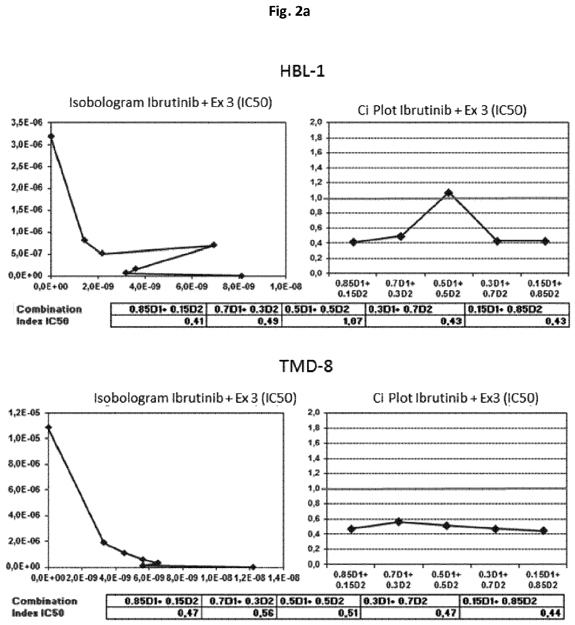

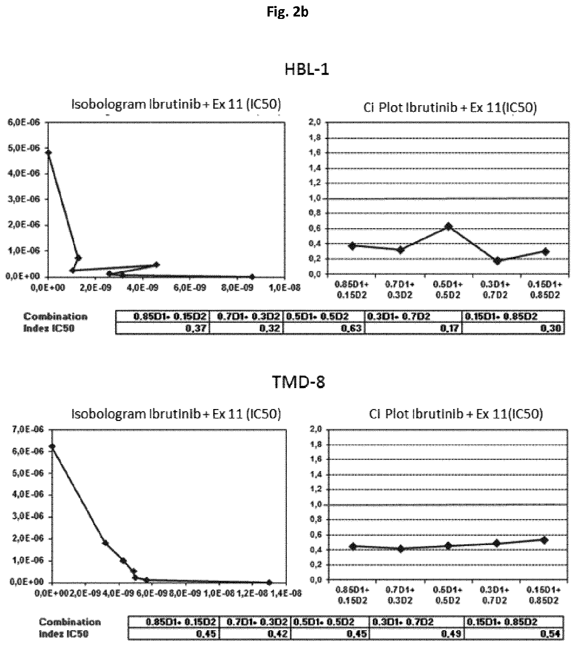

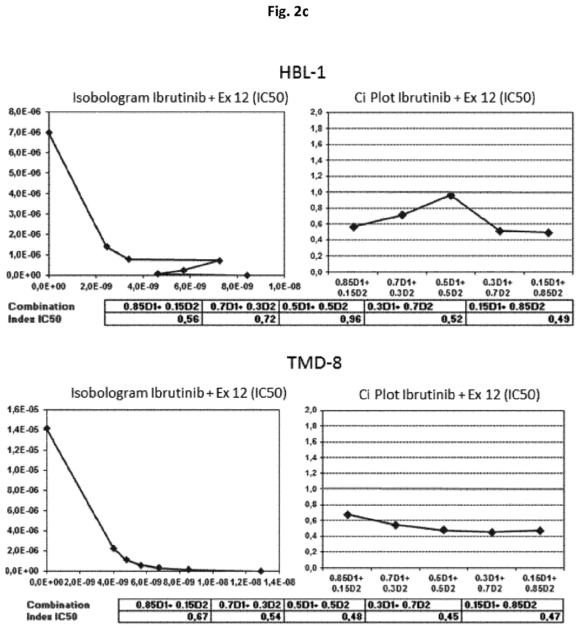

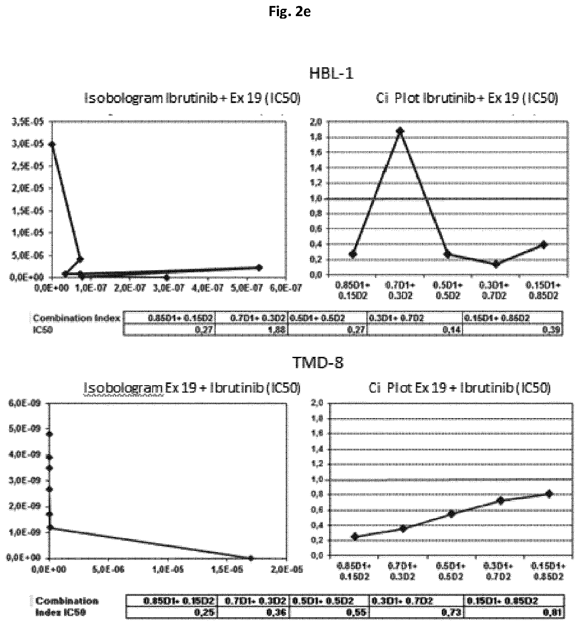

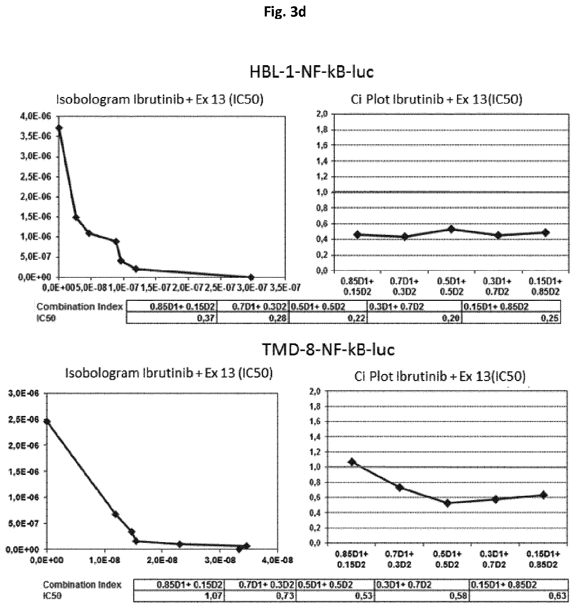

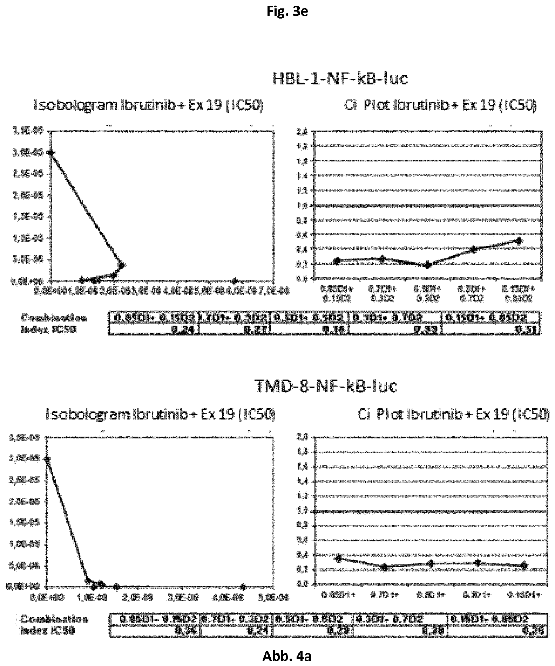

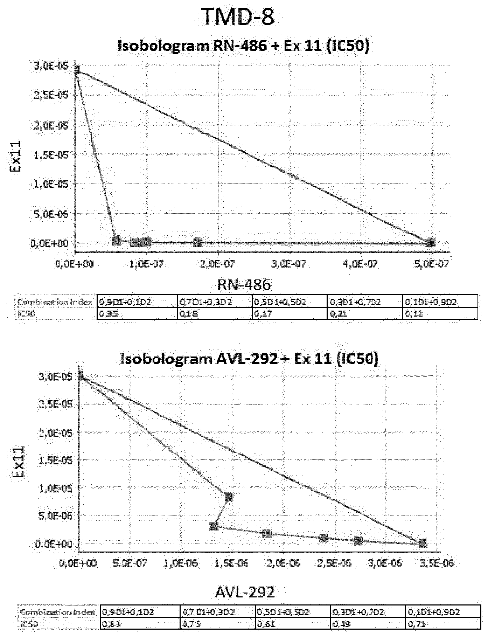

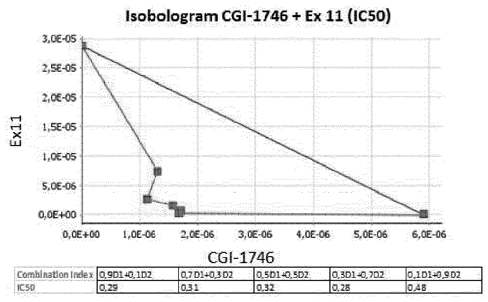

Figures 2a to 2e and 4a to c (Ex means example compound) show the results of a combinatorial cell proliferation measurement in ABC-DLBCLcell lines TMD-8 and HBL-1 and OCI-LY10 on combination of BTK inhibitors ibrutinib or RN486 or AVL-292 or CGI-1746 with the compounds of the general formula (I) (Figure 2a: Ex 03; Figure 2b: Ex 11; Figure 2c: Ex 12; Figure 3d: Ex 13, Figure 2e: Ex 19). IC50 isobolograms for the various combinations with the respective concentrations of substance 1 (D1) (example compound of the general formula (I) as constituents of the inventive combinations) on the y axis and substance 2 (D2) (BTK inhibitors Ibrutinib or RN486 or AVL-29 or CGI-1746 as constituents of the inventive combinations) on the x axis are shown. Data points below, on and above the hypotenuse respectively indicate synergistic, additive and antagonistic effects on cell proliferation.

Legend for Fig. 2a: Effect of the combination of ibrutinib with Example 03 on cell viability of TMD-8 and HBL-1 cells.

Legend for Fig. 2b: Effect of the combination of ibrutinib with Example 11 on cell viability of TMD-8 and HBL-1 cells.

Legend for Fig. 2c: Effect of the combination of ibrutinib with Example 12 on cell viability of TMD-8 and HBL-1 cells.

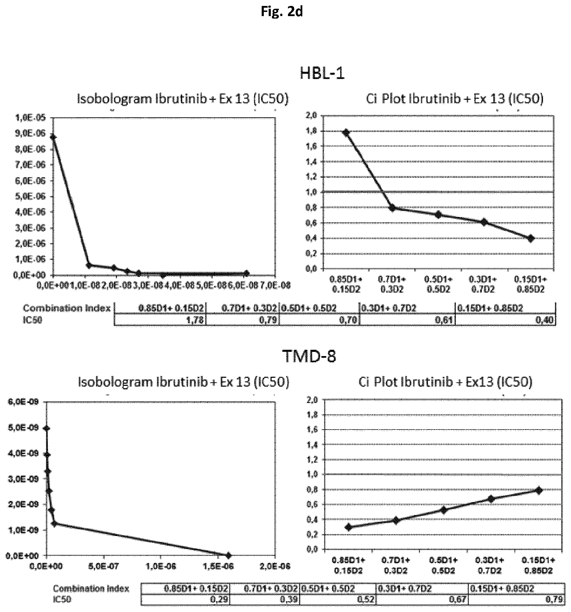

Legend for Fig. 2d: Effect of the combination of ibrutinib with Example 13 on cell viability of TMD-8 and HBL-1 cells.

Legend for Fig. 2e: Effect of the combination of ibrutinib with Example 19 on cell viability of TMD-8 and HBL-1 cells.

Legend Fig. 4a: Effect of combination of BTK inhibitors RN468 or AVL-292 or CGI-1746 with example 11 on cell viability of TMD-8 cells.

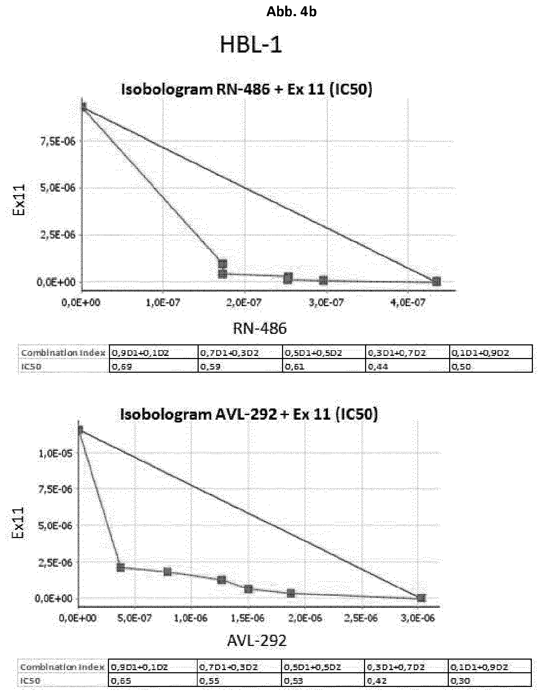

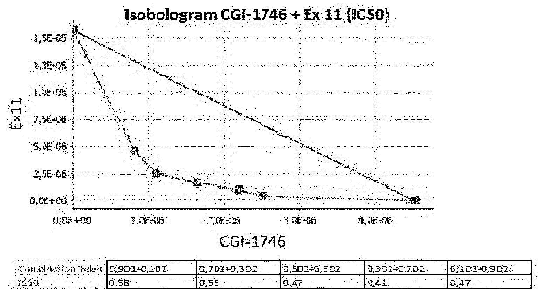

Legend Fig. 4b: Effect of combination of BTK inhibitors RN468 or AVL-292 or CGI-1746 with example 11 on cell viability of HBL-1 cells.

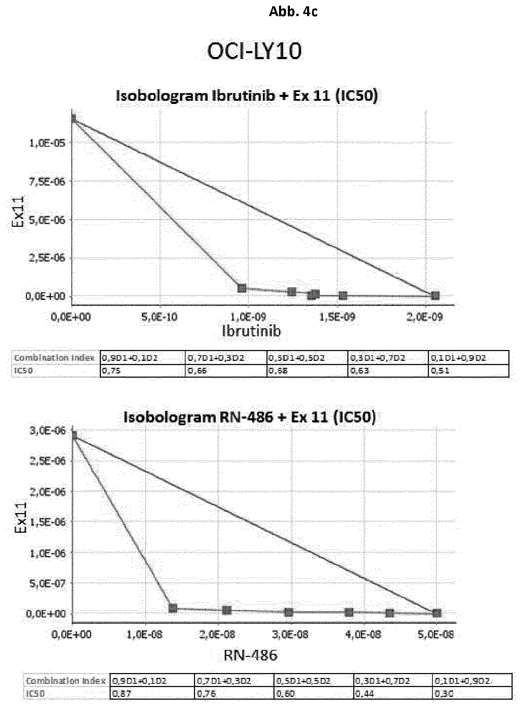

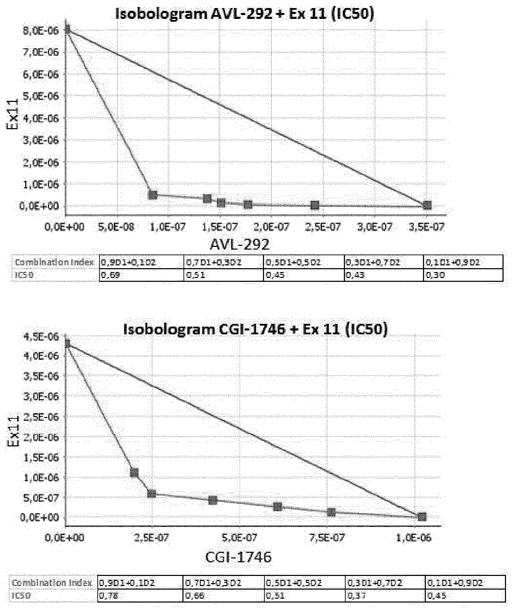

Legend Fig. 4c: Effect of combination of BTK inhibitors RN468 or AVL-292 or CGI-1746 with example 11 on cell viability of OCI-LY10 cells.

### NF-kB reporter assay

The effect of the compounds of the general formula (I) as constituents of the inventive combinations on the NF-kB signalling pathway was examined in vitro in human DLBCL cells. 10 000 TMD-8-NF-kB-luc cells or 10 000 HBL-1-NF-kB-luc reporter cells at 30 µl/cavity in growth medium (RPMI (Biochrom: FG 1215), 20% FCS (Biochrom: S 0615)) were transferred into a 384-cavity plate (Perkin Elmer, white) and incubated at 37°C overnight. After 24 h, the cells were treated with the test substances and incubated at 37°C for 6 h. The compounds were added to the cells by means of an HP D300 digital dispenser in a 7-fold dilution series - either alone or as a combination of two compounds of different concentrations (ratios of substance 1 (example compound of the general formula (I) as constituent of the inventive combinations) and substance 2 (BTK inhibitor ibrutinib as constituent of the inventive combinations): 1:0; 0.85:0.15; 0.7:0.3; 0.5:0.5; 0.3:0.7; 0.15:0.85; 0:1). As control, the cells were treated with the vehicle (DMSO). After 6 h, the cells were treated with 30 µl/well One-Glo solution (Promega, E6110) and incubated at room temperature for 10 min, and the luminescence was measured using a VICTOR V (Perkin Elmer) in order to determine the NF-kB reporter activity at the end of the treatment. The percentage effect on NF-kB reporter activity and the IC50 derived therefrom were determined for each test substance using the values for a known NF-kB inhibitor (= maximum inhibition) and the DMSO control (= minimum inhibition). The IC₅₀ values were calculated using a 4-parameter fit. The combinatorial effect to test substances was determined on the basis of the above-described IC50 determination. The combination index (CI) was calculated on the basis of Chou's formula (Chou TC et al., Pharmacological Reviews September 2006). This index allows a quantitative determination of substance interactions. A Cl < 1, =1, and > 1 respectively describes a synergistic, additive and antagonistic effect. Visualization was effected by means of isobolograms.

In the combination treatment with Example Compound 3 or 11 or 12 or 13 or 19 and ibrutinib, a distinct rise in NF-kB signalling pathway inhibition was recorded compared to single treatments.

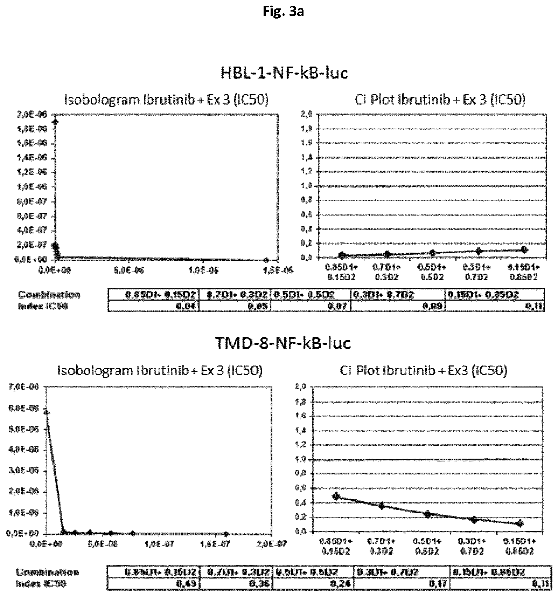

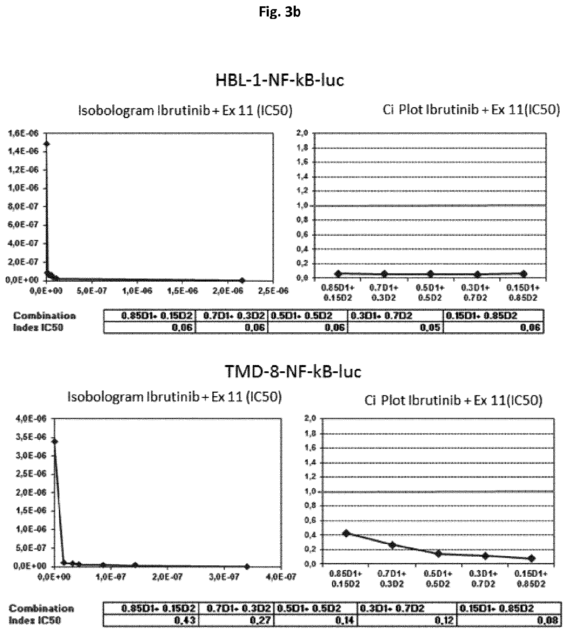

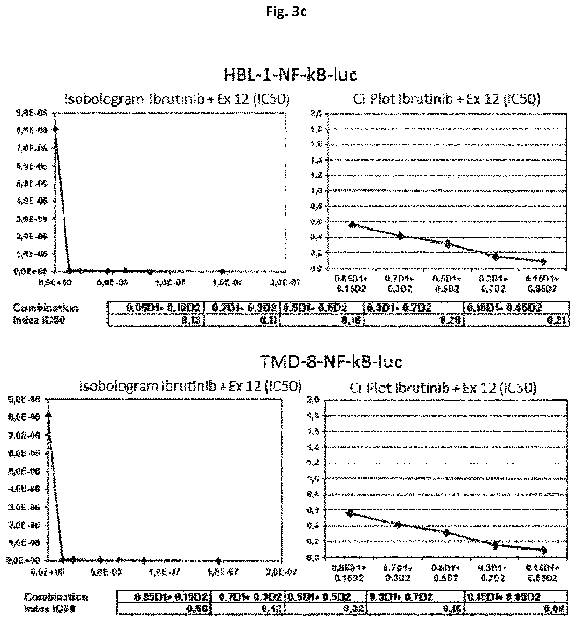

Figures 3a to 3e (Ex means example compound): Results of an NF-kB reporter assay in ABC-DLBCL cell lines TMD-8-NF-kB-luc and HBL-1-NF-kB-luc on combination of ibrutinib with the compounds of the general formula (I) (Fig. 3a: Ex 03; Fig. 3b: Ex 11; Fig. 3c: Ex 12; Fig. 3d: Ex 13, Fig. 3e: Ex 19). IC50 isobolograms for the various combinations with the respective concentrations of substance 1 (D1) (example compound of the general formula (I) as constituent of the inventive combinations) on the y axis and substance 2 (D2) (BTK inhibitor ibrutinib as constituent of the inventive combinations) on the x axis are shown. Data points below, on and above the hypotenuse respectively indicate synergistic, additive and antagonistic effects on the NF-kB signalling pathway.

Legend for Fig. 3a: Effect of the combination of ibrutinib with Example 03 on NF-kB signalling pathway activity in TMD-8 and HBL-1 cells.

Fig. 3b: Effect of the combination of ibrutinib with Example 11 on NF-kB signalling pathway activity in TMD-8 and HBL-1 cells.

Fig. 3c: Effect of the combination of ibrutinib with Example 12 on NF-kB signalling pathway activity in TMD-8 and HBL-1 cells.

Fig. 3d: Effect of the combination of ibrutinib with Example 13 on NF-kB signalling pathway activity in TMD-8 and HBL-1 cells.

Fig. 3e: Effect of the combination of ibrutinib with Example 19 on NF-kB signalling pathway activity in TMD-8 and HBL-1 cells.

### Working examples of pharmaceutical compositions

The component A compounds as constituents of the inventive combinations can be converted to pharmaceutical formulations as follows:

### Tablet:

### Composition:

100 mg of the compound of Example 11 or the compound of Example 12, 50 mg of lactose (monohydrate), 50 mg of maize starch (native), 10 mg of polyvinylpyrrolidone (PVP 25) (from BASF, Ludwigshafen, Germany) and 2 mg of magnesium stearate.

Tablet weight 212 mg. Diameter 8 mm, radius of curvature 12 mm.

### Production:

The mixture of a compound of the formula (I) as constituent of the inventive combinations, lactose and starch is granulated with a 5% solution (m/m) of the PVP in water. The granules are dried and then mixed with the magnesium stearate for 5 minutes. This mixture is compressed in a conventional tabletting press (see above for format of the tablet). The guide value used for the pressing is a pressing force of 15 kN.

### Suspension for oral administration

### Composition:

1000 mg of the compound of Example 11 or the compound of Example 12, 1000 mg of ethanol (96%), 400 mg of Rhodigel^{®} (xanthan gum from FMC, Pennsylvania, USA) and 99 g of water.

10 ml of oral suspension correspond to a single dose of 100 mg of the compound.

### Production:

The Rhodigel is suspended in ethanol; the compound is added to the suspension. The water is added while stirring. The mixture is stirred for approx. 6 h until the Rhodigel has finished swelling.

### Solution for oral administration:

### Composition

500 mg of the compound of Example 11 or the compound of Example 12, 2.5 g of polysorbate and 97 g of polyethylene glycol 400. 20 g of oral solution correspond to a single dose of 100 mg of the compound.

### Production

The compound is suspended in the mixture of polyethylene glycol and polysorbate with stirring. The stirring operation is continued until dissolution of the compound is complete. Saturated aqueous ammonium chloride solution was added, the mixture was three times extracted with ethyl acetate and the extracts were washed with sodium chloride solution and concentrated. Preparative HPLC gave 22 mg (31% of theory) of the title compound. UPLC-MS (Method A2): Rₜ = 1.15 min (UV detector: TIC), mass found 464.20. ¹H-NMR (400MHz, DMSO-d₆): δ [ppm]= 1.56 (s, 6H), 1.64 (s, 6H), 2.07 - 2.34 (m, 4H), 4.49 (t, 2H), 5.32 (s, 1H), 6.05 (s, 1H), 7.60 (s, 1H), 7.87 (dd, 1H), 7.99 - 8.05 (m, 2H), 8.35 (s, 1H), 8.79 (s, 1H), 12.45 (s, 1H).

### Assessment of physiological efficacy

### IRAK4 kinase assay

The IRAK4-inhibitory activity of the inventive substances of the formula (I) as constituents of the inventive combinations was measured in the Irak4 TR-FRET assay (TR-FRET = Time Resolved Fluorescence Resonance Energy Transfer) described hereinafter.

Recombinant fusion protein from N-terminal GST (glutathione S-transferase) and human Irak4, expressed in baculovirus-infected insect cells (Hi5, BTI-TN-5B1-4, cell line purchased from Invitrogen, catalogue No. B855-02) and purified via affinity chromatography, was used as enzyme. The substrate used for the kinase reaction was the biotinylated peptide biotin-Ahx-KKARFSRFAGSSPSQASFAEPG (C-terminus in amide form) which can be purchased, for example, from Biosyntan GmbH (Berlin-Buch).

For the assay, 11 different concentrations in the range from 20 µM to 0.073 nM were prepared from a 2 mM solution of the test substance in DMSO. 50 nl of the respective solution were pipetted into a black low-volume 384-well microtitre plate (Greiner Bio-One, Frickenhausen, Germany), 2 µl of a solution of Irak4 in assay buffer [50 mM HEPES pH 7.5, 5 mM MgCl2, 1.0 mM dithiothreitol, 30 µM activated sodium orthovanadate, 0.1% (w/v) of bovine gamma-globulin (BGG) 0.04% (v/v) nonidet-P40 (Sigma)] were added and the mixture was incubated for 15 min to allow prebinding of the substances to the enzyme prior to the kinase reaction. The kinase reaction was then started by addition of 3 µl of a solution of adenosine triphosphate (ATP, 1.67 mM = final concentration in 5 µl of assay volume: 1 mM) and peptide substrate (0.83 µM = final concentration in 5 µl assay volume: 0.5 µM) in assay buffer, and the resulting mixture was incubated at 22°C for the reaction time of 45 min. The concentration of the Irak4 was adjusted to the respective activity of the enzyme and set such that the assay was carried out in the linear range. Typical concentrations were in the order of about 0.2 nM. The reaction was stopped by addition of 5 µl of a solution of TR-FRET detection reagents [0.1 µM streptavidin-XL665 (Cisbio Bioassays; France, catalogue No. 610SAXLG)] and 1.5 nM anti-phosphoserine antibody [Merck Millipore, "STK Antibody", catalogue No. 35-002] and 0.6 nM LANCE EU-W1024-labelled anti-mouse-IgG antibody (Perkin-Elmer, product No. AD0077; alternatively, it is possible to use a terbium cryptate-labelled anti-mouse-IgG antibody from Cisbio Bioassays) in aqueous EDTA solution (100 mM EDTA, 0.4 % [w/v] bovine serum albumin [BSA] in 25 mM HEPES pH 7.5).

The resulting mixture was incubated at 22°C for 1 h to allow formation of a complex of the biotinylated phosphorylated substrate and the detection reagents. The amount of the phosphorylated substrate was then evaluated by measuring the resonance energy transfer from europium chelate-labelled anti-mouse-IgG antibody to streptavidin-XL665. To this end, the fluorescence emissions at 620 nm and 665 nm were measured after excitation at 350 nm in a TR-FRET measuring instrument, for example a Rubystar (BMG Labtechnologies, Offenburg, Germany) or a Viewlux (Perkin-Elmer). The ratio of the emissions at 665 nm and 622 nm was taken as a measure of the amount of phosphorylated substrate. The data were normalized (enzyme reaction without test substance = 0% inhibition; all other assay components but no enzyme = 100% inhibition). Typically, the test substances were tested on the same microtitre plates at 11 different concentrations in the range from 20 µM to 0.073 nM (20 µM, 5.7 µM, 1.6 µM, 0.47 µM, 0.13 µM, 38 nM, 11 nM, 3.1 nM, 0.89 nM, 0.25 nM and 0.073 nM). The dilution series were prepared prior to the assay (2 mM to 7.3 nM in 100% DMSO) by serial dilutions. The IC50 values were calculated using a 4-parameter fit.

**Table 1: IC₅₀ values of the example compounds of formula (I) as constituents of the inventive combinations in the IRAK4 kinase assay**

| Example | IC₅₀ [nM] |
|---|---|
| 1 | 30.6 |
| 2 | 135.6 |
| 3 | 7.2 |
| 4 | 52.7 |
| 5 | 264.5 |
| 6 | 35.7 |
| 7 | 867.3 |
| 8 | 15.0 |
| 9 | 103.8 |
| 10 | 18.5 |
| 11 | 3.4 |
| 12 | 10.7 |
| 13 | 1.3 |
| 14 | 10.8 |
| 15 | 12.3 |
| 16 | 21.5 |
| 17 | 36.0 |
| 18 | 47.5 |
| 19 | 8.9 |
| 20 | 13.3 |
| 21 | 117.2 |

### TNF-α secretion in THP-1 cells

With the aid of this test, it is possible to test substances for their ability to inhibit secretion of TNF-α (tumour necrosis factor alpha) in THP-1 cells (human monocytic acute leukaemia cell line). TNF-α is a cytokine involved in inflammatory processes. In this test, TNF-α secretion is triggered by incubation with bacterial lipopolysaccharide (LPS). THP-1 cells are kept in continuous suspension cell culture [RPMI 1460 medium with L-Glutamax (Gibco, Cat No. 61870-044) supplemented with foetal calf serum (FCS) 10% (Invitrogen, Cat No. 10082-147), 1% penicillin/streptomycin (Gibco BRL, Cat No. 15140-114)] and should not exceed a cell concentration of 1×10⁶ cells/ml. The assay is carried out in cell culture medium (RPMI 1460 medium with L-Glutamax supplemented with FCS 10%).

In each case 2-2.5 µl of the cell suspension (corresponds to 4000 cells) per well were dispensed into a 384-well test plate (Greiner, Cat No. 784076), in each of which 40-50 nl substance had been dissolved in 100% DMSO. Here, in each case 10 different concentrations in the range from 20 µM to 0.073 nM were used for each substance. The cells were incubated at room temperature for 15 min. 2-2.5 µl of 0.1 µg/ml LPS (Sigma, *Escherichia coli* 055:B5, Cat. No. L5418) dissolved in cell culture medium (final concentration 0.05 µg/ml) were then dispensed into each well. As a neutral control, cells were treated with 0.05 µg/ml LPS and 1% DMSO and, as inhibitor control, only once with 1% DMSO.

The plates are centrifuged at 80 g for 30 s and incubated at 37°C, 5% CO₂ and 95% atmospheric humidity for 17 h. The amount of TNF-α was determined using the TNF-alpha HTRF Detection Kit (Cisbio, Cat No. 62TNFPEB/C). To this end, in each case 2 µl of the detection solution consisting of anti-TNF-α-XL665 conjugate and anti-TNF-α-cryptate conjugate, dissolved in accordance with the manufacturer's instructions in the reconstitution buffer, were added for the HTRF (Homogeneous Time-Resolved Fluorescence) test. After the addition, the mixture was incubated either at room temperature for 3 h or at 4°C overnight. The signals were then read at 620/665 nm using an HTRF-enabled measuring instrument such as the BMG PheraStar.

The activity of the substances is expressed as the ratio between neutral and inhibitor control in per cent. The IC₅₀ values were calculated using a 4-parameter fit.

**Table 2: IC₅₀ values of the exemplary compounds with respect to the secretion of TNF-α in THP-1 cells**

| Example | IC₅₀ [µM] |
|---|---|
| 1 | 1.0 |
| 2 | 15.1 |
| 3 | 0.7 |
| 4 | 5.6 |
| 5 | 5.4 |
| 6 | 0.9 |
| 7 | 16.4 |
| 8 | 1.0 |
| 9 | 6.5 |
| 10 | 1.0 |
| 11 | 0.2 |
| 12 | 0.3 |
| 13 | 0.1 |
| 14 | 0.2 |
| 15 | 0.2 |
| 16 | 0.2 |
| 17 | 0.5 |
| 18 | 0.3 |
| 19 | 0.1 |
| 20 | 0.2 |
| 21 | 1.8 |

### In Vitro LPS (lipopolysaccharide)-induced cytokine production in human PBMCs (peripheral blood mononuclear cells)

The effect of the compounds of the general formula (I) as constituents of the inventive combinations on induced cytokine production in human PBMCs was examined. Here, cytokine production was induced by LPS, a TLR4 ligand, which leads to activation of the IRAK4-mediated signalling pathway.

The human PBMCs were obtained from anti-coagulated human whole blood. To this end, 15 ml of Ficoll-Paque (Biochrom, Cat. No. L6115) were initially charged in Leucosep tubes and 20 ml of human blood were added. After centrifugation of the blood at 800 g for 15 min at room temperature, the plasma including the platelets was removed and discarded. The PBMCs were transferred into centrifugation tubes and made up with PBS (phosphatebuffered saline) (Gibco, Cat. No. 14190). The cell suspension was centrifuged at room temperature at 250 g for 10 min and the supernatant was discarded. The PBMCs were resuspended in complete medium (RPMI 1640, without L-glutamine (PAA, Cat. No. E15-039), 10% FCS; 50 U/ml penicillin, 50 µg/ml streptomycin (PAA, Cat. No. P11-010) and 1% L-glutamine (Sigma, Cat. No. G7513)).

The assay was also carried out in complete medium. The PBMCs were sown in 96-well plates at a cell density of 2.5x10⁵ cells/well. The compounds were subjected to serial dilution in a constant volume of 100% DMSO and employed in the assay at 8 different concentrations in the range from 10 µM to 3 nM such that the final DMSO concentration was 0.4% DMSO. Prior to the actual stimulation, the cells were then pre-incubated therewith for 30 min. To induce cytokine secretion, the cells were stimulated with 0.1 µg/ml LPS (Sigma, *Escherichia coli* 0128:B12, Cat. No. L2887) for 24 hours. Cell viability was determined using the CellTiter-Glo luminescent assay (Promega, Cat. No. G7571 (G755/G756A)) in accordance with the manufacturer's instructions. The amount of secreted TNF-alpha in the cell culture supernatant was determined using the Human ProInflammatory 9-Plex Tissue Culture Kit (MSD, Cat. No. K15007B) in accordance with the manufacturer's instructions. Mentioned by way of example are Example Compound 11 and Example Compound 12 of activity ≤ 1 µM.

### In vivo β-cell lymphoma-associated xenotransplantation model

The anti-tumour activity of the compounds of the general formula (I) as constituents of the inventive combinations was examined in murine xenotransplantation models. For this purpose, female C.B-17 SCID mice were implanted subcutaneously with tumour cell lines of human B-cell lymphoma, e.g. TMD-8. At a mean tumour size of 20-30 mm², monotherapeutic treatment or treatment in combination with the standard ibrutinib treatment commenced, each of which were administered orally. This was preceded by randomization of the animals. The treatment was ended as soon as the untreated control group had tumours of area ≤ 150 mm². The tumour size and the body weight were determined weekly for three weeks. Changes in the body weight were a measure of treatment-related toxicity (> 10% = critical, stoppage in treatment until recovery, > 20% = toxic, termination). The tumour area was detected by means of an electronic caliper gauge [length (mm) x width (mm)]. The anti-tumour efficacy defined the ratio of the tumour area of treatment versus control [tumour area of the treatment group on day X/tumour area of the control group on day X]. The compound having a T/C greater than 0.5 was defined as active (effective). Statistical analysis was effected using single-factor ANOVA and comparison with the control group by means of pair-by-pair comparative analysis (Dunnett's test).

Figure 1 shows the efficacy of Example Compound 11 in monotherapy and in combination with ibrutinib in the treatment of human TMD-8 ABC-DLBCL tumours. TMD-8 tumour cells were implanted subcutaneously on day 0 into female C.B-17 SCID mice. The treatment was started on day 15 at a tumour area of about 26 mm². Example Compound 11 was administered orally at a daily dosage of 40 mg/kg. Ibrutinib was likewise administered orally at a daily dosage of 10 mg/kg. Tumour growth was assessed by determining the tumour area (see Figure 1, upper part A), and the health of the animals by determining the body weight (see Figure 1, lower part B).

Example Compound 11 did not show any inhibitory effect on the tumour growth of TMD-8 when it was administered as monotherapy. Ibrutinib in monotherapy showed a moderate inhibitory effect on the tumour growth of TMD-8, with a T/C value of 0.60, but one which was statistically significant compared to the control group. In the combination treatment comprising Example Compound 11 and ibrutinib, a significant rise in antitumour action was recorded, which is reflected in the T/C value of 0.09 and a statistically significant reduction in tumour area compared to the control and ibrutinib. The treatments were very well tolerated; no critical weight loss was recorded.

In summary, it was shown in this study that the combination of Example Compound 11 with the BTK inhibitor ibrutinib can achieve a distinct rise in the antitumour effect of the respective monotherapies in the model of an ABC-DLBCL.

### Figure 1:

Antitumour activity of Example Compound 11 in monotherapy and in combination with ibrutinib in TMD-8 C.B-17 SCID mice

| TMD-8: human ABC-DLBCL x enograft model | | | | |
|---|---|---|---|---|
| Study No. | ONC20 14.00714 | | | |
| Substance | Dosage | T/C^{a} area | Max. weight loss^{b} (%) | Toxicity |
| Vehicle | 10 ml/kg QD p.o. + 10 ml/kg QD p.o. | 1.00 | - | 0/8 |
| Example 11 | 40 mg/kg QD p.o. | 0.88 | -9 | 0/8 |
| Ibrutinib | 10 mg/kg QD p.o. | 0.60^{#} | -5 | 0/8 |
| Example 11 + Ibrutinib | 40 mg/kg QD p.o. + 10 mg/kg QD p.o. | 0.09^{∗#} | -8 | 0/8 |

| | | | | |
|---|---|---|---|---|
| ^{∗} P < 0.05 (compared to vehicle control) ^{#} P < 0.05 (compared to ibrutinib monotherapy) a) T/C = ratio of the tumour area of treatment versus control [tumour area of the treatment group on day X/tumour area of the control group on day X]. b) Loss of body weight: Changes in body weight compared to the initial body weight at the start of treatment (> 10% = critical, stoppage in treatment until recovery, > 20% = toxic, termination). | | | | |

Figure 1 shows the antitumour activity of Example Compound 11 in monotherapy and in combination with ibrutinib in TMD-8 C.B-17 SCID mice. Legend for Figure 1: The abbreviation Ex means Example, QD means once per day and po means peroral.

### Cell proliferation measurement

The antiproliferative activity of the compounds of the general formula (I) as constituents of the inventive combinations was examined in vitro in human ABC-DLBCL cells. For this purpose 4000 TMD-8 or HBL-1 cells (both from ATCC) or OCI-LY10 at 30 µl/cavity in growth medium (RPMI (Biochrom: FG 1215), 20% FCS (Biochrom: S 0615)) were transferred into a 384-cavity plate (Perkin Elmer, white) and incubated at 37°C overnight. After 24 h, cells on one plate (0 h plate) were treated with 30 µl/cavity of CTG solution (Promega Cell Titer Glo (catalogue # G755B and G756B)) and incubated at room temperature for 10 min, and luminescence was measured by means of a VICTOR V (Perkin Elmer), in order to determine cell viability on commencement of treatment. The cells on the test plate were treated with the compounds of the general formula (I) as constituents of the inventive combinations and incubated at 37°C for 72 h. The compounds were added to the cells by means of an HP D300 digital dispenser in a 7-step 3-fold dilution series - either alone or as a combination of two compounds of different concentrations (ratios of substance 1 (example compound of the general formula (I) as constituent of the inventive combinations) and substance 2 (BTK inhibitors ibrutinib or RN486 or AVL-292 or AVL-292 as constituent of the inventive combinations): 1:0; 0.85:0.15; 0.7:0.3; 0.5:0.5; 0.3:0.7; 0.15:0.85; 0:1). As control, the cells were treated with vehicle (DMSO). After 72 h, the cells were treated with 30 µl/cavity of CTG solution (Promega Cell Titer Glo (catalogue # G755B and G756B)) and incubated at room temperature for 10 min, and luminescence was measured by means of a VICTOR V (Perkin Elmer), in order to determine cell viability at the end of treatment. The percentage effect on cell growth and the IC50 derived therefrom were determined for each test substance using the values from the 0 h plate (= maximum inhibition) and the DMSO control (= minimum inhibition). The IC50 values were calculated using a 4-parameter fit. The combinatorial effect to test substances was determined on the basis of the above-described IC50 determination. The combination index (CI) was calculated on the basis of Chou's formula (Chou TC et al., Pharmacological Reviews September 2006). This index allows a quantitative determination of substance interactions. A Cl < 1, =1, and > 1 respectively describe synergistic, additive and antagonistic effects. Visualization is effected by means of isobolograms.

In the combination treatment with Example Compound 3 or 11 or 12 or 13 or 19 and ibrutinib or RN486 or AVL-292 or CGI-1746, a distinct rise in antitumour action was almost always recorded compared to single treatments.

Figures 2a to 2e and 4a to c (Ex means example compound) show the results of a combinatorial cell proliferation measurement in ABC-DLBCL cell lines TMD-8 and HBL-1 and OCI-LY10 on combination of BTK inhibitors ibrutinib or RN486 or AVL-292 or CGI-1746 with the compounds of the general formula (I) (Figure 2a: Ex 03; Figure 2b: Ex 11; Figure 2c: Ex 12; Figure 3d: Ex 13, Figure 2e: Ex 19). IC50 isobolograms for the various combinations with the respective concentrations of substance 1 (D1) (example compound of the general formula (I) as constituents of the inventive combinations) on the y axis and substance 2 (D2) (BTK inhibitors Ibrutinib or RN486 or AVL-29 or CGI-1746 as constituents of the inventive combinations) on the x axis are shown. Data points below, on and above the hypotenuse respectively indicate synergistic, additive and antagonistic effects on cell proliferation.

Legend for Fig. 2a: Effect of the combination of ibrutinib with Example 03 on cell viability of TMD-8 and HBL-1 cells.

Legend for Fig. 2b: Effect of the combination of ibrutinib with Example 11 on cell viability of TMD-8 and HBL-1 cells.

Legend for Fig. 2c: Effect of the combination of ibrutinib with Example 12 on cell viability of TMD-8 and HBL-1 cells.

Legend for Fig. 2d: Effect of the combination of ibrutinib with Example 13 on cell viability of TMD-8 and HBL-1 cells.

Legend for Fig. 2e: Effect of the combination of ibrutinib with Example 19 on cell viability of TMD-8 and HBL-1 cells.

Legend Fig. 4a: Effect of combination of BTK inhibitors RN468 or AVL-292 or CGI-1746 with example 11 on cell viability of TMD-8 cells.

Legend Fig. 4b: Effect of combination of BTK inhibitors RN468 or AVL-292 or CGI-1746 with example 11 on cell viability of HBL-1 cells.

Legend Fig. 4c: Effect of combination of BTK inhibitors RN468 or AVL-292 or CGI-1746 with example 11 on cell viability of OCI-LY10 cells.

### NF-kB reporter assay

The effect of the compounds of the general formula (I) as constituents of the inventive combinations on the NF-kB signalling pathway was examined in vitro in human DLBCL cells. 10 000 TMD-8-NF-kB-luc cells or 10 000 HBL-1-NF-kB-luc reporter cells at 30 µl/cavity in growth medium (RPMI (Biochrom: FG 1215), 20% FCS (Biochrom: S 0615)) were transferred into a 384-cavity plate (Perkin Elmer, white) and incubated at 37°C overnight. After 24 h, the cells were treated with the test substances and incubated at 37°C for 6 h. The compounds were added to the cells by means of an HP D300 digital dispenser in a 7-step 3-fold dilution series - either alone or as a combination of two compounds of different concentrations (ratios of substance 1 (example compound of the general formula (I) as constituent of the inventive combinations) and substance 2 (BTK inhibitor ibrutinib as constituent of the inventive combinations): 1:0; 0.85:0.15; 0.7:0.3; 0.5:0.5; 0.3:0.7; 0.15:0.85; 0:1). As control, the cells were treated with the vehicle (DMSO). After 6 h, the cells were treated with 30 µl/well One-Glo solution (Promega, E6110) and incubated at room temperature for 10 min, and the luminescence was measured using a VICTOR V (Perkin Elmer) in order to determine the NF-kB reporter activity at the end of the treatment. The percentage effect on NF-kB reporter activity and the IC50 derived therefrom were determined for each test substance using the values for a known NF-kB inhibitor (I-kappa B kinase inhibitor) (-)-7-[2-(Cyclopropylmethoxy)-6-hydroxyphenyl]-5-[(3S)-3-piperidinyl]-1,4-dihydro-2H-pyrido[2,3-d][1,3]-oxazin-2-on (CAS-Nummer 600734-02-9, see WO 2003076447) (= maximum inhibition) and the DMSO control (= minimum inhibition). The IC₅₀ values were calculated using a 4-parameter fit. The combinatorial effect to test substances was determined on the basis of the above-described IC50 determination. The combination index (CI) was calculated on the basis of Chou's formula (Chou TC et al., Pharmacological Reviews September 2006). This index allows a quantitative determination of substance interactions. A Cl < 1, =1, and > 1 respectively describes a synergistic, additive and antagonistic effect. Visualization was effected by means of isobolograms.

In the combination treatment with Example Compound 3 or 11 or 12 or 13 or 19 and ibrutinib, a distinct rise in NF-kB signalling pathway inhibition was recorded compared to single treatments.

Figures 3a to 3e (Ex means example compound): Results of an NF-kB reporter assay in ABC-DLBCL cell lines TMD-8-NF-kB-luc and HBL-1-NF-kB-luc on combination of ibrutinib with the compounds of the general formula (I) (Fig. 3a: Ex 03; Fig. 3b: Ex 11; Fig. 3c: Ex 12; Fig. 3d: Ex 13, Fig. 3e: Ex 19). IC50 isobolograms for the various combinations with the respective concentrations of substance 1 (D1) (example compound of the general formula (I) as constituent of the inventive combinations) on the y axis and substance 2 (D2) (BTK inhibitor ibrutinib as constituent of the inventive combinations) on the x axis are shown. Data points below, on and above the hypotenuse respectively indicate synergistic, additive and antagonistic effects on the NF-kB signalling pathway.

Legend for Fig. 3a: Effect of the combination of ibrutinib with Example 03 on NF-kB signalling pathway activity in TMD-8 and HBL-1 cells.

Fig. 3b: Effect of the combination of ibrutinib with Example 11 on NF-kB signalling pathway activity in TMD-8 and HBL-1 cells.

Fig. 3c: Effect of the combination of ibrutinib with Example 12 on NF-kB signalling pathway activity in TMD-8 and HBL-1 cells.

Fig. 3d: Effect of the combination of ibrutinib with Example 13 on NF-kB signalling pathway activity in TMD-8 and HBL-1 cells.

Fig. 3e: Effect of the combination of ibrutinib with Example 19 on NF-kB signalling pathway activity in TMD-8 and HBL-1 cells.

### Working examples of pharmaceutical compositions

The compounds of the formula (I) as constituents of the inventive combinations can be converted to pharmaceutical formulations as follows:

### Tablet:

### Composition:

100 mg of the compound of Example 11 or the compound of Example 12, 50 mg of lactose (monohydrate), 50 mg of maize starch (native), 10 mg of polyvinylpyrrolidone (PVP 25) (from BASF, Ludwigshafen, Germany) and 2 mg of magnesium stearate.

Tablet weight 212 mg. Diameter 8 mm, radius of curvature 12 mm.

### Production:

The mixture of a compound of the formula (I) as constituent of the inventive combinations, lactose and starch is granulated with a 5% solution (m/m) of the PVP in water. The granules are dried and then mixed with the magnesium stearate for 5 minutes. This mixture is compressed in a conventional tabletting press (see above for format of the tablet). The guide value used for the pressing is a pressing force of 15 kN.

### Suspension for oral administration

### Composition:

1000 mg of the compound of Example 11 or the compound of Example 12, 1000 mg of ethanol (96%), 400 mg of Rhodigel^{®} (xanthan gum from FMC, Pennsylvania, USA) and 99 g of water.

10 ml of oral suspension correspond to a single dose of 100 mg of the compound.

### Production:

The Rhodigel is suspended in ethanol; the compound is added to the suspension. The water is added while stirring. The mixture is stirred for approx. 6 h until the Rhodigel has finished swelling.

### Solution for oral administration:

### Composition

500 mg of the compound of Example 11 or the compound of Example 12, 2.5 g of polysorbate and 97 g of polyethylene glycol 400. 20 g of oral solution correspond to a single dose of 100 mg of the compound.

### Production

The compound is suspended in the mixture of polyethylene glycol and polysorbate with stirring. The stirring operation is continued until dissolution of the compound is complete.

## Claims

1. A pharmaceutical combination of:
• a component A, which is an IRAK4-inhibiting compound, which is:
1) N-[6-(2-hydroxypropan-2-yl)-2-(2-methoxyethyl)-2H-indazol-5-yl]-6-(trifluoromethyl)pyridine-2-carboxamide;
2) N-[6-(hydroxymethyl)-2-(2-methoxyethyl)-2H-indazol-5-yl]-6-(trifluoromethyl)pyridine-2-carboxamide;
3) N-[6-(2-hydroxypropan-2-yl)-2-(3-methoxypropyl)-2H-indazol-5-yl]-6-(trifluoromethyl)pyridine-2-carboxamide;
4) N-[6-(hydroxymethyl)-2-(3-methoxypropyl)-2H-indazol-5-yl]-6-(trifluoromethyl)pyridine-2-carboxamide;
5) N-[2-(2-hydroxyethyl)-6-(2-hydroxypropan-2-yl)-2H-indazol-5-yl]-6-(trifluoromethyl)pyridine-2-carboxamide;
6) N-[6-(2-hydroxypropan-2-yl)-2-(3-hydroxypropyl)-2H-indazol-5-yl]-6-(trifluoromethyl)pyridine-2-carboxamide;
7) N-[2-(2-hydroxyethyl)-6-(hydroxymethyl)-2H-indazol-5-yl]-6-(trifluoromethyl)pyridine-2-carboxamide;
8) N-[6-(2-hydroxypropan-2-yl)-2-(oxetan-3-ylmethyl)-2H-indazol-5-yl]-6-(trifluoromethyl)pyridine-2-carboxamide;
9) N-[6-(hydroxymethyl)-2-(oxetan-3-ylmethyl)-2H-indazol-5-yl]-6-(trifluoromethyl)pyridine-2-carboxamide;
10) N-{6-(2-hydroxypropan-2-yl)-2-[3-(methylsulphonyl)propyl]-2H-indazol-5-yl}-6-(trifluoromethyl)pyridine-2-carboxamide;
11) N-[2-(3-hydroxy-3-methylbutyl)-6-(2-hydroxypropan-2-yl)-2H-indazol-5-yl]-6-(trifluoromethyl)pyridine-2-carboxamide;
12) N-{6-(2-hydroxypropan-2-yl)-2-[2-(methylsulphonyl)ethyl]-2H-indazol-5-yl}-6-(trifluoromethyl)pyridine-2-carboxamide;
13) 6-(difluoromethyl)-N-[2-(3-hydroxy-3-methylbutyl)-6-(2-hydroxypropan-2-yl)-2H-indazol-5-yl]pyridine-2-carboxa mide;
14) 6-(difluoromethyl)-N-{6-(2-hydroxypropan-2-yl)-2-[2-(methylsulphonyl)ethyl]-2H-indazol-5-yl}pyridine-2-carboxamide;
15) 6-(difluoromethyl)-N-[6-(2-hydroxypropan-2-yl)-2-(3-hydroxypropyl)-2H-indazol-5-yl]pyridine-2-carboxa mide;
16) N-[6-(2-hydroxypropan-2-yl)-2-(4,4,4-trifluorobutyl)-2H-indazol-5-yl]-6-(trifluoromethyl)pyridine-2-carboxamide;
17) N-{6-(2-hydroxypropan-2-yl)-2-[3-(trifluoromethoxy)propyl]-2H-indazol-5-yl}-6-(trifluoromethyl)pyridine-2-carboxamide;
18) N-{6-(2-hydroxypropan-2-yl)-2-[3-(2,2,2-trifluoroethoxy)propyl]-2H-indazol-5-yl}-6-(trifluoromethyl)pyrid ine-2-carboxa mide;
19) 5-fluoro-N-[2-(3-hydroxy-3-methylbutyl)-6-(2-hydroxypropan-2-yl)-2H-indazol-5-yl]-6-methylpyridine-2-carboxa mide;
20) N[2-(3-hydroxy-3-methylbutyl)-6-(2-hydroxypropan-2-yl)-2H-indazol-5-yl]-6-methylpyridine-2-carboxamide ; or
21) 6-(2-hydroxypropan-2-yl)-N-[6-(2-hydroxypropan-2-yl)-2-(4,4,4-trifluorobutyl)-2H-indazol-5-yl]pyridine-2-carboxamide;
or a salt, a solvate, or a solvate of said salt, thereof;
and
• a component B, which is a BTK-inhibiting compound selected from the following list:
o ibrutinib, or a pharmaceutically acceptable salt thereof;
○ 4-tert-butyl-N-[2-methyl-3-(4-methyl-6-{[4-(morpholin-4-ylcarbonyl)phenyl]amino}-5-oxo-4,5-dihydropyrazin-2-yl)phenyl]benzamide (CGI-1746, CAS 910232-84-7) ;
o N-{3-[(5-fluoro-2-{[4-(2-methoxyethoxy)phenyl]amino}pyrimidin-4-yl)amino]phenyl}acrylamide (AVL-292, CAS 1202757-89-8); or
○ 6-cyclopropyl-8-fluoro-2-[2-(hydroxymethyl)-3-(1-methyl-5-{[5-(4-methylpiperazin-1-yl)pyridin-2-yl]amino}-6-oxo-1,6-dihydropyridin-3-yl)phenyl]isoquinolin-1(2H)-one (RN486, CAS 1242156-23-5)]; for use in the treatment and/or prophylaxis of non-Hodgkin's lymphoma (abbreviated to "NHL"), especially primary therapy or secondary therapy of recurrent or refractory, indolent or aggressive non-Hodgkin's lymphoma (NHL), especially of follicular lymphoma (abbreviated to "FL"), of chronic lymphatic leukaemia (abbreviated to "CLL"), of marginalzone lymphoma (abbreviated to "MZL"), of diffuse large-cell B-cell lymphoma (abbreviated to "DLBCL"), especially of activated B-cell-like diffuse large-cell B-cell lymphoma (abbreviated to "ABC-DLBCL"), of mantle cell lymphoma (abbreviated to "MCL"), of transformed lymphoma (abbreviated to "TL"), of peripheral T-cell lymphoma (abbreviated to "PTCL") or of lymphoplasmacytic lymphoma (Waldenström's macroglobulinaemia (abbreviated to "WM")).

2. The pharmaceutical combination for use Z according to claim 1, in Z which
• component B, which is a BTK-inhibiting compound, is
○ ibrutinib, or a pharmaceutically acceptable salt thereof.

3. The combination Z for use Z according to claim 1 or 2, which further comprises a component C,
which is a pharmaceutical agent selected from the following list:
131l-chTNT, abarelix, abiraterone, aclarubicin, ado-trastuzumab emtansine, afatinib, aflibercept, aldesleukin, alemtuzumab, alendronic acid, alitretinoin, altretamine, amifostine, aminoglutethimide, hexyl-5-aminolevulinate, amrubicin, amsacrine, anastrozole, ancestim, anethole dithiolethione, angiotensin II, antithrombin III, aprepitant, arcitumomab, arglabin, arsenic trioxide, asparaginase, axitinib, azacitidine, belotecan, bendamustine, belinostat, bevacizumab, bexarotene, bicalutamide, bisantrene, bleomycin, bortezomib, buserelin, bosutinib, brentuximab vedotin, busulfan, cabazitaxel, cabozantinib, calcium folinate, calcium levofolinate, capecitabine, capromab, carboplatin, carfilzomib, carmofur, carmustine, catumaxomab, celecoxib, celmoleukin, ceritinib, cetuximab, chlorambucil, chlormadinone, chlormethine, cidofovir, cinacalcet, cisplatin, cladribine, clodronic acid, clofarabine, copanlisib, crisantaspase, crizotinib, cyclophosphamide, cyproterone, cytarabine, dacarbazine, dactinomycin, dabrafenib, dasatinib, daunorubicin, decitabine, degarelix, denileukin-diftitox, denosumab, depreotide, deslorelin, dexrazoxane, dibrospidium chloride, dianhydrogalactitol, diclofenac, docetaxel, dolasetron, doxifluridine, doxorubicin, doxorubicin + estrone, dronabinol, edrecolomab, elliptinium acetate, endostatin, enocitabine, enzalutamide, epirubicin, epitiostanol, epoetin-alfa, epoetin-beta, epoetin-zeta, eptaplatin, eribulin, erlotinib, esomeprazole, estramustine, etoposide, everolimus, exemestane, fadrozole, fentanyl, fluoxymesterone, floxuridine, fludarabine, fluorouracil, flutamide, folic acid, formestane, fosaprepitant, fotemustine, fulvestrant, gadobutrol, gadoteridol, gadoteric acid meglumine salt, gadoversetamide, gadoxetic acid disodium salt (Gd-EOB-DTPA disodium salt), gallium nitrate, ganirelix, gefitinib, gemcitabine, gemtuzumab, glucarpidase, glutoxim, goserelin, granisetron, granulocyte colony stimulating factor (G-CSF), granulocyte macrophage colony stimulating factor (GM-CSF), histamine dihydrochloride, histrelin, hydroxycarbamide, I-125 seeds, ibandronic acid, ibritumomab-tiuxetan, idarubicin, ifosfamide, imatinib, imiquimod, improsulfan, indisetron, incadronic acid, ingenol mebutate, interferon-alfa, interferon-beta, interferon-gamma, iobitridol, iobenguane I-123, iomeprol, ipilimumab, irinotecan, itraconazole, ixabepilone, lanreotide, lansoprazole, lapatinib, lasocholine, lenalidomide, lentinan, letrozole, leuprorelin, levamisole, levonorgestrel, levothyroxin-sodium, lipegfilgrastim, lisuride, lobaplatin, lomustine, lonidamine, masoprocol, medroxyprogesteron, megestrol, melarsoprol, melphalan, mepitiostan, mercaptopurine, mesna, methadone, methotrexate, methoxsalen, methylaminolevulinate, methylprednisolone, methyltestosterone, metirosine, mifamurtide, miltefosine, miriplatin, mitobronitol, mitoguazone, mitolactol, mitomycin, mitotan, mitoxantrone, mogamulizumab, molgramostim, mopidamol, morphine hydrochloride, morphine sulfate, nabilone, nabiximols, nafarelin, naloxone + pentazocine, naltrexone, nartograstim, nedaplatin, nelarabine, neridronic acid, nivolumab pentetreotide, nilotinib, nilutamide, nimorazole, nimotuzumab, nimustine, nitracrine, nivolumab, obinutuzumab, octreotide, ofatumumab, omacetaxin mepesuccinate, omeprazole, ondansetron, orgotein, orilotimod, oxaliplatin, oxycodone, oxymetholone, ozogamicin, p53 gene therapy, paclitaxel, palladium-103 seed, palonosetron, pamidronic acid, panitumumab, pantoprazole, pazopanib, pegaspargase, pembrolizumab, Peg-interferon alfa-2b, pemetrexed, pentostatin, peplomycin, perflubutane, perfosfamide, pertuzumab, picibanil, pilocarpine, pirarubicin, pixantron, plerixafor, plicamycin, poliglusam, polyoestradiol phosphate, polyvinylpyrrolidone + sodium hyaluronate, polysaccharide-K, pomalidomide, ponatinib, porfimer-sodium, pralatrexate, prednimustine, prednisone, procarbazine, procodazole, propranolol, quinagolide, rabeprazole, racotumomab, radium-223 chloride, radotinib, raloxifene, raltitrexed, ramosetron, ramucirumab, ranimustine, rasburicase, razoxan, refametinib, regorafenib, risedronic acid, rhenium-186 etidronate, rituximab, romidepsin, romurtid, roniciclib, samarium-153 lexidronam, satumomab, secretin, sipuleucel-T, sizofiran, sobuzoxane, sodium glycididazole, sorafenib, stanozolol, streptozocin, sunitinib, talaporfin, tamibarotene, tamoxifen, tapentadol, tasonermin, teceleukin, technetium-99m nofetumomab merpentan, 99mTc-HYNIC-[Tyr3]-octreotide, tegafur, tegafur + gimeracil + oteracil, temoporfin, temozolomide, temsirolimus, teniposide, testosterone, tetrofosmin, thalidomide, thiotepa, thymalfasin, thyrotropin alfa, tioguanine, tocilizumab, topotecan, toremifene, tositumomab, trabectedin, tramadol, trastuzumab, treosulfan, tretinoin, trifluridine + tipiracil, trametinib, trilostane, triptorelin, trofosfamide, thrombopoietin, ubenimex, valrubicin, vandetanib, vapreotide, vatalanib, vemurafenib, vinblastine, vincristine, vindesine, vinflunine, vinorelbine, vismodegib, vorinostat, yttrium-90 glass microbeads, zinostatin, zinostatin stimalamer, zoledronic acid, zorubicin.

4. A kit consisting of a combination according to any one of claims 1 to 3.

## Patentansprüche

1. Pharmazeutische Kombination von
• einer Komponente A, bei der es sich um eine IRAK4-inhibierende Verbindung handelt, nämlich:
1) N-[6-(2-Hydroxypropan-2-yl)-2-(2-methoxyethyl)-2H-indazol-5-yl]-6-(trifluormethyl)pyridin-2-carboxamid;
2) N-[6-(Hydroxymethyl)-2-(2-methoxyethyl)-2H-indazol-5-yl]-6-(trifluormethyl)pyridin-2-carboxamid;
3) N-[6-(2-Hydroxypropan-2-yl)-2-(3-methoxypropyl)-2H-indazol-5-yl]-6-(trifluormethyl)pyridin-2-carboxamid;
4) N-[6-(Hydroxymethyl)-2-(3-methoxypropyl)-2H-indazol-5-yl]-6-(trifluormethyl)pyridin-2-carboxamid;
5) N-[2-(2-Hydroxyethyl)-6-(2-hydroxypropan-2-yl)-2H-indazol-5-yl]-6-(trifluormethyl)pyridin-2-carboxamid;
6) N-[6-(2-Hydroxypropan-2-yl)-2-(3-hydroxypropyl)-2H-indazol-5-yl]-6-(trifluormethyl)pyridin-2-carboxamid;
7) N-[2-(2-Hydroxyethyl)-6-(hydroxymethyl)-2H-indazol-5-yl]-6-(trifluormethyl)pyridin-2-carboxamid;
8) N-[6-(2-Hydroxypropan-2-yl)-2-(oxetan-3-ylmethyl)-2H-indazol-5-yl]-6-(trifluormethyl)pyridin-2-carboxamid;
9) N-[6-(Hydroxymethyl)-2-(oxetan-3-ylmethyl)-2H-indazol-5-yl]-6-(trifluormethyl)pyridin-2-carboxamid;
10) N-{6-(2-Hydroxypropan-2-yl)-2-[3-(methylsulfonyl)propyl]-2H-indazol-5-yl}-6-(trifluormethyl)pyridin-2-carboxamid;
11) N-[2-(3-Hydroxy-3-methylbutyl)-6-(2-hydroxypropan-2-yl)-2H-indazol-5-yl]-6-(trifluormethyl)pyridin-2-carboxamid;
12) N-{6-(2-Hydroxypropan-2-yl)-2-[2-(methylsulfonyl)ethyl]-2H-indazol-5-yl}-6-(trifluormethyl)pyridin-2-carboxamid;
13) 6-(Difluormethyl)-N-[2-(3-hydroxy-3-methylbutyl)-6-(2-hydroxypropan-2-yl)-2H-indazol-5-yl]pyridin-2-carboxamid;
14) 6-(Difluormethyl)-N-{6-(2-hydroxypropan-2-yl)-2-[2-(methylsulfonyl)ethyl]-2H-indazol-5-yl}pyridin-2-carboxamid;
15) 6-(Difluormethyl)-N-[6-(2-hydroxypropan-2-yl)-2-(3-hydroxypropyl)-2H-indazol-5-yl]pyridin-2-carboxamid;
16) N-[6-(2-Hydroxypropan-2-yl)-2-(4,4,4-trifluorbutyl)-2H-indazol-5-yl]-6-(trifluormethyl)pyridin-2-carboxamid;
17) N-{6-(2-Hydroxypropan-2-yl)-2-[3-(trifluormethoxy)propyl]-2H-indazol-5-yl}-6-(trifluormethyl)pyridin-2-carboxamid;
18) N-{6-(2-Hydroxypropan-2-yl)-2-[3-(2,2,2-trifluorethoxy)propyl]-2H-indazol-5-yl}-6-(trifluormethyl)pyridin-2-carboxamid;
19) 5-Fluor-N-[2-(3-hydroxy-3-methylbutyl)-6-(2-hydroxypropan-2-yl)-2H-indazol-5-yl]-6-methylpyridin-2-carboxamid;
20) N-[2-(3-Hydroxy-3-methylbutyl)-6-(2-hydroxypropan-2-yl)-2H-indazol-5-yl]-6-methylpyridin-2-carboxamid; oder
21) 6-(2-Hydroxypropan-2-yl)-N-[6-(2-hydroxypropan-2-yl)-2-(4,4,4-trifluorbutyl)-2H-indazol-5-yl]pyridin-2-carboxamid;
oder ein Salz, ein Solvat oder ein Solvat des Salzes davon;
und
• einer Komponente B, bei der es sich um eine BTK-inhibierende Verbindung aus der folgenden Liste handelt:
o Ibrutinib, oder ein pharmazeutisch verträgliches Salz davon;
∘ 4-tert-Butyl-N-[2-methyl-3-(4-methyl-6-{[4-(morpholin-4-ylcarbonyl)phenyl]amino}-5-oxo-4,5-dihydropyrazin-2-yl)phenyl]benzamid (CGI-1746, CAS 910232-84-7);
o N-{3-[(5-Fluor-2-{[4-(2-methoxyethoxy)phenyl]amino}pyrimidin-4-yl)amino]phenyl}acrylamid (AVL-292, CAS 1202757-89-8); oder
∘ 6-Cyclopropyl-8-fluor-2-[2-(hydroxymethyl)-3-(1-methyl-5-{[5-(4-methylpiperazin-l-yl)pyridin-2-yl]amino}-6-oxo-1,6-dihydropyridin-3-yl)phenyl]isochinolin-1(2H)-on (RN486, CAS 1242156-23-5)];
zur Verwendung bei der Behandlung und/oder Prophylaxe von Non-Hodgkin-Lymphomen (abgekürzt als "NHL"), insbesondere Erstlinientherapie oder Zweitlinientherapie rezidiver oder refraktärer indolenter oder aggressiver Non-Hodgkin-Lymphome (NHL), insbesondere des Follikulären Lymphoms (abgekürzt als "FL"), der chronischen lymphatischen Leukämie (abgekürzt als "CLL"), des Marginalzonen-Lymphoms (abgekürzt als "MZL"), des diffusen großzelligen B-Zell-Lymphoms (abgekürzt als "DLBCL"), insbesondere des aktivierten B-Zellen-ähnlichen diffusen großzelligen B-Zell-Lymphoms (abgekürzt als "ABC-DLBCL"), des Mantelzelllymphoms (abgekürzt als "MCL"), des transformierten Lymphoms (abgekürzt als "TL"), peripherer T-Zell-Lymphome (abgekürzt als "PTCL") oder der lymphoplasmazytischen Lymphome (Waldenströms Makroglobulinämie (abgekürzt als "WM")).

2. Pharmazeutische Kombination zur Verwendung nach Anspruch 1, in der es sich bei Komponente B, bei der es sich um eine BTK-inhibierende Verbindung handelt, um
o Ibrutinib, oder ein pharmazeutisch verträgliches Salz davon
handelt.

3. Kombination zur Verwendung nach Anspruch 1 oder 2, die ferner eine Komponente C umfasst, bei der es sich um ein pharmazeutisches Mittel aus der folgenden Liste handelt:
131I-chTNT, Abarelix, Abirateron, Aclarubicin, Ado-Trastuzumab-Emtansin, Afatinib, Aflibercept, Aldesleukin, Alemtuzumab, Alendronsäure, Alitretinoin, Altretamin, Amifostin, Aminoglutethimid, Hexyl-5-aminolevulinat, Amrubicin, Amsacrin, Anastrozol, Ancestim, Anetholdithiolethion, Angiotensin II, Antithrombin III, Aprepitant, Arcitumomab, Arglabin, Arsentrioxid, Asparaginase, Axitinib, Azacitidin, Belotecan, Bendamustin, Belinostat, Bevacizumab, Bexaroten, Bicalutamid, Bisantren, Bleomycin, Bortezomib, Buserelin, Bosutinib, Brentuximabvedotin, Busulfan, Cabazitaxel, Cabozantinib, Calciumfolinat, Calciumlevofolinat, Capecitabin, Capromab, Carboplatin, Carfilzomib, Carmofur, Carmustin, Catumaxomab, Celecoxib, Celmoleukin, Ceritinib, Cetuximab, Chlorambucil, Chlormadinon, Chlormethin, Cidofovir, Cinacalcet, Cisplatin, Cladribin, Clodronsäure, Clofarabin, Copanlisib, Crisantaspase, Crizotinib, Cyclophosphamid, Cyproteron, Cytarabin, Dacarbazin, Dactinomycin, Dabrafenib, Dasatinib, Daunorubicin, Decitabin, Degarelix, Denileukin-Diftitox, Denosumab, Depreotid, Deslorelin, Dexrazoxan, Dibrospidiumchlorid, Dianhydrogalactitol, Diclofenac, Docetaxel, Dolasetron, Doxifluridin, Doxorubicin, Doxorubicin + Estron, Dronabinol, Edrecolomab, Elliptiniumacetat, Endostatin, Enocitabin, Enzalutamid, Epirubicin, Epitiostanol, Epoetin-alfa, Epoetin-beta, Epoetin-zeta, Eptaplatin, Eribulin, Erlotinib, Esomeprazol, Estramustin, Etoposid, Everolimus, Exemestan, Fadrozol, Fentanyl, Fluoxymesteron, Floxuridin, Fludarabin, Fluoruracil, Flutamid, Folinsäure, Formestan, Fosaprepitant, Fotemustin, Fulvestrant, Gadobutrol, Gadoteridol, Gadotersäure-Megluminsalz, Gadoversetamid, Gadoxetsäure-Dinatriumsalz (Gd-EOB-DTPA-Dinatriumsalz), Galliumnitrat, Ganirelix, Gefitinib, Gemcitabin, Gemtuzumab, Glucarpidase, Glutoxim, Goserelin, Granisetron, Granulocyte Colony Stimulating Factor (G-CSF), Granulocyte Macrophage Colony Stimulating Factor (GM-CSF), Histamindihydrochlorid, Histrelin, Hydroxycarbamid, I-125-Seeds, Ibandronsäure, Ibritumomab-Tiuxetan, Idarubicin, Ifosfamid, Imatinib, Imiquimod, Improsulfan, Indisetron, Incadronsäure, Ingenolmebutat, Interferon-alfa, Interferon-beta, Interferongamma, Iobitridol, Iobenguan I-123, Iomeprol, Ipilimumab, Irinotecan, Itraconazol, Ixabepilon, Lanreotid, Lansoprazol, Lapatinib, Lasocholin, Lenalidomid, Lentinan, Letrozol, Leuprorelin, Levamisol, Levonorgestrel, Levothyroxin-Natrium, Lipegfilgrastim, Lisurid, Lobaplatin, Lomustin, Lonidamin, Masoprocol, Medroxyprogesteron, Megestrol, Melarsoprol, Melphalan, Mepitiostan, Mercaptopurin, Mesna, Methadon, Methotrexat, Methoxsalen, Methylaminolevulinat, Methylprednisolon, Methyltestosteron, Metirosin, Mifamurtid, Miltefosin, Miriplatin, Mitobronitol, Mitoguazon, Mitolactol, Mitomycin, Mitotan, Mitoxantron, Mogamulizumab, Molgramostim, Mopidamol, Morphinhydrochlorid, Morphinsulfat, Nabilon, Nabiximols, Nafarelin, Naloxon + Pentazocin, Naltrexon, Nartograstim, Nedaplatin, Nelarabin, Neridronsäure, Nivolumabpentetreotid, Nilotinib, Nilutamid, Nimorazol, Nimotuzumab, Nimustin, Nitracrin, Nivolumab, Obinutuzumab, Octreotid, Ofatumumab, Omacetaxin-Mepesuccinat, Omeprazol, Ondansetron, Orgotein, Orilotimod, Oxaliplatin, Oxycodon, Oxymetholon, Ozogamicin, p53-Gentherapie, Paclitaxel, Palladium-103-Seed, Palonosetron, Pamidronsäure, Panitumumab, Pantoprazol, Pazopanib, Pegaspargase, Pembrolizumab, Peg-interferon-alfa-2b, Pemetrexed, Pentostatin, Peplomycin, Perflubutan, Perfosfamid, Pertuzumab, Picibanil, Pilocarpin, Pirarubicin, Pixantron, Plerixafor, Plicamycin, Poliglusam, Polyestradiolphosphat, Polyvinylpyrrolidon + Natriumhyaluronat, Polysaccharid-K, Pomalidomid, Ponatinib, Porfimer-Natrium, Pralatrexat, Prednimustin, Prednison, Procarbazin, Procodazol, Propranolol, Quinagolid, Rabeprazol, Racotumomab, Radium-223-chlorid, Radotinib, Raloxifen, Raltitrexed, Ramosetron, Ramucirumab, Ranimustin, Rasburicase, Razoxan, Refametinib, Regorafenib, Risedronsäure, Rhenium-186-etidronat, Rituximab, Romidepsin, Romurtid, Roniciclib, Samarium(153)-lexidronam, Satumomab, Secretin, Sipuleucel-T, Sizofiran, Sobuzoxan, Natriumglycididazol, Sorafenib, Stanozolol, Streptozocin, Sunitinib, Talaporfin, Tamibaroten, Tamoxifen, Tapentadol, Tasonermin, Teceleukin, Technetium(99m)-Nofetumomab-Merpentan, 99mTc-HYNIC-[Tyr3]-octreotid, Tegafur, Tegafur + Gimeracil + Oteracil, Temoporfin, Temozolomid, Temsirolimus, Teniposid, Testosteron, Tetrofosmin, Thalidomid, Thiotepa, Thymalfasin, Thyrotropin alfa, Tioguanin, Tocilizumab, Topotecan, Toremifen, Tositumomab, Trabectedin, Tramadol, Trastuzumab, Treosulfan, Tretinoin, Trifluridin + Tipiracil, Trametinib, Trilostan, Triptorelin, Trofosfamid, Thrombopoietin, Ubenimex, Valrubicin, Vandetanib, Vapreotid, Vatalanib, Vemurafenib, Vinblastin, Vincristin, Vindesin, Vinflunin, Vinorelbin, Vismodegib, Vorinostat, Yttrium-90-Glasmikrokugeln, Zinostatin, Zinostatin-Stimalamer, Zoledronsäure, Zorubicin.

4. Kit, bestehend aus einer Kombination nach einem der Ansprüche 1 bis 3.

## Revendications

1. Combinaison pharmaceutique de :
• un composant A, qui est un composé inhibant IRAK4, qui est :
1) N-[6-(2-hydroxypropan-2-yl)-2-(2-méthoxyéthyl)-2H-indazol-5-yl]-6-(trifluorométhyl)pyridine-2-carboxamide ;
2) N-[6-(hydroxyméthyl)-2-(2-méthoxyéthyl)-2H-indazol-5-yl]-6-(trifluorométhyl)pyridine-2-carboxamide ;
3) N-[6-(2-hydroxypropan-2-yl)-2-(3-méthoxypropyl)-2H-indazol-5-yl]-6-(trifluorométhyl)pyridine-2-carboxamide ;
4) N-[6-(hydroxyméthyl)-2-(3-méthoxypropyl)-2H-indazol-5-yl]-6-(trifluorométhyl)pyridine-2-carboxamide ;
5) N-[2-(2-hydroxyéthyl)-6-(2-hydroxypropan-2-yl)-2H-indazol-5-yl]-6-(trifluorométhyl)pyridine-2-carboxamide ;
6) N-[6-(2-hydroxypropan-2-yl)-2-(3-hydroxypropyl)-2H-indazol-5-yl]-6-(trifluorométhyl)pyridine-2-carboxamide ;
7) N-[2-(2-hydroxyéthyl)-6-(hydroxyméthyl)-2H-indazol-5-yl]-6-(trifluorométhyl)pyridine-2-carboxamide ;
8) N-[6-(2-hydroxypropan-2-yl)-2-(oxétan-3-ylméthyl)-2H-indazol-5-yl]-6-(trifluorométhyl)pyridine-2-carboxamide ;
9) N-[6-(hydroxyméthyl)-2-(oxétan-3-ylméthyl)-2H-indazol-5-yl]-6-(trifluorométhyl)pyridine-2-carboxamide ;
10) N-{6-(2-hydroxypropan-2-yl)-2-[3-(méthylsulfonyl)propyl]-2H-indazol-5-yl}-6-(trifluorométhyl)pyridine-2-carboxamide ;
11) N-[2-(3-hydroxy-3-méthylbutyl)-6-(2-hydroxypropan-2-yl)-2H-indazol-5-yl]-6-(trifluorométhyl)pyridine-2-carboxamide ;
12) N-{6-(2-hydroxypropan-2-yl)-2-[2-(méthylsulfonyl)éthyl]-2H-indazol-5-yl}-6-(trifluorométhyl)pyridine-2-carboxamide ;
13) 6-(difluorométhyl)-N-[2-(3-hydroxy-3-méthylbutyl)-6-(2-hydroxypropan-2-yl)-2H-indazol-5-yl]pyridine-2-carboxamide ;
14) 6-(difluorométhyl)-N-{6-(2-hydroxypropan-2-yl)-2-[2-(méthylsulfonyl)éthyl]-2H-indazol-5-yl}pyridine-2-carboxamide ;
15) 6-(difluorométhyl)-N-[6-(2-hydroxypropan-2-yl)-2-(3-hydroxypropyl)-2H-indazol-5-yl]pyridine-2-carboxamide ;
16) N-[6-(2-hydroxypropan-2-yl)-2-(4,4,4-trifluorobutyl)-2H-indazol-5-yl]-6-(trifluorométhyl)pyridine-2-carboxamide ;
17) N-{6-(2-hydroxypropan-2-yl)-2-[3-(trifluorométhoxy)propyl]-2H-indazol-5-yl}-6-(trifluorométhyl)pyridine-2-carboxamide ;
18) N-{6-(2-hydroxypropan-2-yl)-2-[3-(2,2,2-trifluoroéthoxy)propyl]-2H-indazol-5-yl}-6-(trifluorométhyl)pyridine-2-carboxamide ;
19) 5-fluoro-N-[2-(3-hydroxy-3-méthylbutyl)-6-(2-hydroxypropan-2-yl)-2H-indazol-5-yl]-6-méthylpyridine-2-carboxamide ;
20) N-[2-(3-hydroxy-3-méthylbutyl)-6-(2-hydroxypropan-2-yl)-2H-indazol-5-yl]-6-méthylpyridine-2-carboxamide ; ou
21) 6-(2-hydroxypropan-2-yl)-N-[6-(2-hydroxypropan-2-yl)-2-(4,4,4-trifluorobutyl)-2H-indazol-5-yl]pyridine-2-carboxamide ;
ou un sel, un solvate, ou un solvate dudit sel, correspondant ;
et
• un composant B, qui est un composé inhibant BTK choisi parmi la liste suivante :
o ibrutinib ou un sel pharmaceutiquement acceptable correspondant ;
∘ 4-tert-butyl-N-[2-méthyl-3-(4-méthyl-6-{[4-(morpholin-4-ylcarbonyl)phényl]amino}-5-oxo-4,5-dihydropyrazin-2-yl)phényl]benzamide (CGI-1746, CAS 910232-84-7) ;
o N-{3-[(5-fluoro-2-{[4-(2-méthoxyéthoxy)phényl]amino}pyrimidin-4-yl)amino]phényl}acrylamide (AVL-292, CAS 1202757-89-8) ; ou
∘ 6-cyclopropyl-8-fluoro-2-[2-(hydroxyméthyl)-3-(1-méthyl-5-{[5-(4-méthylpipérazin-l-yl)pyridin-2-yl]amino}-6-oxo-1,6-dihydropyridin-3-yl)phényl]isoquinoléin-1(2H)-one (RN486, CAS 1242156-23-5)] ;
pour une utilisation dans le traitement et/ou la prophylaxie d'un lymphome non hodgkinien (abrégé « NHL »), notamment en thérapie primaire ou thérapie secondaire d'un lymphome non hodgkinien (NHL) récurrent ou réfractaire, indolent ou agressif, notamment d'un lymphome folliculaire (abrégé « FL »), d'une leucémie lymphatique chronique (abrégée « CLL »), d'un lymphome de la zone marginale (abrégé « MZL »), d'un lymphome diffus à grande cellules B (abrégé « DLBCL »), notamment d'un lymphome diffus à grandes cellules B de type cellules B activées (abrégé « ABC-DLBCL »), d'un lymphome à cellules du manteau (abrégé « MCL »), d'un lymphome transformé (abrégé « TL »), d'un lymphome à cellules T périphérique (abrégé « PTCL ») ou d'un lymphome lymphoplasmocytaire (macroglobulinémie de Waldenström (abrégée « WM »)).

2. Combinaison pharmaceutique pour une utilisation selon la revendication 1, dans laquelle
• le composant B, qui est un composé inhibant BTK, est
∘ l'ibrutinib ou un sel pharmaceutiquement acceptable correspondant.

3. Combinaison pour une utilisation selon la revendication 1 ou 2, qui comprend en outre un composant C, qui est un agent pharmaceutique choisi dans la liste suivante : 131I-chTNT, abarelix, abiratérone, aclarubicine, ado-trastuzumab emtansine, afatinib, aflibercept, aldesleukine, alemtuzumab, acide alendronique, alitrétinoïne, altrétamine, amifostine, aminoglutéthimide, 5-aminolévulinate d'hexyle, amrubicine, amsacrine, anastrozole, ancestim, anéthole dithioléthione, angiotensine II, antithrombine III, aprépitant, arcitumomab, arglabine, trioxyde d'arsenic, asparaginase, axitinib, azacitidine, bélotécan, bendamustine, belinostat, bévacizumab, bexarotène, bicalutamide, bisantrène, bléomycine, bortézomib, buséréline, bosutinib, brentuximab védotine, busulfan, cabazitaxel, cabozantinib, folinate de calcium, lévofolinate de calcium, capécitabine, capromab, carboplatine, carfilzomib, carmofur, carmustine, catumaxomab, célécoxib, célmoleukine, céritinib, cétuximab, chlorambucil, chlormadinone, chlorméthine, cidofovir, cinacalcet, cisplatine, cladribine, acide clodronique, clofarabine, copanlisib, crisantaspase, crizotinib, cyclophosphamide, cyprotérone, cytarabine, dacarbazine, dactinomycine, dabrafénib, dasatinib, daunorubicine, décitabine, dégarélix, dénileukine-diftitox, dénosumab, dépréotide, desloréline, dexrazoxane, dibrospidium chlorure, dianhydrogalactitol, diclofénac, docétaxel, dolasétron, doxifluridine, doxorubicine, doxorubicine + œstrone, dronabinol, édrécolomab, elliptinium acétate, endostatine, enocitabine, enzalutamide, épirubicine, épitiostanol, époétine-alfa, époétine-beta, epoetin-zeta, eptaplatine, eribuline, erlotinib, ésoméprazole, estramustine, étoposide, évérolimus, éxémestane, fadrozole, fentanyl, fluoxymestérone, floxuridine, fludarabine, fluorouracil, flutamide, acide folique, formestane, fosaprépitant, fotémustine, fulvestrant, gadobutrol, gadotéridol, acide gadotérique sel avec la méglumine, gadoversétamide, acide gadoxétique sel disodique (Gd-EOB-DTPA sel disodique), nitrate de gallium, ganirélix, géfitinib, gemcitabine, gemtuzumab, glucarpidase, glutoxim, goséréline, granisétron, facteur de stimulation des colonies de granulocytes (G-CSF), facteur de stimulation des colonies de macrophages de granulocytes (GM-CSF), dichlorhydrate d'histamine, histréline, hydroxycarbamide, grains d'I-125, acide ibandronique, ibritumomab-tiuxétan, idarubicine, ifosfamide, imatinib, imiquimod, improsulfan, indisétron, acide incadronique, ingenol mébutate, interféron-alfa, interféron-bêta, interféron-gamma, iobitridol, iobenguane I-123, ioméprol, ipilimumab, irinotécan, itraconazole, ixabépilone, lanréotide, lansoprazole, lapatinib, lasocholine, lenalidomide, lentinan, létrozole, leuproreline, lévamisole, lévonorgestrel, lévothyroxin-sodium, lipégfilgrastim, lisuride, lobaplatine, lomustine, lonidamine, masoprocol, médroxyprogesteron, mégestrol, mélarsoprol, melphalan, mépitiostan, mercaptopurine, mesna, méthadone, méthotrexate, méthoxsalen, méthylaminolévulinate, méthylprednisolone, méthyltestostérone, métirosine, mifamurtide, miltéfosine, miriplatine, mitobronitol, mitoguazone, mitolactol, mitomycine, mitotan, mitoxantrone, mogamulizumab, molgramostim, mopidamol, chlorhydrate de morphine, sulfate de morphine, nabilone, nabiximols, nafaréline, naloxone + pentazocine, naltrexone, nartograstim, nedaplatine, nélarabine, acide neridronique, nivolumab pentetréotide, nilotinib, nilutamide, nimorazole, nimotuzumab, nimustine, nitracrine, nivolumab, obinutuzumab, octréotide, ofatumumab, omacétaxine mépésuccinate, oméprazole, ondansétron, orgotéine, orilotimod, oxaliplatine, oxycodone, oxymétholone, ozogamicine, thérapie génique par la p53, paclitaxel, grains de palladium-103, palonosetron, acide pamidronique, panitumumab, pantoprazole, pazopanib, pegaspargase, pembrolizumab, Peg-interféron alfa-2b, pémétrexed, pentostatine, péplomycine, perflubutane, perfosfamide, pertuzumab, picibanil, pilocarpine, pirarubicine, pixantron, plérixafor, plicamycine, poliglusam, polyoestradiol phosphate, polyvinylpyrrolidone + hyaluronate de sodium, polysaccharide-K, pomalidomide, ponatinib, porfimer-sodium, pralatréxate, prednimustine, prednisone, procarbazine, procodazole, propranolol, quinagolide, rabéprazole, racotumomab, chlorure de radium-223, radotinib, raloxifène, raltitréxed, ramosétron, ramucirumab, ranimustine, rasburicase, razoxan, réfamétinib, régorafénib, acide risédronique, étidronate de rhénium-186, rituximab, romidepsine, romurtid, roniciclib, samarium (153Sm) lexidronam, satumomab, secrétine, sipuleucel-T, sizofiran, sobuzoxane, glycididazole sodique, sorafénib, stanozolol, streptozocine, sunitinib, talaporfine, tamibarotène, tamoxifèné, tapentadol, tasonermine, técéleukine, technétium (99mTc) nofétumomab merpentan, 99mTc-HYNIC-[Tyr3]-octréotide, tégafur, tégafur + giméracil + otéracil, témoporfine, témozolomide, temsirolimus, téniposide, testostérone, tétrofosmine, thalidomide, thiotépa, thymalfasine, thyrotropine alfa, tioguanine, tocilizumab, topotécan, torémifène, tositumomab, trabectédine, tramadol, trastuzumab, tréosulfan, tréitoïne, trifluridine + tipiracil, tramétinib, trilostane, triptoréline, trofosfamide, thrombopoiétine, ubénimex, valrubicine, vandétanib, vapréotide, vatalanib, vémurafénib, vinblastine, vincristine, vindésine, vinflunine, vinorelbine, vismodégib, vorinostat, microbilles de verre d'yttrium-90, zinostatine, zinostatine stimalamer, acide zolédronique, zorubicine.

4. Kit constitué d'une combinaison selon l'une quelconque des revendications 1 à 3.
